(19) Europäisches Patentamt
European Patent Office
Office européen des brevets

(11) Publication number: 0 365 201 A1

# (12) EUROPEAN PATENT APPLICATION

(21) Application number: 89310361.4

(22) Date of filing: 10.10.89

(51) Int. Cl.5: C07D 231/54 , A01N 47/38 , C07D 471/04 , C07D 487/04 , C07D 491/04 , C07D 495/04

(30) Priority: 11.10.88 US 255311

(43) Date of publication of application: 25.04.90 Bulletin 90/17

(84) Designated Contracting States: ES GR

(71) Applicant: E.I. DU PONT DE NEMOURS AND COMPANY
1007 Market Street
Wilmington Delaware 19898(US)

(72) Inventor: Lahm, George Philip
148 Fairhill Drive
Wilmington Delaware 19808(US)

(74) Representative: Hildyard, Edward Martin et al
Frank B. Dehn & Co. Imperial House 15-19 Kingsway
London WC2B 6UZ(GB)

## (54) Substituted indazoles as arthropodicides.

(57) Indazole arthropodicides, compositions containing them and a method for controlling arthropods employing these indazoles as the active ingredient. The indazoles have the following general formula:

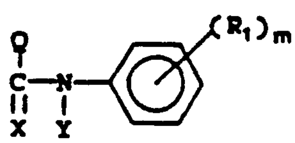

wherein
Q is a substituted indazole residue;
X is O or S;
Y is H or an organic group of defined structure;
$R_1$ is H or a substituent group; and
m is 1 to 3.

EP 0 365 201 A1

# SUBSTITUTED INDAZOLES AS ARTHROPODICIDES

## Background of the Invention

This invention pertains to use of certain substituted indazoles in compositions that are effective in the control of arthropods.

U.S. 4,070,365 and U.S. 4,663,341 disclose certain insecticidal compounds. However, such compounds are not those employed in the practice of this invention and the patents contain no teaching or suggestion that such compounds are useful against nonagronomic arthropod species.

## Summary of the Invention

This invention pertains to use of the compounds of Formula I, including all geometric and stereoisomers and suitable salts thereof, in compositions for the control of nonagronomic arthropods. This invention also pertains to the pharmaceutical and veterinary compositions that comprise such compounds. Useful compounds are:

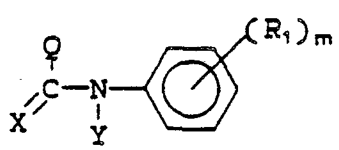

I

wherein

Q is 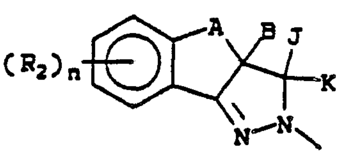

A is a 1, 2 or 3-atom bridge having 0 to 3 carbon atoms and 0 to 1 oxygen atom, $NR_6$ group, or $S(O)_q$ group, wherein each carbon individually can be substituted with 1 to 2 substituents selected from 1 to 2 halogen, $C_1$ to $C_6$ alkyl, $C_2$ to $C_4$ alkoxycarbonyl or phenyl optionally substituted with 1 to 3 substituents selected from W and one of the carbon atoms can be C(O) or C(S);

B is H, $C_1$ to $C_6$ alkyl, $C_4$ to $C_7$ cycloalkylalkyl, $C_3$ to $C_6$ cycloalkyl optionally substituted with 1 to 2 halogens or 1 to 2 $CH_3$; $C_1$ to $C_6$ haloalkyl, $C_2$ to $C_6$ alkenyl, $C_2$ to $C_6$ haloalkenyl, $C_2$ to $C_6$ alkynyl, $OR_7$, $C_2$ to $C_6$ alkoxyalkyl, $C_2$ to $C_6$ cyanoalkyl, $C_3$ to $C_8$ alkoxycarbonylalkyl, $CO_2R_3$, $C(O)R_3$, $C(O)NR_3R_4$, $C(S)NR_3R_4$, $C(S)R_3$, $C(S)SR_3$, phenyl, phenyl substituted by $(R_5)_p$, benzyl, or benzyl substituted, with 1 to 3 substituents independently selected from W;

J is H, $C_1$ to $C_4$ alkyl or phenyl optionally substituted with W;

K is H or $CH_3$;

W is halogen, CN, $NO_2$, $C_1$ to $C_2$ alkyl, $C_1$ to $C_2$ haloalkyl, $C_1$ to $C_2$ alkoxy, $C_1$ to $C_2$ haloalkoxy, $C_1$ to $C_2$ alkylthio, $C_1$ to $C_2$ haloalkylthio, $C_1$ to $C_2$ alkylsulfonyl or $C_1$ to $C_2$ haloalkylsulfonyl;

$R_1$, $R_2$ and $R_5$ are independently $R_3$, halogen, CN, $N_3$, SCN, $NO_2$, $OR_3$, $SR_3$, $SOR_3$, $SO_2R_3$, $OC(O)R_3$, $OSO_2R_3$, $CO_2R_3$, $C(O)R_3$, $C(O)NR_3R_4$, $SO_2NR_3R_4$, $NR_3R_4$, $NR_4C(O)R_3$, $OC(O)NHR_3$, $NR_4C(O)NHR_3$, $NR_4SO_2R_3$, or when m, n or p is 2, $R_1$, $R_2$ or $R_5$ can be a 5- or 6-membered fused ring selected from the group $-OCH_2O-$, $-OCH_2CH_2O-$, or $-CH_2CH_2O-$, each of which can be substituted with 1 to 4 halogen atoms or 1 to 2 methyl groups;

$R_3$ is H, $C_1$ to $C_6$ alkyl, $C_1$ to $C_6$ haloalkyl, $C_2$ to $C_6$ alkenyl, $C_2$ to $C_6$ haloalkenyl, $C_2$ to $C_6$ alkynyl, $C_2$ to $C_6$ haloalkynyl, $C_2$ to $C_6$ alkoxyalkyl, $C_2$ to $C_6$ alkylthioalkyl, $C_1$ to $C_6$ nitroalkyl, $C_2$ to $C_6$ cyanoalkyl, $C_3$ to $C_8$ alkoxycarbonylalkyl, $C_3$ to $C_6$ cycloalkyl, $C_3$ to $C_6$ halocycloalkyl, phenyl, benzyl, or phenyl or benzyl substituted with 1 to 3 substituents independently selected from W;

$R_4$ is H, $C_1$ to $C_4$ alkyl or $R_3$ and $R_4$ can be taken together as $(CH_2)_4$ $(CH_2)_5$ or $(CH_2CH_2OCH_2CH_2)$;

$R_6$ is H, $C_1$ to $C_4$ alkyl, $C_1$ to $C_4$ haloalkyl, $C_2$ to $C_4$ alkenyl, $C_2$ to $C_4$ haloalkenyl, phenyl, benzyl, phenyl optionally substituted with W or benzyl optionally substituted with W;

$R_7$ is H, $C_1$ to $C_4$ alkyl, $C_2$ to $C_4$ alkenyl, $C_2$ to $C_4$ alkynyl, $C_2$ to $C_4$ alkylcarbonyl, $C_2$ to $C_4$ alkoxycarbonyl, $C_1$ to $C_4$ alkylsulfonyl;

X is O or S;

n is 1 to 4;

m is 1 to 5;

p is 1 to 3;

q is 0 to 2; and

Y is H, $C_1$ to $C_6$ alkyl, benzyl, $C_2$ to $C_6$ alkoxyalkyl, CHO, $C_2$ to $C_6$ alkylcarbonyl, $C_2$ to $C_6$ alkoxycarbonyl, $C_2$ to $C_6$ haloalkylcarbonyl, $C_1$ to $C_6$ alkylthio, $C_1$ to $C_6$ haloalkylthio, phenylthio, or phenylthio substituted with 1 to 3 substituents independently selected from W.

Preferred compounds (A) for this use are those of Formula I wherein Q is

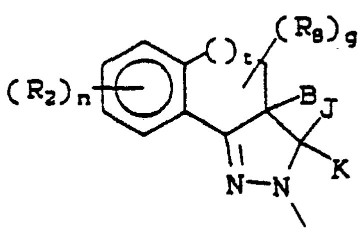 ;

Q-1

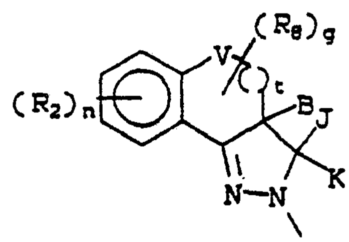 ;

Q-2

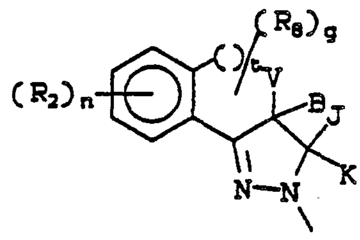 ; 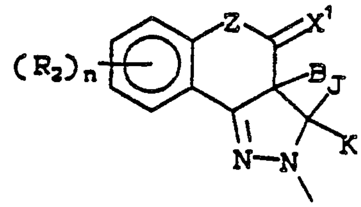 ; or

Q-3 Q-4

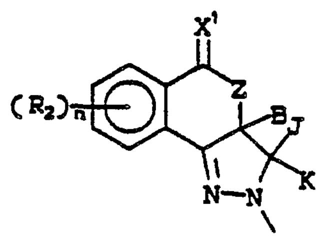 ;

Q-5

X is O or S;
$X^1$ is O or S;
t is 0, 1 or 2;
v is O, S(O)q or $NR_6$;
Z is O or $NR_6$;
$R_8$ is halogen, $C_1$ to $C_6$ alkyl, $C_2$ to $C_4$ alkoxycarbonyl or phenyl optionally substituted by 1 to 3 substitutents selected from W; and
g is 0, 1 or 2.

Preferred compounds (B) for this use are preferred compounds (A) wherein
B is H, $C_1$ to $C_6$ alkyl, $C_1$ to $C_6$ haloalkyl, $C_2$ to $C_6$ alkenyl, $C_2$ to $C_6$ alkynyl, $C_2$ to $C_6$ alkoxyalkyl, $C_3$ to $C_8$ alkoxycarbonylalkyl, $CO_2R_3$, $C(O)R_3$, $C(O)NR_3R_4$, $C(S)NR_3R_4$, $C(S)R_3$, $C(S)SR_3$, phenyl or phenyl substituted by $(R_5)_p$;
J is H or $CH_3$;
$R_3$ is H, $C_1$ to $C_4$ alkyl, $C_1$ to $C_2$ haloalkyl, $C_3$ to $C_4$ alkenyl, $C_3$ to $C_4$ haloalkenyl, propargyl, phenyl, benzyl, or phenyl or benzyl substituted with one of F, $C_1$, Br, $CF_3$ or $NO_2$;
$R_6$ is H, Cl to $C_4$ alkyl, allyl or propargyl;
n is 1 or 2;
p is 1 or 2;
m is 1 to 3; and
Y is H, $C_1$ to $C_4$ alkyl, $SCH_3$, $SCCl_3$, $SO_2CH_3$, $SC_6H_5$, 2-$NO_2$-$C_6H_4S$, $C(O)CH_3$, $C(O)CF_3$; $CO_2CH_3$ or $CO_2CH_2CH_3$.

Preferred compounds (C) for this use are preferred compounds (B) wherein
B is H, $C_1$ to $C_4$ alkyl, $C_3$-$C_4$ alkoxycarbonylalkyl, $CO_2R_3$, $C(O)R_3$, phenyl or phenyl substituted by $(R_5)_p$;
J is H;
K is H;
$R_1$ is H, halogen, CN, SCN, $NO_2$, $OR_3$, $SR_3$, $SO_2R_3$, $CO_2R_3$, $C(O)R_3$ or $R_3$ with one $R_1$ substituent in the 4-position, or when m is 2 then $R_1$ can be taken together as $-CH_2C(CH_3)_2O-$, $OCH_2CH_2O-$, $OCF_2CF_2O$-or $-CF_2CF_2O-$ to form a 5 or 6 membered fused ring;
$R_2$ and $R_5$ are H, $R_3$, halogen, CN, SCN, $NO_2$, $OR_3$, $SR_3$, $SO_2R_3$, $OC(O)R_3$, $OSO_2R_3$, $CO_2R_3$, $C(O)R_3$, C-

(O)$NR_3R_4$, $SO_2NR_3R_4$ or $NR_3R_4$;
$R_3$ is $C_1$ to $C_4$ alkyl, $C_1$ to $C_2$ haloalkyl, $C_3$ to $C_4$ alkenyl or propargyl;
$R_4$ and $R_8$ are H or $CH_3$;
X is O;
X′ is O;
m is 1 or 2;
t is 1; and
q is O.

Preferred compounds (D) for this use are preferred compounds (C) wherein
$R_1$ is Cl, F, Br, $CF_3$, CN, $OCH_2CF_3$, $OCF_3$, $OCF_2H$ or $SCF_2H$;
$R_2$ is H, Cl, F, Br, CN, $CF_3$, $CH_3$, $OCH_2CF_3$, $OCF_2H$, $OCF_3$, $SCH_3$, $SCF_2H$, $SO_2CH_3$ or $NO_2$;
$R_6$ is H or $CH_3$;
B is H, $CH_3$, $CH_2CH_3$, $CH(CH_3)_2$, $CO_2CH_3$, $CO_2CH_2CH_3$, phenyl or phenyl substituted by halogen, CN, $CF_3$ or $NO_2$; and
Y is H, $COCH_3$ or $CO_2CH_3$.

Preferred compounds (E) for this use are preferred compounds (D) wherein
Q is Q-1.

Preferred compounds (F) for this use are preferred compounds (D) wherein
Q is Q-2.

Specifically preferred compounds are preferred compounds (E):
Methyl 3,3a,4,5-tetrahydro-2-[[4-(trifluoromethyl)phenylamino]carbonyl]-2H-benz[g]indazole-3a-carboxylate, (G)
Methyl 7-chloro-3,3a,4,5-tetrahydro-2-[[4-trifluoromethyl)phenylamino]carbonyl]-2H-benz[g]indazole-3a-carboxylate, (H)
7-Chloro-3,3a,4,5-tetrahydro-N-[4-(trifluoromethyl)phenyl]-2H-benz[g]indazole-2-carboxamide, (I)
Methyl 6-fluoro-3,3a,4,5-tetrahydro-2-[[4-(trifluoromethyl)phenylamino]carbonyl]-2H-benz[g]indazole-3a-carboxylate, (J).

The active compounds described herein, in addition to being useful as household insecticides, can be combined with other pesticides for household uses that will readily occur to one skilled in the art. Reference to compositions of this invention or to compounds of Formula I includes geometric and stereoisomers of the latter as well as suitable salts which, for brevity only, are not referred to specifically hereafter.

## Details of the Invention

For descriptive purposes, the following numbering system will be used to describe the values of Q in both the discussion and tables that follow.

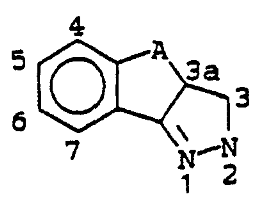

Compounds of Formula I can be prepared by the reaction of aryl isocyanates of Formula II and substituted indazoles of Formula III as shown in Scheme I. Typical reactions involve the combination of equimolar amounts of II and III in conventional organic solvents including ether, tetrahydrofuran (THF), methylene chloride, chloroform and benzene. The reaction can be run at temperatures ranging from about -20° C to 100° C with temperatures in the range of about -10° to 30° C generally preferred.

SCHEME 1

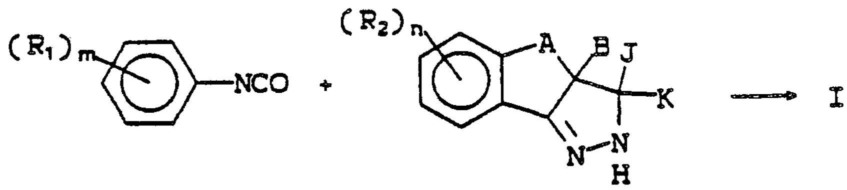

II III I

Substituted indazoles of Formula III, where B is equal to H, can be prepared by the reaction of hydrazine with an $\alpha$, $\beta$-unsaturated ketone of Formula IV (or their precursors) by procedures well documented in the chemical literature (Scheme 2). For a review of pyrazoline synthesis, see El-Rayyes and Al-Awadi, Synthesis, 1028 (1985). For literature describing the synthesis of 3,4- and 3,5-disubstituted pyrazolines, which can be applied to the synthesis of compounds of Formula III, where B is H, see U.S. Patent 3,991,073 and U.S. Patent 4,070,365.

SCHEME 2

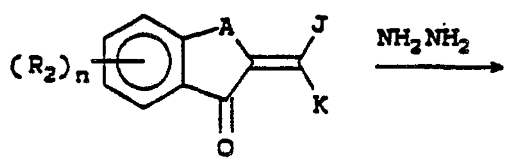


III

(B=H)

IV

Preparation of compounds of Formula I, where B is other than H, $OR_7$, phenyl or substituted phenyl can be achieved by metallation of the 3a- position of Formula I (where B is H) followed by reaction with a suitable electrophile as depicted in Scheme 3. Procedures for the related transformation of 4-substituted pyrazolines to 4,4-disubstituted pyrazolines are reported in European Patent Application 153127 and can be applied to substituted indazoles of Formula I. Metallation can be accomplished by deprotonation with a strong base, in a suitable solvent and at temperatures ranging from -78 °C to 100 °C. Useful bases for this reaction include lithium dialkylamides such as lithium diisopropylamide and lithium tetramethylpiperide and alkyl lithium reagents such as n-butyllithium. Deprotonation of compounds of Formula I, where B is equal to hydrogen, may require two equivalents of base when Y is equal to hydrogen. The reaction can be conducted in many conventional organic solvents and in certain instances a cosolvent may be useful. Suitable solvents include diethylether, tetrahydrofuran, tetrahydropyran, dimethylformamide, hexamethylphosphoramide, benzene and the like. Suitable electrophilic reagents for reaction with the metallated Formula I compounds include alkyl and substituted alkyl halides, alkyl chloroformates, acyl halides, isocyanates, dialkyl carbamoylhalides and related electrophiles which will be known to those skilled in the art.

SCHEME 3

I (B is H) —— 1. Base, 2. $E^+$ ——→ I (B is other than H, $OR_7$, phenyl or substituted phenyl)

where $E^+$ is an electrophilic reagent.

An alternative procedure for introduction of the 3a-substituent, which in certain instances may be preferred over that of Scheme 3 due to higher yields and/or greater ease of synthesis, proceeds via the intermediacy of Compound V, wherein the 2-nitrogen has been derivatized with a suitable protecting group. Deprotonation with a strong base such as lithium diisopropylamide, typically in stoichiometric quantities, followed by reaction with any of the previously described electrophiles provides compounds of Formula V where B is other than hydrogen, $OR_7$, phenyl or substituted phenyl. Removal of the nitrogen protecting group provides the required Formula III intermediate. Nitrogen-protecting groups are well documented in the chemical literature, as are procedures for their preparation and cleavage. Examples include acetyl, trifluoroacetyl, benzoyl, substituted benzoyl, alkoxycarbonyl, benzyl and substituted benzyl. For a review see Greene "Protective Groups in Organic Synthesis" (New York: John Wiley and Sons, 1981) pp. 218 to 287.

SCHEME 4

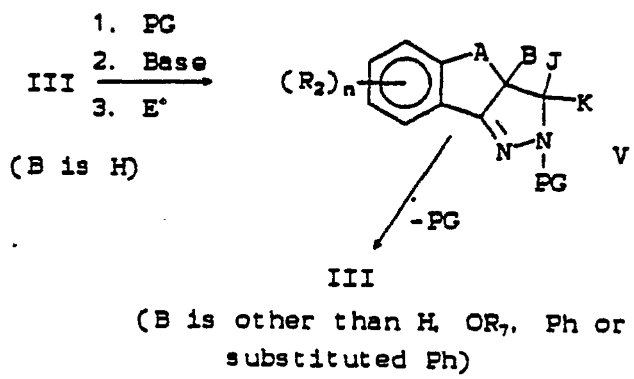


where $E^+$ is an electrophile and PG is representative of a suitable nitrogen-protecting group.

Formula III compounds where B is equal to hydroxy are prepared by the reaction of hydrazine with a suitably substituted epoxide of Formula VI (Scheme 5, Reaction 1). The epoxides are available from the enones of Formula IV by procedures known in the art, the most widely employed method being epoxidation via the use of alkaline hydrogen peroxide (see, e.g., Wasson and House, Org. Syn., Coll. Vol. 4, 552 (1963)).

Compounds of Formula III where B is equal to $OR_7$ and $R_7$ is other than hydrogen, may be prepared from the Formula III compounds where B is equal to hydroxy via reaction with electrophilic reagents such as alkyl halides, acyl halides, alkyl chloroformates, chlorosulfonates, isocyanates and dialkyl carbamoylhalides (Scheme 5, Reaction 2). Those skilled in the art will recognize that conditions necessary for this transformation including solvent, catalyst, temperature, etc., will vary with the specific electrophile chosen and that protection of the 2-nitrogen may be necessary.

SCHEME 5

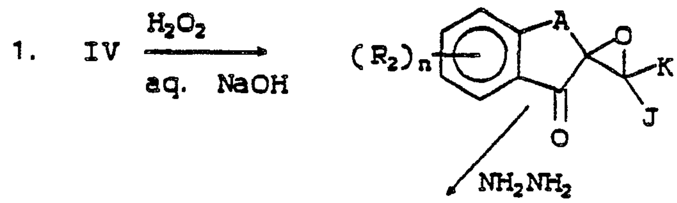


III

(B is OH)

2. III (B is OH)

1. PG

2. E+

3. -PG

III (B is $OR_7$)

where $E^+$ and PG are as previously defined.

Compounds of Formula III where B is phenyl or substituted phenyl may be prepared by reaction of a substituted ketone of Formula VIII with hydrazine. The Formula VIII compounds are in turn prepared from compounds of Formula VII in a two-step process involving first an aldol or Mannich type reaction on the Formula VII compound followed by conversion of the hydroxyl or dialkylamino group to a leaving group L, examples of which include conversion to the chloride, bromide, tosylate or mesylate by procedures well known in the art.

SCHEME 6

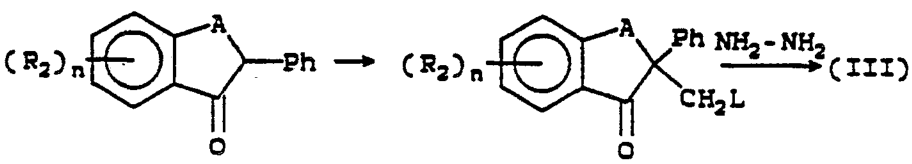


VII VIII

(B is Ph or substituted phenyl)

Compounds of Formula IV (or their precursors) are readily prepared from compounds of Formula IX via an aldol condensation followed by dehydration of the aldol, or when J and K are hydrogen via a Mannich reaction followed by elimination of the dialkylamino group. For a general review of these reactions, see March, "Advanced Organic Chemistry", 3rd ed., (New York: John Wiley and Sons, 1985) pp. 829 to 834, and House, "Modern Synthetic Reactions", 2nd ed., (Menlo Park, CA: N.A. Benjamine, 1972) pp. 655 to 660.

SCHEME 7

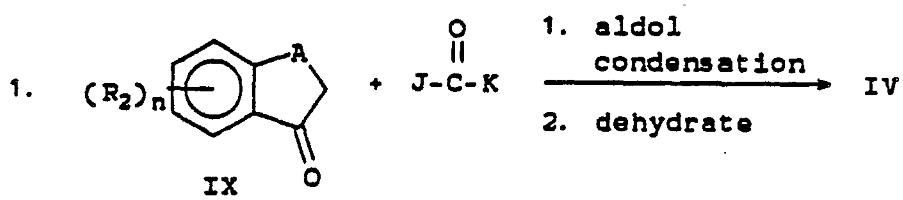


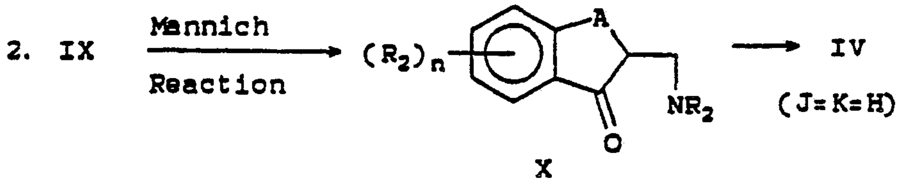


Those skilled in the art will recognize the intermediates of Formula IX, which include compounds such as indanones, tetralones, chromanones, thiochromanones, and the like are well documented in the chemical literature as are procedures for their preparation. Selected examples of Formula IX are available commercially as well.

An alternate synthesis of compounds of Formula I is via the phosphorus intermediate XI.

Intramolecular cyclization of olefin XII to the tetrahydropyrazole XI, subsequent removal of the phosphorus moiety, and addition of the C(O)NH-aryl moiety results in the preparation of Compound I.

SCHEME 8

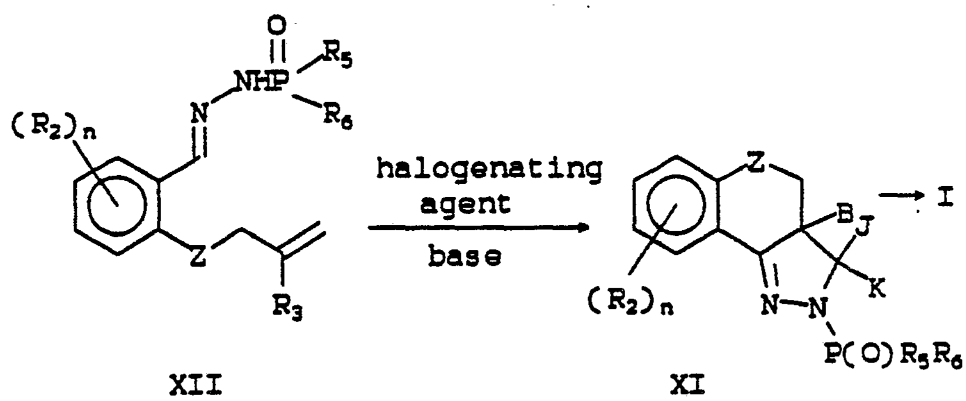


XII XI

Starting reactant XII can be prepared by reacting aldehyde XIII with phosphoryl hydrazide XIV.

SCHEME 9

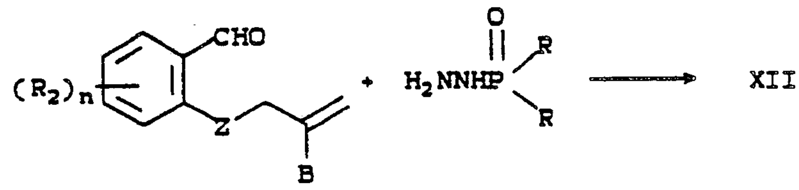

XIII XIV

This reaction is typically carried out in an inert solvent such as ethanol or dichloromethane containing 0 to about 5 mol percent of an acid catalyst such as acetic or p-toluenesulfonic. Approximately equimolar amounts of the two components are mixed in the solvent and catalyst, if any, and maintained at a temperature between about 0 to 30 °C for a period of about 5 to 150 minutes. Compound XII can crystallize from the reaction solvent; if not, it can be isolated by evaporating the bulk of the solvent, diluting with water, filtering the product, and drying under vacuum. In the event that the product does not crystallize from the aqueous medium, it can be extracted into an inert solvent such as dichloromethane. After drying the organic extract with a suitable drying agent such as sodium sulfate, it can be used in the next step, or concentrated and optionally purified by recrystallization or chromatography on silica gel. Suitable solvent mixtures for recrystallization include aqueous ethanol or methanol, ether-hexanes, or ethyl acetate-hexanes. The latter mixture can be used for silica gel chromatography.

Conversion of olefin XII to pyrazoline XI can be conducted by gradually adding a base, such as triethylamine, to a solution or suspension of XII and a moderately reactive halogenating agent, such as N-chlorosuccinimide, in an inert, dry solvent such as ether, dichloromethane, toluene, and the like.

Alternatively, the halogenating agent can be gradually added to a mixture of the base and the compound of Formula XII. In either case, optimum yields of XI are obtained when the temperature (between -20 and 40 °C) and concentration (between about 0.0001 and 3M) are as low as possible and when the component being added to the reaction mixture is added gradually. Thus, conditions should be chosen so that the intramolecular cycloaddition of the postulated intermediate is favored over bimolecular side reactions, such as oligomerization.

The reagent, N-chlorosuccinimide, is preferred but other moderately reactive halogenating agents such as N-bromosuccinimide, t-butyl hypochlorite, cupric bromide, iodine, or sulfuryl chloride can also be used. The base of choice generally depends upon the reactivity of the halogen source. For example, whereas a trialkylamine is suitable for use with N-haloamides, an alkaline-earth or alkali-metal carbonate, bicarbonate, or oxide may be preferable when using the more reactive t-butyl hypochlorite. For optimum yields, it is important to maintain anhydrous conditions.

Isolation and purification of XI is generally accomplished by washing an ether solution thereof to remove water-soluble by-products, e.g., succinimide and trialkylamine hydrohalides, drying the purified extract with an inert inorganic salt such as magnesium sulfate or another suitable drying agent, concentration of the filtered ether solution, and crystallization from a suitable organic solvent or solvent mixture, e.g., ether and hexane. If XI fails to crystallize it can nevertheless be carried on to the next step or, if not sufficiently pure, it can be chromatographed on a silica gel column using a suitable mixture of solvents such as ethyl acetate and hexanes.

Removal of the phosphate protecting group of Compound XI is performed by reacting a compound of Formula XI with about 0.9 to 5.0 equivalents of a strong acid such as HCl, $H_2SO_4$, or p-toluenesulfonic in a suitable solvent such as tetrahydrofuran or a lower alkanol optionally containing 0.1 to 10 equivalents of water at a temperature of between about 20 to 100 °C. After an appropriate contact time, usually between 1 to 48 hours, the mixture is cooled to about 0-5 °C and the precipitated pyrazoline intermediate III is collected by filtration. In cases where the deprotected pyrazoline fails to crystallize, it can be precipitated by the addition of ether (optionally after concentration to remove excess reaction solvent). Alternatively, the reaction medium can be neutralized with an aqueous solution of an inorganic base such as sodium bicarbonate. The reaction solvent is then removed by evaporation and the free base of III is extracted into an organic solvent such as ether, ethyl acetate or dichloromethane. The organic extracts are then used directly in the preparation of Compound I.

The following procedures illustrate preparation of active compounds employed in compositions of the invention.

## Procedure 1

3,3a,4,5-Tetrahydro-N-[4-(trifluoromethyl)phenyl]-2H-benz[g]indazole-2-carboxamide

A mixture of 10.0 g of α-tetralone, 2.0 g of paraformaldehyde, 6.5 g of dimethylamine hydrochloride and 1.75 ml of conc. HCl in 20 ml of ethanol was heated at reflux for 24 h, cooled to room temperature and then partitioned between ether and water. The aqueous extracts were made basic with 1N aqueous NaOH and extracted with ether. The ether extracts were dried over magnesium sulfate and concentrated to 12.3 g of a yellow oil. The residual oil was dissolved in 40 ml of n-propyl alcohol, combined with 6.7 ml of hydrazine hydrate and heated at reflux for 1 h. The reaction mixture was then concentrated under vacuum, partitioned between 5% aqueous $NaHCO_3$ and methylene chloride and dried over magnesium sulfate. To the methylene chloride extracts were added 12.5 g of 4-trifluoromethylphenyl isocyanate and the mixture was then refluxed one hour, cooled to room temperature and concentrated to 26.1 g of a brown oil. Chromatography on silica gel followed by trituration with ether afforded 11.78 g of the title compound as a tan powder, m.p. 149-151 °C. $^1H$ NMR ($CDCl_3$), δ 1.9 (m,1H), 2.4 (m,1H), 3.0 (m,2H), 3.5 (m,2H), 4.4 (m,1H), 7.3 (m,3H), 7.58 (d,2H), 7.65 (d,2H), 8.0 (d,2H), 8.3 (bs,1H).

## Procedure 2

3,3a,4,5-tetrahydro-3a-methyl-N-[4-(trifluoromethyl)phenyl]-2H-benz[g]indazole-2-carboxamide

To a solution of 0.9 ml of diisopropylamine in 10 ml of THF, at -78 °C, was added 2.3 ml of 2.5 M n-butyl lithium in hexane and the mixture was stirred for 5 mins. To this -78 °C solution was added a solution of 1.0 g of the title compound of Example 1 in 5 ml of THF. The reaction was warmed to 0 °C, recooled to -78 °C and then 0.4 ml of methyl iodide was added. The reaction mixture was then stirred for 24 h, with gradual warming to room temperature, quenched with 0.5 ml of glacial acetic acid, and poured into a 5% solution of aqueous $NaHCO_3$. The mixture was extracted with chloroform, dried over magnesium sulfate and concentrated. Crystallization from 1:1 ethylacetate/hexane afforded 0.41 g of a yellow solid, m.p. 130 ° to 135 °C. Chromatography of the filtrate on silica gel with 30% ethyl acetate hexane afforded an additional 0.17 g of a higher purity sample of the title compound, m.p. 152.5 ° to 153.5 °C. $^1H$ NMR ($CDCl_3$), δ 1.29 (s, 3H), 2.1 (m,2H), 2.9-3.3 (m, 2H), 3.70 (d, 1H), 4.12 (d,1H), 7.3 (m,3H), 7.57 (d,2H), 7.68 (d,2H), 7.98 (d,1H), 8.3 (bs,1H).

## Procedures 3

Methyl 3,3a,4,5-tetrahydro-2-[[4-(trifluoromethyl)phenylamino]carbonyl]-2H-benz[g]indazole-3a-carboxylate

Application of the procedure of Example 2 with 0.75 ml of methyl chloroformate afforded, after chromatography on silica gel, 0.25 g of the title compound as a white solid, m.p. 177 ° to 180 °C. $^1H$ NMR ($CDCl_3$), δ 2.1 (m,1H), 2.7 (m,1H), 3.0 (m,2H), 3.71 (s,3H), 3.76 (d,1H), 4.58 (d,1H), 7.3 (m,3H), 7.57 (d,2H), 7.66 (d,2H), 8.0 (d,1H), 8.22 (s,1H).

## Procedure 4

7-Chloro-3,3a,4,5-tetrahydro-N-[4-(trifluoromethyl)phenyl]-2H-benz[g]indazole-2-carboxamide

Step A: ((2-(3-chlorophenyl)ethyl)methanesulfonate

To a 0° C solution of 30.0 g of 3-chlorophenethyl alcohol and 15.3 ml of methane sulfonyl chloride in 150 ml of THF was added, dropwise, a solution of 28.0 ml of triethylamine in 50 ml of THF. The reaction was warmed to room temperature, stirred overnight, and then filtered. The filtrate was partitioned between aqueous sodium bicarbonate and ether. The organic extracts were then dried over magnesium sulfate, filtered and concentrated under reduced pressure to afford 45.93 g of a clear, colorless oil. $^1H$ NMR was consistent with the structure for Step A.

Step B: 3-chlorobenzenebutanoic acid

To a mixture of 8.0 g of 60% sodium hydride in 300 ml of THF, under $N_2$, was added dropwise a solution of 31.0 ml of diethyl malonate in 50 ml of THF. Upon complete addition of the diethyl malonate, a pale yellow homogenous solution was obtained. To this was added a solution of 45.93 g of the sulfonate from Step A and the mixture was then heated at reflux overnight. The reaction was then cooled to room temperature, poured into 400 ml of 1N HCl, and extracted with ether. The ether extracts were dried over magnesium sulfate, filtered and concentrated under reduced pressure to afford 68.9 g of a yellow oil. The crude oil was dissolved in 400 ml of methanol, 100 ml of $H_2O$ and 40 ml of 50% aqueous NaOH. The reaction was stirred overnight and the methanol was then removed at reduced pressure. The crude residue was partitioned between $H_2O$ and ether, the aqueous extracts were acidified with concentrated HCl and then extracted several times with ether. The ether extracts were dried over magnesium sulfate, filtered and concentrated to afford 51.7 g of a yellow oil. The crude residue was dissolved in 200 ml of toluene and heated at reflux for 4 days under $N_2$ to effect decarboxylation. After this time toluene was removed by concentration at reduced pressure to afford 35.72 g of a yellow oil. $^1H$ NMR analysis of the crude product was consistent with 3-chlorobenzenebutanoic acid of purity estimated to be 80%. The crude product was used without further purification directly in the next step.

Step C: 6-chloro-3,4-dihydro-1(2H)-naphthalenone

A mixture of 35.72 of the product from Step B and 50 ml of thionyl chloride was heated at reflux for 2 hours and then stirred at room temperature for 18 hrs. After this time thionyl chloride was removed at reduced pressure and the product was dissolved in carbon tetrachloride and concentrated at reduced pressure. The residue was dissolved in 150 ml of dichloroethane, cooled to 0° C, and 28 g of aluminum chloride was added portionwise over about 1 hr in approximately 3 g portions. After stirring for 3 hrs the reaction was poured over a mixture of ice/1N HCl and extracted three times with methylene chloride. The organic extracts were dried over magnesium sulfate and concentrated to approximately 30 g of a brown oil. Chromatography on silica gel with 10% ethyl acetate/hexane afforded 17.83 g of 6-chloro-3,4-dihydro-1(2H)-naphthalenone as a brown oil. $^1H$ NMR was consistent with the structure.

Step D: 7-chloro-3,3a,4,5-tetrahydro-N-[4-(trifluoromethyl)phenyl]-2H-benz[g]indazole-2-carboxamide

A mixture of 6-chloro-3,4-dihydro-1(2H)-naphthalenone (Step C), 2.5 g of dimethylamine hydrochloride, 1.0 g of paraformaldehyde, 0.7 ml of concentrated HCl and 15 ml of ethanol was combined and heated at reflux for 18 hrs. The reaction was then concentrated at reduced pressure and partitioned between $H_2O$ and ether. The aqueous extracts were made basic with 1N NaOH and then extracted three times with ether. The ether extracts were dried over magnesium sulfate and concentrated to 4.64 g of a yellow oil. This compound was dissolved in 25 ml of ethanol and 1.5 ml of hydrazine hydrate was added followed by 5 to 6 drops of 50% sodium hydroxide. The reaction was then heated at reflux, under $N_2$, for 2 to 3 hrs after which time it was cooled and most of the ethanol was removed by concentration at reduced pressure. The crude residue was partitioned between saturated aqueous sodium bicarbonate and methylene chloride. The methylene chloride extracts were dried over magnesium sulfate and filtered. The methylene chloride extracts were then combined with 3.5 g of 4-trifluoromethylphenyl isocyanate and stirred under $N_2$ overnight. The reaction was then concentrated and the crude residue triturated with ether to afford 3.35 g of the title compound as a white powder, m.p. 196 to 199° C.

$^1H$ NMR($CDCl_3$) $\delta$ 1.9 (m, 1H), 2.2 (m, 1H), 3.0 (m, 2H), 3.5 (m, 2H), 4.43 (m, 1H), 7.24 (m, 2H), 7.55 (d,

2H), 7.67 (d, 2H), 7.92 (d, 1H), 8.20 (s, 1H).

Procedure 5

Methyl 7-chloro-3,3a,4,5-tetrahydro-2-[[4-(trifluoromethyl)phenylamino]carbonyl]-2H-benz[g]indazole-3a-carboxylate

A solution of 50 ml of THF and 6.7 ml of diisopropylamine was cooled under $N_2$ to -78°C and then 17.5 ml of 2.5 M n-butyllithium in hexane was added. After 5 min, a solution of 7.8 g of the title compound of Example 4 in 15 ml of THF was added dropwise and the dark red solution that formed was stirred at -78°C for an additional 15 min. After this time a solution of 4.6 ml of methyl chloroformate in 10 ml of THF was added dropwise and the red color dissipated rapidly. The reaction was warmed to room temperature and after 1 hr quenched with 5% aqueous sodium bicarbonate. The reaction mixture was partitioned between ether and 5% aqueous sodium bicarbonate. The ether extracts were dried over magnesium sulfate and concentrated to 14.1 g of a yellow oily solid. The crude product was triturated with ether and the resulting white precipitate was filtered and dried to afford 5.56 g of the title compound as a white solid, m.p. 234 to 236°C. $^1$H NMR($CDCl_3$) δ 2.1 (m, 1H), 2.75 (m, 1H), 2.95 (m, 2H), 3.71 (s, 3H), 3.75 (d, 1H, J=6Hz), 4.59 (d, 1H, J=6Hz), 7.25 (m, 2H), 7.57 (d, 2H), 7.66 (d, 2H), 7.94 (d, 1H), 8.18 (d, 1H).

Procedure 6

7-chloro-2,3,3a,4-tetrahydro-N-[4-(trifluoromethyl)phenyl]-[1]benzothiopyrano[4,3-c]pyrazole-2-carboxamide

Step A: 7-chloro-3-[(dimethylamino)methyl-2,3-dihydro-4H-1-benzothiopyran-4-one

A mixture of 15.0 g (75.7 mmol) of 7-chlorothiochroman-4-one (Chu et al., Hua Hsüeh Hsüeh Pao, 1956, 22, 371-8; Chem. Abstracts, 1958, 52, 11044a), 7.41 g (90.8 mmol) of dimethylamine hydrochloride, 3.41 g (113 mmol) of paraformaldehyde, and 1.26 ml (15 mmol) of concentrated HCl in 25 ml of ethanol under a nitrogen atmosphere was heated under reflux for 22 hrs. The reaction mixture was poured into 150 ml of water and extracted with ether (2 x 50 ml). The aqueous layer was made basic (pH 10) with 1N NaOH and then was extracted with ethyl acetate (3 x 100 ml). After being dried ($MgSO_4$), these ethyl acetate extracts were concentrated under reduced pressure to give 14.9 g of a yellow solid. The $^1$H NMR was consistent with the structure for the title compound.

Step B: 7-chloro-2,3,3a,4-tetrahydro-N-[4-(trifluoro methyl)phenyl]-[1]benzothiopyrano[4,3-c] pyrazole-2-carboxamide

To a stirred mixture of 14.0 g (54.7 mmol) of the Mannich base from Step A and 6.19 ml (6.38 g, 110 mmol) of hydrazine hydrate in 36 ml of n-propanol under a nitrogen atmosphere was added 0.29 ml (5.5 mmol) of 50% aqueous NaOH and the resulting mixture was heated at 100°C for 2 hrs. The reaction mixture was concentrated under reduced pressure, diluted with 250 ml of dichloromethane, and washed with 200 ml of dilute aqueous sodium bicarbonate. The aqueous layer was extracted with 50 ml of dichloromethane. The combined organic layers were dried ($MgSO_4$) and filtered to give 370 ml of solution. A 120 ml portion of this solution was treated under a nitrogen atmosphere with 3.75 g (20.0 mmol) of 4-(trifluoromethyl)phenyl isocyanate and allowed to stir overnight. The reaction mixture was concentrated under reduced pressure, and the residue was triturated with 35 ml of ether and filtered to give 4.51 g (63%) of the title compound as a yellow solid melting at 188 to 192°C. $^1$H NMR ($CDCl_3$, 200 MHz) δ 3.19 (dd, 4.5Hz, 12.6Hz, 1H), 3.32 (t, 12.0Hz, 1H), 3.69 (t, 10.8Hz, 1H), 3.86 (dq, 4.5Hz, 11Hz, 1H), 4.43 (t, 10.5Hz, 1H), 7.13 (dd, 1.9Hz, 8.8Hz, 1H), 7.24 (d, 1.9Hz, 1H), 7.58 (d, 8.8Hz, 2H), 7.66 (d, 8.8Hz, 2H), 7.93 (d, 8.4Hz, 1H), 8.13 (s, 1H).

Procedure 7

7-chloro-2,3,3a,4-tetrahydro-4,4-dimethyl-N-[4-(trifluoromethyl)phenyl]-[1]benzothiopyrano[4,3-c]pyrazole-2-carboxamide

Step A: 3-(3-chlorophenylthio)-3-methylbutanoic acid

A mixture of 20.0 g (138 mmol) of 3-chlorothiophenol, 13.85 g (138 mmol) of 3-methyl-2-butanoic acid, and 4.1 ml (3.5 g, 42 mmol) of piperidine under a nitrogen atmosphere was heated under reflux at 105° C for 28 hrs. The reaction mixture was poured into 200 ml of ether and washed with 1N HCl (2 x 80 ml) and then the aqueous layers were extracted with 50 ml of ether. The combined organic layers were dried ($MgSO_4$) and concentrated under reduced pressure to give 33.1 g of a yellow oil. The $^1H$ NMR was consistent with the structure for the title compound.

Step B: 7-chloro-2,3-dihydro-2,2-dimethyl-4H-1-benzothiopyran-4-one

To 270 g of polyphosphoric acid heated to 70° C was added, portionwise, 31.9 g of the product from Step A over 15 min with mechanical stirring under a nitrogen atmosphere. After heating at 70° C for an additional 45 min, the reaction mixture was cooled to 0° C and 150 ml of water was carefully added followed by 100 ml of ether. The resulting mixture was further diluted with 170 ml of water and 110 ml of ether. The layers were separated and the aqueous layer was extracted with ether (2 x 200 ml). The combined ether layers were washed with 100 ml of 1N NaOH and were then dried ($MgSO_4$) and concentrated under reduced pressure to give 25.8 g of a yellow solid. The $^1H$ NMR was consistent with a 68:32 mixture of the structure for Step B and its 5-chloro isomer, respectively.

Step C: 7-chloro-3-[(dimethylamino)methyl]-2,3-dihydro-2,2-dimethyl-4H-1-benzothiopyran-4-one

A mixture of 10.0 g (44.1 mmol) of the isomeric thiochromanones from Step B, 1.99 g (66.2 mmol) of paraformaldehyde, 4.32 g (52.9 mmol) of dimethylamine hydrochloride, and 0.74 ml of concentrated HCl in 15 ml of ethanol under a nitrogen atmosphere was heated under reflux for 49 hours. The reaction mixture was poured into 1000 ml of water and extracted with ether (5 x 200 ml). The aqueous layer was made basic (pH 10) with 1N NaOH and then extracted with ethyl acetate (3 x 200 ml). After being dried ($MgSO_4$), these ethyl acetate extracts were concentrated under reduced pressure to give 3.59 g of an oil. The $^1H$ NMR was consistent with a 80:20 mixture of the structure for the title compound of Step C and its 5-chloro isomer, respectively.

Step D: 7-chloro-2,3,3a,4-tetrahydro-4,4-dimethyl-N-[4-(trifluoromethyl)phenyl]-[1]benzothiopyrano[4,3-c]-pyrazole-2-carboxamide

To a stirred mixture of 3.20 g (11.3 mmol) of the Mannich bases from Step C and 1.28 ml (1.32 g, 22.6 mmol) of hydrazine hydrate in 7.5 ml of n-propanol under a nitrogen atmosphere was added 90 mg (1.1 mmol) of 50% aqueous NaOH and the resulting mixture was heated at 100° C for 2 hrs. The reaction mixture was concentrated under reduced pressure, diluted with 50 ml of dichloromethane, and washed with 100 ml of dilute aqueous sodium bicarbonate. The aqueous layer was extracted with 20 ml of dichloromethane. The combined organic layers were dried ($MgSO_4$) and filtered to give 93 ml of solution. A 27 ml portion of this solution was treated with 0.68 g (3.6 mmol) of 4-(trifluoromethyl)phenyl isocyanate and allowed to stir at room temperature for 2.5 hrs. The reaction mixture was concentrated under reduced pressure, and the residue was triturated with 15 ml of ether and filtered to give 1.03 g (71%) of the title compound as a yellow solid, m.p. 200° to 202° C. $^1H$ NMR ($CDCl_3$, 200 MHz) $\delta$ 1.35 (s, 3H), 1.45 (s, 3H), 3.83 (m, 2H), 4.21 (m, 1H), 7.14 (dd, 2.0 Hz, 8.4Hz, 1H), 7.22 (d, 1.8Hz, 1H), 7.58 (d, 8.8Hz, 2H), 7.66 (d, 8.9Hz, 2H), 7.95 (d, 8.1Hz, 1H), 8.13 (s, 1H).

Procedure 8

3a-(4-chlorophenyl)-3,3a,4,5-tetrahydro-N-[4-(trifluoromethyl)phenyl]-2H-benz[g]indazole-2-carboxamide

Step A: 4-chloro-2-(2-phenylethyl)benzeneacetic acid, methyl ester

To a solution of 4.35 g of 60% sodium hydride in 50 ml of DMF was added dropwise 20.0 g of methyl 4-chlorophenyl acetate in 25 ml of DMF. Once the evolution had ceased a mixture of 20.0 g of 2-bromoethyl benzene in 25 ml of DMF was added and the reaction was stirred at room temperature overnight. The reaction was then partitioned between 400 ml of ether and 200 ml of $H_2O$. The ether extracts were washed with $H_2O$, dried over magnesium sulfate, filtered and concentrated to 32.9 g of a yellow oil. Chromatography on silica gel with 5% ethyl acetate in hexane afforded 12.6 g of the title compound as a clear, colorless oil.

Step B: 2-(4-chlorophenyl)-3,4-dihydro-1(2H)-naphthalenone

A mixture of 12.6 g of the title compound from Step A, 100 ml of methanol, 30 ml of $H_2O$ and 10 ml of 50% NaOH were refluxed under $N_2$ overnight. The reaction was then concentrated at reduced pressure and concentrated HCl was added until acidic. The mixture was extracted three times with ether, dried over magnesium sulfate and concentrated to afford 12.14 g of a yellow oil. The residue was dissolved in 20 ml of thionyl chloride and heated at reflux under $N_2$, overnight. Thionyl chloride was removed by concentration at reduced pressure and the residue was concentrated twice from carbon tetrachloride. The crude product was dissolved in 50 ml of dichloroethane, cooled under $N_2$ to 0°C and 5.9 g of aluminum trichloride was added. The reaction was stirred overnight at room temperature, poured into 50 ml of 1N HCl and extracted with methylene chloride. The organic extracts were dried over magnesium sulfate, filtered and concentrated to 12.3 g of a brown solid. Chromatography on silica gel with 10% ethyl acetate/hexane followed by trituration with methanol afforded 5.51 g of the title compound as a brown solid, m.p. 97 to 99°C.

Step C: 2-(4-chlorophenyl)-3,4-dihydro-2-(hydroxymethyl)-1(2H)-naphthalenone

To 10 ml of ethanol was added a solution of 1.0 g of the title compound from Step B in 2 ml of toluene followed by 1.5 ml of 37% formaldehyde and then 7.8 ml of 0.5 M NaOH. The reaction was stirred at room temperature, under $N_2$, for four hours after which time TLC confirmed disappearance of the starting tetralone. The reaction was poured into a mixture of 100 ml 5% aqueous sodium bicarbonate and 100 ml of ethyl acetate. The aqueous extracts were washed twice with ethyl acetate, the organic extracts were then dried over magnesium sulfate, filtered and concentrated at reduced pressure. Chromatography on silica gel with 20% ethyl acetate/hexane afforded 1.19 g of a brown oil. $^1H$ NMR was consistent with the structure of the title compound.

Step D: 2-(4-chlorophenyl)-3,4-dihydro-2-[(methylsulfonyloxy)methyl]-1(2H)-naphthalenone

To a mixture of 0.99 g of the title compound of Step C and 0.6 ml of methanesulfonyl chloride in 20 ml of THF at 0°C was added 1.05 ml of triethylamine. The reaction was stirred overnight at room temperature, partitioned between 5% aqueous sodium bicarbonate and chloroform, dried over magnesium sulfate and concentrated. The residue was chromatographed on silica gel with 30% ethyl acetate/hexane to afford 1.1 g of the title compound as a viscous yellow oil.

Step E: 3a-(4-chlorophenyl)-3,3a,4,5-tetrahydro-N-[4-(trifluoromethyl)phenyl]-2H-benz[g]indazole-2-carboxamide

A mixture of 0.9 g of the title compound of Step D, 10 ml of ethanol and 0.4 ml of hydrazine hydrate was refluxed under $N_2$ for 4 days. The reaction was cooled to room temperature and the white precipitate

which formed was filtered. The filtrate was partitioned between saturated aqueous potassium bicarbonate and ethyl acetate. The organic extracts were dried over magnesium sulfate, filtered and concentrated at reduced pressure to afford 0.58 g of a gummy yellow solid. The crude residue was dissolved in 5 ml of THF and 0.4 ml of 4-trifluoromethylphenyl isocyanate was added. After 15 minutes TLC on silica gel (30% ethyl acetate/hexane) indicated formation of a fluorescent product. Concentration of the reaction mixture under reduced pressure and chromatography on silica gel with 20% ethyl acetate/hexane followed by chromatography on silica gel with 10% methylene chloride in n-butylchloride afforded after trituration with hexane 49.2 mg of a beige powder. $^1H$ NMR was consistent with the structure of the title compound. $^1H$ NMR ($CDCl_3$) δ 2.30 (dt, 1H), 2.5-2.9 (m, 3H), 3.90 (d, 1H), 4.33 (d, 1H), 7.14 (m, 3H), 7.2-7.4 (m, 3H), 7.56 (d, 2H), 7.65 (d, 2H), 8.11 (m, 1H), 8.28 (bs, 1H).

By the general procedures described above, or obvious modifications thereof, the compounds of Tables 1 through 18 can be prepared. The compounds can then be combined with suitable carriers, as will be obvious to one skilled in the art, to produce the compositions of this invention.

## General Structures for Tables 1-18

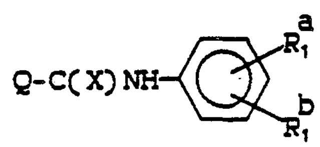

| Table | Q |
|---|---|
| 1 | Q-1 (J=K=H, $R_8$=H) |
| 2 | Q-2 (J=K=H, $R_8$=H) |
| 3 | Q-3 (J=K=H, $R_8$=H) |
| 4 | Q-4 (J=K=H, X=O, $X^1$=O) |
| 5 | Q-5 (J=K=H, X=O, $X^1$=O) |

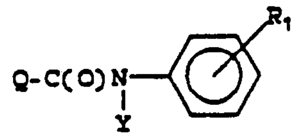

| | |
|---|---|
| 6 | Q-1 (J=K=H) |
| 7 | Q-2 (J=K=H) |
| 8 | Q-3 (J=K=H) |
| 9 | Q-4 (J=K=H, $X^1$=O) |
| 10 | Q-5 (J=K=H, $X^1$=O)) |

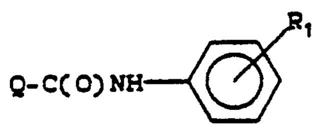

| | |
|---|---|
| 11 | Q-1 |
| 12 | Q-2 |
| 13 | Q-3 |
| 14 | Q-4 ($X^1$=O) |
| 15 | Q-5 ($X^1$=O) |

16 Q is 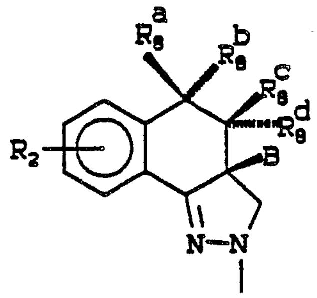

17 Q is 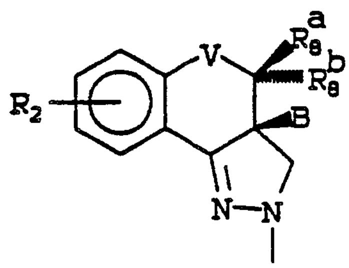

18 Q is 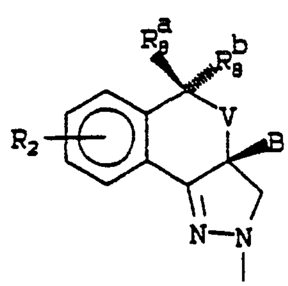

For the tables that follow, the values of Q will be numbered as shown:

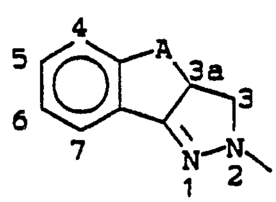

For Tables 16, 17 and 18 only one of the two possible enantiomers of Q is shown for clarity. For compounds in Tables 17 and 18, when V is SO, the α isomer represents compounds where the B substituent is cis to the oxygen of the sulfoxide. The β isomer represents those compounds where the B

substituent is trans to the oxygen of the sulfoxide.

In all the Tables, the last column contains melting points (in °C) for the designated compounds.

TABLE 1

| $R_1^a$ | $R_1^b$ | $R_2$ | B | x | t |
|---|---|---|---|---|---|
| H | H | H | H | 0 | 0 |
| 4-Cl | H | H | H | 0 | 0 |
| 4-F | H | H | H | 0 | 0 |
| 4-Br | H | H | H | 0 | 0 |
| $4-CF_3$ | H | H | H | 0 | 0 |
| $4-OCF_2H$ | H | H | H | 0 | 0 |
| $4-CF_3$ | 3-Cl | H | H | 0 | 0 |
| $4-CO_2Me$ | H | H | H | 0 | 0 |
| $4-CO_2-i-Pr$ | H | H | H | 0 | 0 |
| $4-NO_2$ | H | H | H | 0 | 0 |
| 4-SMe | H | H | H | 0 | 0 |
| $4-SO_2Me$ | H | H | H | 0 | 0 |
| $4-CF_2Cl$ | H | H | H | 0 | 0 |
| $4-OCF_3$ | H | H | H | 0 | 0 |
| 4-I | H | H | H | 0 | 0 |
| $4-OCF_2CF_2H$ | H | H | H | 0 | 0 |
| 4-CN | H | H | H | 0 | 0 |
| 4-Me | H | H | H | 0 | 0 |
| $3,4-CF_2CF_2O$ | | H | H | 0 | 0 |
| $3,4-OCF_2CF_2$ | | H | H | 0 | 0 |
| $3,4-(Me)_2CCH_2O$ | | H | H | 0 | 0 |
| 4-Cl | H | H | Me | 0 | 0 |
| 4-Br | H | H | Me | 0 | 0 |
| $4-CF_3$ | H | H | Me | 0 | 0 |
| 4-SMe | H | H | Me | 0 | 0 |
| $4-OCF_2H$ | H | H | Me | 0 | 0 |
| $4-CF_2Cl$ | H | H | Me | 0 | 0 |
| $4-CO_2i-Pr$ | H | H | Me | 0 | 0 |
| 4-Cl | H | H | allyl | 0 | 0 |
| 4-Br | H | H | allyl | 0 | 0 |
| $4-CF_3$ | H | H | allyl | 0 | 0 |
| 4-Cl | H | H | $CH_2Ph$ | 0 | 0 |
| 4-Br | H | H | $CH_2Ph$ | 0 | 0 |
| $4-CF_3$ | H | H | $CH_2Ph$ | 0 | 0 |

| $R_1^a$ | $R_1^b$ | $R_2$ | B | x | t |
|---|---|---|---|---|---|
| 4-$CF_3$ | 3-Cl | H | 4-F-$CH_2$Ph | O | 0 |
| 4-SMe | H | H | 4-F-$CH_2$Ph | O | 0 |
| 4-Cl | H | H | 4-F-$CH_2$Ph | O | 0 |
| 4-F | H | H | 4-Cl-$CH_2$Ph | O | 0 |
| 4-$NO_2$ | H | H | 4-Cl-$CH_2$Ph | O | 0 |
| 4-$OCF_2H$ | H | H | Ph | O | 0 |
| 4-$OCF_3$ | H | H | Ph | O | 0 |
| 4-$CF_3$ | H | H | Ph | O | 0 |
| 4-Cl | 3-Cl | H | 4-Cl-Ph | O | 0 |
| 4-Br | H | H | 4-Cl-Ph | O | 0 |
| 4-OMe | H | H | 4-Cl-Ph | O | 0 |
| 4-$CF_3$ | H | H | n-Bu | O | 0 |
| 4-Cl | H | H | n-Bu | O | 0 |
| 4-$CF_3$ | H | H | $(CH_2)_3Cl$ | O | 0 |
| 4-Cl | H | H | $(CH_2)_3Cl$ | O | 0 |
| 4-Br | H | H | $CH_2CF_3$ | O | 0 |
| 4-$OCF_3$ | H | H | $CH_2CF_3$ | O | 0 |
| 4-$SCF_2H$ | H | H | $CH_2OMe$ | O | 0 |
| 4-$CF_3$ | H | H | $CH_2OEt$ | O | 0 |
| 4-Cl | H | H | $CH_2CO_2Me$ | O | 0 |
| 4-Br | H | H | $CH_2CO_2Me$ | O | 0 |
| 4-$OCF_2H$ | H | H | $CH_2CN$ | O | 0 |
| 4-$OCF_2CF_2H$ | H | H | $(CH_2)_2CN$ | O | 0 |
| 4-$CF_3$ | H | H | $CO_2Me$ | O | 0 |
| 4-Cl | H | H | $CO_2Me$ | O | 0 |
| 4-Br | H | H | $CO_2Me$ | O | 0 |
| 4-F | H | H | $CO_2Me$ | O | 0 |
| 4-$CF_2Cl$ | H | H | $CO_2Me$ | O | 0 |
| 4-$OCF_3$ | H | H | $CO_2Me$ | O | 0 |

| $R_1^a$ | $R_1^b$ | $R_2$ | B | x | t |
|---|---|---|---|---|---|
| 3,4-$CF_2CF_2O$* | | H | $CO_2Me$ | 0 | 0 |
| 3,4-$OCF_2CF_2$ | | H | $CO_2Me$ | 0 | 0 |
| 3,4-$(Me)_2CCH_2O$ | | H | $CO_2Me$ | 0 | 0 |
| 4-$CF_3$ | H | H | $CO_2Et$ | 0 | 0 |
| 4-Br | H | H | $CO_2Et$ | 0 | 0 |
| 4-$CF_3$ | H | H | $CO_2H$ | 0 | 0 |
| 4-Cl | H | H | $CO_2H$ | 0 | 0 |
| 4-$CF_3$ | H | H | $CO_2Ph$ | 0 | 0 |
| 4-F | H | H | $CO_2Ph$ | 0 | 0 |
| 4-$CF_3$ | H | H | $CO_2CH_2CF_3$ | 0 | 0 |
| 4-$CF_3$ | H | H | $CO_2CH_2CF_3$ | 0 | 0 |
| 4-$CF_3$ | H | H | $CO_2CH_2CN$ | 0 | 0 |
| 4-$CF_3$ | H | H | $CO_2CH_2CN$ | 0 | 0 |
| 4-$CF_3$ | H | H | $CO_2$n-$C_6H_{13}$ | 0 | 0 |
| 4-$CF_3$ | H | H | $CO_2$allyl | 0 | 0 |
| 4-$CF_3$ | H | H | $CO_2CH_2Ph$ | 0 | 0 |
| 4-$CF_3$ | H | H | $CO_2CH_2SMe$ | 0 | 0 |
| 4-$CF_3$ | H | H | $C(O)NH_2$ | 0 | 0 |
| 4-Cl | H | H | $C(O)NH_2$ | 0 | 0 |
| 4-Br | H | H | C(O)NHMe | 0 | 0 |
| 4-$OCF_3$ | H | H | C(O)NHMe | 0 | 0 |
| 4-$CF_3$ | H | H | $C(O)NMe_2$ | 0 | 0 |
| 4-Cl | H | H | $C(O)NMe_2$ | 0 | 0 |
| 4-Br | H | H | C(O)NHEt | 0 | 0 |
| 4-$OCF_3$ | H | H | $C(S)NH_2$ | 0 | 0 |
| 4-$CF_3$ | H | H | C(S)NHMe | 0 | 0 |
| 4-Cl | H | H | $C(S)NMe_2$ | 0 | 0 |

* as a representative example this substitution pattern designates

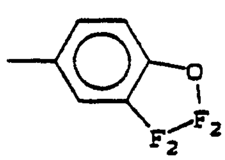

| $R_1^a$ | $R_1^b$ | $R_2$ | B | x | t | |
|---|---|---|---|---|---|---|
| 4-Br | H | H | C(S)Me | O | 0 | |
| 4-$OCF_3$ | H | H | C(S)Et | O | 0 | |
| 4-$CF_3$ | H | H | C(S)$NMe_2$ | O | 0 | |
| 4-Cl | H | H | C(S)NHMe | O | 0 | |
| 4-$OCF_3$ | H | H | OH | O | 0 | |
| 4-$CF_3$ | H | H | OH | O | 0 | |
| 4-Cl | H | H | OH | O | 0 | |
| 4-Br | H | H | OH | O | 0 | |
| 4-$OCF_2H$ | H | H | OH | O | 0 | |
| 4-$OCF_3$ | H | H | OMe | O | 0 | |
| 4-$CF_3$ | H | H | OMe | O | 0 | |
| 4-Cl | H | H | OMe | O | 0 | |
| 4-Br | H | H | O-allyl | O | 0 | |
| 4-$OCF_2H$ | H | H | O-allyl | O | 0 | |
| 4-$OCF_3$ | H | H | O-allyl | O | 0 | |
| 4-$CF_3$ | H | H | OAc | O | 0 | |
| 4-Cl | H | H | OAc | O | 0 | |
| 4-Br | H | H | OCONHMe | O | 0 | |
| 4-$OCF_2H$ | H | H | $OCONMe_2$ | O | 0 | |
| 4-$OCF_3$ | H | H | $OCO_2Me$ | O | 0 | |
| 4-$CF_3$ | H | H | $OCO_2Ph$ | O | 0 | |
| 4-Cl | H | H | $OSO_2Me$ | O | 0 | |
| 4-Br | H | H | $OSO_2Me$ | O | 0 | |
| 4-$OCF_2H$ | H | H | $OCH_2Ph$ | O | 0 | |
| 4-$OCF_3$ | H | H | $OCH_2Ph$ | O | 0 | |
| H | H | H | H | O | 1 | |
| 4-Cl | H | H | H | O | 1 | 91.5 to 96 |
| 4-F | H | H | H | O | 1 | |
| 4-Br | H | H | H | O | 1 | 151 to 152 |
| 4-$CF_3$ | H | H | H | O | 1 | 149 to 151 |
| 4-$OCF_2H$ | H | H | H | O | 1 | |
| 4-$CF_3$ | 3-Cl | H | H | O | 1 | |
| 4-$CO_2Me$ | H | H | H | O | 1 | |
| 4-$CO_2$-i-Pr | H | H | H | O | 1 | |

| $R_1^a$ | $R_1^b$ | $R_2$ | B | x | t | |
|---|---|---|---|---|---|---|
| 4-$NO_2$ | H | H | H | O | 1 | |
| 4-SMe | H | H | H | O | 1 | |
| 4-$SO_2$Me | H | H | H | O | 1 | |
| 4-$CF_2$Cl | H | H | H | O | 1 | |
| 4-$OCF_3$ | H | H | H | O | 1 | 104 to 107 |
| 4-I | H | H | H | O | 1 | |
| 4-$OCF_2CF_2H$ | H | H | H | O | 1 | |
| 4-CN | H | H | H | O | 1 | |
| 4-Me | H | H | H | O | 1 | |
| 3,4-$CF_2CF_2O$ | | H | H | O | 1 | |
| 3,4-$OCF_2CF_2$ | | H | H | O | 1 | |
| 3,4-$(Me)_2CCH_2O$ | | H | H | O | 1 | |
| 4-Cl | H | H | Me | O | 1 | |
| 4-Br | H | H | Me | O | 1 | |
| 4-$CF_3$ | H | H | Me | O | 1 | 151.5 to 153.5 |
| 4-SMe | H | H | Me | O | 1 | |
| 4-$OCF_2H$ | H | H | Me | O | 1 | |
| 4-$CF_3$ | H | H | Et | O | 1 | 120 to 123 |
| 4-$CO_2$i-Pr | H | H | Me | O | 1 | |
| 4-Cl | H | H | allyl | O | 1 | |
| 4-Br | H | H | allyl | O | 1 | |
| 4-$CF_3$ | H | H | allyl | O | 1 | 158 to 163 |
| 4-Cl | H | H | $CH_2Ph$ | O | 1 | |
| 4-Br | H | H | $CH_2Ph$ | O | 1 | |
| 4-$CF_3$ | H | H | $CH_2Ph$ | O | 1 | 51 to 55 |
| 4-$CF_3$ | 3-Cl | H | 4-F-$CH_2Ph$ | O | 1 | |
| 4-SMe | H | H | 4-F-$CH_2Ph$ | O | 1 | |
| 4-$CF_3$ | H | H | 4-F-$CH_2Ph$ | O | 1 | |
| 4-$CF_3$ | H | H | 4-Cl-$CH_2Ph$ | O | 1 | gum |
| 4-$NO_2$ | H | H | 4-Cl-$CH_2Ph$ | O | 1 | |
| 4-$OCF_2H$ | H | H | Ph | O | 1 | |
| 4-$OCF_3$ | H | H | Ph | O | 1 | |

| $R_1^a$ | $R_1^b$ | $R_2$ | B | x | t | |
|---|---|---|---|---|---|---|
| 4-$CF_3$ | H | H | Ph | O | 1 | |
| 4-Cl | 3-Cl | H | 4-Cl-Ph | O | 1 | |
| 4-Br | H | H | 4-Cl-Ph | O | 1 | 185 to 187 |
| 4-OMe | H | H | 4-Cl-Ph | O | 1 | |
| 4-$CF_3$ | H | H | n-Bu | O | 1 | |
| 4-Cl | H | H | n-Bu | O | 1 | |
| 4-$CF_3$ | H | H | $(CH_2)_3Cl$ | O | 1 | |
| 4-Cl | H | H | $(CH_2)_3Cl$ | O | 1 | |
| 4-Br | H | H | $CH_2CF_3$ | O | 1 | |
| 4-$OCF_3$ | H | H | $CH_2CF_3$ | O | 1 | |
| 4-$SCF_2H$ | H | H | $CH_2OMe$ | O | 1 | |
| 4-$CF_3$ | H | H | $CH_2OEt$ | O | 1 | |
| 4-Cl | H | H | $CH_2CO_2Me$ | O | 1 | |
| 4-Br | H | H | $CH_2CO_2Me$ | O | 1 | |
| 4-$OCF_2H$ | H | H | $CH_2CN$ | O | 1 | |
| 4-$OCF_2CF_2H$ | H | H | $(CH_2)_2CN$ | O | 1 | |
| 4-$CF_3$ | H | H | $(CH_2)_2CN$ | O | 1 | 165 to 169 |
| 4-$CF_3$ | H | H | $(CH_2)_3CN$ | O | 1 | 186 to 188 |
| 4-$CF_3$ | H | H | $CO_2Me$ | O | 1 | 177 to 180 |
| 4-Cl | H | H | $CO_2Me$ | O | 1 | 175 to 177 |
| 4-Br | H | H | $CO_2Me$ | O | 1 | 175 to 178 |
| 4-F | H | H | $CO_2Me$ | O | 1 | |
| 4-$CF_2Cl$ | H | H | $CO_2Me$ | O | 1 | |
| 4-$OCF_3$ | H | H | $CO_2Me$ | O | 1 | |
| 3,4-$CF_2CF_2O$ | | H | $CO_2Me$ | O | 1 | |
| 3,4-$OCF_2CF_2O$ | | H | $CO_2Me$ | O | 1 | |
| 3,4-$(Me)_2CCH_2O$ | | H | $CO_2Me$ | O | 1 | |
| 4-$CF_3$ | H | H | $CO_2Et$ | O | 1 | 220 to 222 |
| 4-Br | H | H | $CO_2Et$ | O | 1 | 179 to 182 |
| 4-Cl | H | H | $CO_2Et$ | O | 1 | 172 to 174 |
| 4-$CF_3$ | H | H | $CO_2H$ | O | 1 | 182 to 183 |

| $R_1^a$ | $R_1^b$ | $R_2$ | B | x | t | |
|---|---|---|---|---|---|---|
| 4-$CF_3$ | H | H | $CO_2Na$ | O | 1 | 230 |
| 4-$CF_3$ | H | H | $CO_2Ph$ | O | 1 | |
| 4-F | H | H | $CO_2Ph$ | O | 1 | |
| 4-$CF_3$ | H | H | $CO_2CH_2CCl_3$ | O | 1 | 194 to 196 |
| 4-$CF_3$ | H | H | $CO_2CH_2CF_3$ | O | 1 | 160 to 163 |
| 4-$CF_3$ | H | H | $CO_2(CH_2)Cl$ | O | 1 | 185 to 188 |
| 4-$CF_3$ | H | H | $CO_2(CH_2)Br$ | O | 1 | 162 to 167 |
| 4-$CF_3$ | H | H | $CO_2CH_2CN$ | O | 1 | |
| 4-$CF_3$ | H | H | $CO_2n\text{-}C_6H_{13}$ | O | 1 | |
| 4-$CF_3$ | H | H | $CO_2$allyl | O | 1 | |
| 4-$CF_3$ | H | H | $CO_2CH_2Ph$ | O | 1 | |
| 4-$CF_3$ | H | H | $CO_2CH_2SMe$ | O | 1 | |
| 4-$CF_3$ | H | H | $C(O)NH_2$ | O | 1 | 250 |
| 4-Cl | H | H | $C(O)NH_2$ | O | 1 | |
| 4-Br | H | H | C(O)NHMe | O | 1 | |
| 4-$OCF_3$ | H | H | C(O)NHMe | O | 1 | |
| 4-$CF_3$ | H | H | $C(O)NMe_2$ | O | 1 | 226 to 230 |
| 4-Cl | H | H | $C(O)NMe_2$ | O | 1 | |
| 4-Br | H | H | C(O)NHEt | O | 1 | |
| 4-$CF_3$ | H | H | C(O)NHMe | O | 1 | 220 |
| 4-$CF_3$ | H | H | C(O)NHallyl | O | 1 | 250 |
| 4-$CF_3$ | H | H | C(O)N(Me)allyl | O | 1 | 219 to 221 |
| 4-$CF_3$ | H | H | C(O)NHPh-4-Cl | O | 1 | 255 |
| 4-$CF_3$ | H | H | C(O)-piperidinyl | O | 1 | >235 |
| 4-$OCF_3$ | H | H | $C(S)NH_2$ | O | 1 | |
| 4-$CF_3$ | H | H | C(S)NHMe | O | 1 | |
| 4-Cl | H | H | $C(S)NMe_2$ | O | 1 | |
| 4-Br | H | H | C(S)Me | O | 1 | |
| 4-$OCF_3$ | H | H | C(S)Et | O | 1 | |

| $R_1^a$ | $R_1^b$ | $R_2$ | B | X | t | |
|---|---|---|---|---|---|---|
| 4-$CF_3$ | H | H | C(S)$NMe_2$ | O | 1 | |
| 4-Cl | H | H | C(S)NHMe | O | 1 | |
| 4-$OCF_3$ | H | H | OH | O | 1 | |
| 4-$CF_3$ | H | H | OH | O | 1 | |
| 4-Cl | H | H | OH | O | 1 | |
| 4-Br | H | H | OH | O | 1 | |
| 4-$OCF_2H$ | H | H | OH | O | 1 | |
| 4-$OCF_3$ | H | H | OMe | O | 1 | |
| 4-$CF_3$ | H | H | OMe | O | 1 | |
| 4-Cl | H | H | OMe | O | 1 | |
| 4-Br | H | H | O-allyl | O | 1 | |
| 4-$OCF_2H$ | H | H | O-allyl | O | 1 | |
| 4-$OCF_3$ | H | H | O-allyl | O | 1 | |
| 4-$CF_3$ | H | H | OAc | O | 1 | |
| 4-Cl | H | H | OAc | O | 1 | |
| 4-$OCF_3$ | H | H | $OCO_2Me$ | O | 1 | |
| 4-$CF_3$ | H | H | $OCO_2Ph$ | O | 1 | |
| 4-Cl | H | H | $OSO_2Me$ | O | 1 | |
| 4-Br | H | H | $OSO_2Me$ | O | 1 | |
| 4-$OCF_2H$ | H | H | $OCH_2Ph$ | O | 1 | |
| 4-$OCF_3$ | H | H | $OCH_2Ph$ | O | 1 | |
| H | H | H | H | O | 2 | |
| 4-Cl | H | H | H | O | 2 | 136.5 to 138 |
| 4-F | H | H | H | O | 2 | |
| 4-Br | H | H | H | O | 2 | 142 to 144 |
| 4-$CF_3$ | H | H | H | O | 2 | 167 to 168.5 |
| 4-$OCF_2H$ | H | H | H | O | 2 | |
| 4-$CF_3$ | 3-Cl | H | H | O | 2 | |
| 4-$CO_2Me$ | H | H | H | O | 2 | |
| 4-$CO_2$-i-Pr | H | H | H | O | 2 | |
| 4-$NO_2$ | H | H | H | O | 2 | |
| 4-SMe | H | H | H | O | 2 | |
| 4-$SO_2Me$ | H | H | H | O | 2 | |

| $R_1^a$ | $R_1^b$ | $R_2$ | B | X | t | |
|---|---|---|---|---|---|---|
| 4-$CF_2Cl$ | H | H | H | O | 2 | |
| 4-$OCF_3$ | H | H | H | O | 2 | |
| 4-I | H | H | H | O | 2 | |
| 4-$OCF_2CF_2H$ | H | H | H | O | 2 | |
| 4-CN | H | H | H | O | 2 | |
| 4-Me | H | H | H | O | 2 | |
| 4-Cl | H | H | Me | O | 2 | 137 to 141.5 |
| 4-Br | H | H | Me | O | 2 | |
| 4-$CF_3$ | H | H | Me | O | 2 | 134 to 139 |
| 4-SMe | H | H | Me | O | 2 | |
| 4-$OCF_2H$ | H | H | Me | O | 2 | |
| 4-$CF_2Cl$ | H | H | Me | O | 2 | |
| 4-$CO_2$i-Pr | H | H | Me | O | 2 | |
| 4-Cl | H | H | allyl | O | 2 | |
| 4-Br | H | H | allyl | O | 2 | |
| 4-$CF_3$ | H | H | allyl | O | 2 | |
| 4-Cl | H | H | $CH_2Ph$ | O | 2 | |
| 4-Br | H | H | $CH_2Ph$ | O | 2 | |
| 4-$CF_3$ | H | H | $CH_2Ph$ | O | 2 | |
| 4-$CF_3$ | 3-Cl | H | 4-F-$CH_2Ph$ | O | 2 | |
| 4-SMe | H | H | 4-F-$CH_2Ph$ | O | 2 | |
| 4-Cl | H | H | 4-F-$CH_2Ph$ | O | 2 | |
| 4-F | H | H | 4-Cl-$CH_2Ph$ | O | 2 | |
| 4-$NO_2$ | H | H | 4-Cl-$CH_2Ph$ | O | 2 | |
| 4-$OCF_2H$ | H | H | Ph | O | 2 | |
| 4-$OCF_3$ | H | H | Ph | O | 2 | |
| 4-$CF_3$ | H | H | Ph | O | 2 | |
| 4-Cl | 3-Cl | H | 4-Cl-Ph | O | 2 | |
| 4-Br | H | H | 4-Cl-Ph | O | 2 | |
| 4-OMe | H | H | 4-Cl-Ph | O | 2 | |
| 4-$CF_3$ | H | H | n-Bu | O | 2 | |
| 4-Cl | H | H | n-Bu | O | 2 | |
| 4-$CF_3$ | H | H | $(CH_2)_3Cl$ | O | 2 | |
| 4-Cl | H | H | $(CH_2)_3Cl$ | O | 2 | |

| $R_1^a$ | $R_1^b$ | $R_2$ | B | x | t | |
|---|---|---|---|---|---|---|
| 4-Br | H | H | $CH_2CF_3$ | O | 2 | |
| 4-$OCF_3$ | H | H | $CH_2CF_3$ | O | 2 | |
| 4-$SCF_2H$ | H | H | $CH_2OMe$ | O | 2 | |
| 4-$CF_3$ | H | H | $CH_2OEt$ | O | 2 | |
| 4-Cl | H | H | $CH_2CO_2Me$ | O | 2 | |
| 4-Br | H | H | $CH_2CO_2Me$ | O | 2 | |
| 4-$OCF_2H$ | H | H | $CH_2CN$ | O | 2 | |
| 4-$OCF_2CF_2H$ | H | H | $(CH_2)_2CN$ | O | 2 | |
| 4-$CF_3$ | H | H | $CO_2Me$ | O | 2 | 115 to 117 |
| 4-Cl | H | H | $CO_2Me$ | O | 2 | 173 to 176 |
| 4-Br | H | H | $CO_2Me$ | O | 2 | 184 to 186 |
| 4-F | H | H | $CO_2Me$ | O | 2 | |
| 4-$CF_2Cl$ | H | H | $CO_2Me$ | O | 2 | |
| 4-$OCF_3$ | H | H | $CO_2Me$ | O | 2 | |
| 4-$CF_3$ | H | H | $CO_2Et$ | O | 2 | |
| 4-Br | H | H | $CO_2Et$ | O | 2 | |
| 4-$CF_3$ | H | H | $CO_2H$ | O | 2 | |
| 4-Cl | H | H | $CO_2H$ | O | 2 | |
| 4-$CF_3$ | H | H | $CO_2Ph$ | O | 2 | |
| 4-F | H | H | $CO_2Ph$ | O | 2 | |
| 4-$CF_3$ | H | H | $CO_2CH_2CF_3$ | O | 2 | |
| 4-$CF_3$ | H | H | $CO_2CH_2CF_3$ | O | 2 | |
| 4-$CF_3$ | H | H | $CO_2CH_2CN$ | O | 2 | |
| 4-$CF_3$ | H | H | $CO_2CH_2CN$ | O | 2 | |
| 4-$CF_3$ | H | H | $CO_2n$-$C_6H_{13}$ | O | 2 | |
| 4-$CF_3$ | H | H | $CO_2$allyl | O | 2 | |
| 4-$CF_3$ | H | H | $CO_2CH_2Ph$ | O | 2 | |
| 4-$CF_3$ | H | H | $CO_2CH_2SMe$ | O | 2 | |
| 4-$CF_3$ | H | H | $C(O)NH_2$ | O | 2 | |

| $R_1^a$ | $R_1^b$ | $R_2$ | B | x | t |
|---|---|---|---|---|---|
| 4-Cl | H | H | $C(O)NH_2$ | O | 2 |
| 4-Br | H | H | C(O)NHMe | O | 2 |
| $4-OCF_3$ | H | H | C(O)NHMe | O | 2 |
| $4-CF_3$ | H | H | $C(O)NMe_2$ | O | 2 |
| 4-Cl | H | H | $C(O)NMe_2$ | O | 2 |
| 4-Br | H | H | C(O)NHEt | O | 2 |
| $4-OCF_3$ | H | H | $C(S)NH_2$ | O | 2 |
| $4-CF_3$ | H | H | C(S)NHMe | O | 2 |
| 4-Cl | H | H | $C(S)NMe_2$ | O | 2 |
| 4-Br | H | H | C(S)Me | O | 2 |
| $4-OCF_3$ | H | H | C(S)Et | O | 2 |
| $4-CF_3$ | H | H | $C(S)NMe_2$ | O | 2 |
| 4-Cl | H | H | C(S)NHMe | O | 2 |
| $4-OCF_3$ | H | H | OH | O | 2 |
| $4-CF_3$ | H | H | OH | O | 2 |
| 4-Cl | H | H | OH | O | 2 |
| 4-Br | H | H | OH | O | 2 |
| $4-OCF_2H$ | H | H | OH | O | 2 |
| $4-OCF_3$ | H | H | OMe | O | 2 |
| $4-CF_3$ | H | H | OMe | O | 2 |
| 4-Cl | H | H | OMe | O | 2 |
| 4-Br | H | H | O-allyl | O | 2 |
| $4-OCF_2H$ | H | H | O-allyl | O | 2 |
| $4-OCF_3$ | H | H | O-allyl | O | 2 |
| $4-CF_3$ | H | H | OAc | O | 2 |
| 4-Cl | H | H | OAc | O | 2 |
| 4-Br | H | H | OCONHMe | O | 2 |
| $4-OCF_2H$ | H | H | $OCONMe_2$ | O | 2 |
| $4-OCF_3$ | H | H | $OCO_2Me$ | O | 2 |
| $4-CF_3$ | H | H | $OCO_2Ph$ | O | 2 |
| 4-Cl | H | H | $OSO_2Me$ | O | 2 |
| 4-Br | H | H | $OSO_2Me$ | O | 2 |

| $R_1^a$ | $R_1^b$ | $R_2$ | B | x | t | |
|---|---|---|---|---|---|---|
| 4-$OCF_2H$ | H | H | $OCH_2Ph$ | 0 | 2 | |
| 4-$OCF_3$ | H | H | $OCH_2Ph$ | 0 | 2 | |
| 4-$CF_3$ | H | 5-Cl | H | 0 | 0 | |
| 4-$OCF_3$ | H | 4-Cl | Me | 0 | 0 | |
| 4-$OCF_2H$ | H | 5-Cl | $CO_2Me$ | 0 | 0 | |
| 4-Cl | H | 5-Cl | $CO_2CH_2CH_2Cl$ | 0 | 0 | |
| 4-Br | H | 5-Cl | H | 0 | 1 | 164 to 166 |
| 4-$CF_3$ | H | 5-Cl | Me | 0 | 1 | |
| 4-$OCF_3$ | H | 5-Cl | Et | 0 | 1 | |
| 4-Cl | H | 5-Cl | H | 0 | 1 | 155 to 157 |
| 4-SMe | H | 5-Cl | H | 0 | 1 | 159 to 161 |
| 4-$CO_2Et$ | H | 5-Cl | H | 0 | 1 | 153 to 156 |
| 4-Br | H | 5-Cl | $CO_2Me$ | 0 | 1 | 234 to 236 |
| 3-SMe | H | 5-Cl | $CO_2Me$ | 0 | 1 | 202 to 204 |
| 4-$CO_2Et$ | H | 5-Cl | $CO_2Me$ | 0 | 1 | 197 to 199 |
| 4-$OCF_2H$ | H | 5-Cl | $CO_2Me$ | 0 | 1 | |
| 4-Cl | H | 5-Cl | $CO_2Me$ | 0 | 1 | 222 to 233 |
| 4-Br | H | 5-Cl | C(O)Me | 0 | 1 | |
| 4-$CF_3$ | H | 5-Cl | C(O)Et | 0 | 1 | |
| 4-$OCF_3$ | H | 5-Cl | $C(O)NMe_2$ | 0 | 1 | |
| 4-$OCF_2H$ | H | 5-Cl | OH | 0 | 1 | |
| 4-Cl | H | 5-Cl | OAc | 0 | 1 | |
| 4-Br | H | 5-Cl | H | 0 | 2 | |
| 4-$CF_3$ | H | 5-Cl | Me | 0 | 2 | |
| 4-$OCF_3$ | H | 5-Cl | $CO_2Me$ | 0 | 2 | |
| 4-$OCF_2H$ | H | 5-Cl | $CO_2$-n-Bu | 0 | 2 | |
| 4-Cl | H | 5-Cl | $CH_2Ph$ | 0 | 2 | |
| 4-Br | H | 5-Cl | C(O)NHPh | 0 | 2 | |
| 4-$CF_3$ | H | 5-Cl | OMe | 0 | 2 | |
| 4-$OCF_3$ | H | 5-Cl | OC(O)NHMe | 0 | 2 | |
| 4-$CF_3$ | H | 5-F | $CO_2Me$ | 0 | 0 | |
| 4-$OCF_3$ | H | 5-F | $CO_2Me$ | 0 | 0 | |
| 4-$OCF_2H$ | H | 5-F | $CO_2Me$ | 0 | 0 | |
| 4-$CF_3$ | H | 5-F | H | 0 | 1 | 175 to 178 |

| $R_1^a$ | $R_1^b$ | $R_2$ | B | x | t | |
|---|---|---|---|---|---|---|
| 4-Cl | H | 5-F | H | O | 1 | |
| 4-Br | H | 5-F | H | O | 1 | |
| 4-$OCF_2H$ | H | 5-F | H | O | 1 | |
| 4-Cl | H | 5-F | $CO_2Me$ | O | 1 | |
| 4-Br | H | 5-F | $CO_2Me$ | O | 1 | |
| 4-$CF_3$ | H | 5-F | $CO_2Me$ | O | 1 | 223 to 225 |
| 4-$OCF_3$ | H | 5-F | $CO_2Me$ | O | 2 | |
| 4-$OCF_2H$ | H | 5-F | $CO_2Me$ | O | 2 | |
| 4-Cl | H | 5-F | $CO_2Me$ | O | 2 | |
| 4-Br | H | 5-$OCF_2H$ | $CO_2Me$ | O | 0 | |
| 4-$CF_3$ | H | 5-$OCF_2H$ | $CO_2Me$ | O | 0 | |
| 4-$OCF_3$ | H | 5-$OCF_2H$ | $CO_2Me$ | O | 0 | |
| 4-$OCF_2H$ | H | 5-$OCF_2H$ | $CO_2Me$ | O | 1 | |
| 4-Cl | H | 5-$OCF_2H$ | $CO_2Me$ | O | 1 | |
| 4-Br | H | 5-$OCF_2H$ | $CO_2Me$ | O | 1 | |
| 4-$CF_3$ | H | 5-$OCF_2H$ | $CO_2Me$ | O | 2 | |
| 4-$OCF_3$ | H | 5-$OCF_2H$ | $CO_2Me$ | O | 2 | |
| 4-$OCF_2H$ | H | 5-$OCF_2H$ | $CO_2Me$ | O | 2 | |
| 4-Cl | H | 5-Br | $CO_2Me$ | O | 1 | |
| 4-Br | H | 5-$NO_2$ | $CO_2Me$ | O | 1 | |
| 4-$CF_3$ | H | 5-$CF_3$ | $CO_2Me$ | O | 1 | |
| 4-$OCF_3$ | H | 5-$OCH_3$ | $CO_2Me$ | O | 1 | |
| 4-$CF_3$ | H | 5-$OCH_2Ph$ | Me | O | 1 | |
| 4-$CF_3$ | H | 5-C(O)Me | Et | O | 1 | |
| 4-$CF_3$ | H | 5-$OCF_2CF_2H$ | $CO_2Me$ | O | 1 | |
| 4-$CF_3$ | H | 5-I | Me | O | 1 | |
| 4-$CF_3$ | H | 5-$OCF_3$ | Et | O | 1 | |
| 4-$CF_3$ | H | 5-Et | C(O)Me | O | 1 | |
| 4-$CF_3$ | H | 5-Me | C(O)$CF_3$ | O | 1 | |
| 4-$CF_3$ | H | 5-CN | Me | O | 1 | |
| 4-$CF_3$ | H | 5-OMe | $CO_2Me$ | O | 1 | 180 to 186 |

| $R_1^a$ | $R_1^b$ | $R_2$ | B | x | t | |
|---|---|---|---|---|---|---|
| 4-$CF_3$ | H | 5-OMe | $CO_2Et$ | O | 1 | 160 to 166 |
| 4-$CF_3$ | H | 5-OMe | H | O | 1 | 158 to 160 |
| 4-Cl | H | 5-OMe | H | O | 1 | 169 to 171 |
| 4-$CF_3$ | H | 5-$CO_2Me$ | n-$C_6H_{13}$ | O | 1 | |
| 4-$CF_3$ | H | 5-$SCF_2H$ | C(O)H | O | 1 | |
| 4-$CF_3$ | H | 5-$SO_2Me$ | $(CH_2)_4Cl$ | O | 1 | |
| 4-$CF_3$ | H | 5,6-di-Cl | H | O | 1 | 229 to 230 |
| 4-$CF_3$ | H | 5,6-di-Cl | $CO_2Me$ | O | 1 | 238 to 241 |
| 4-$CF_3$ | H | 4-F | H | O | 1 | 183 to 184 |
| 4-Cl | H | 4-F | $CO_2Me$ | O | 1 | |
| 4-Br | H | 4-F | $CO_2Me$ | O | 1 | |
| 4-$CF_3$ | H | 4-Cl | H | O | 1 | 179 to 180 |
| 4-$OCF_2H$ | H | 4-Cl | H | O | 1 | |
| 4-$OCF_2H$ | H | 4-Cl | $CO_2Me$ | O | 1 | |
| 4-$CF_3$ | H | 4-Br | H | O | 1 | |
| 4-$CF_3$ | H | 4-Br | $CO_2Me$ | O | 1 | |
| 4-$CF_3$ | H | 6-Cl | $CO_2Me$ | O | 1 | 212 to 214 |
| 4-$CF_3$ | H | 6-F | $CO_2Me$ | O | 1 | |
| 4-$CF_3$ | H | 6-$CF_3$ | $CO_2Me$ | O | 1 | |
| 4-$CF_3$ | H | 4-Cl | $CO_2Me$ | O | 1 | |
| 4-$CF_3$ | H | 4-F | $CO_2Me$ | O | 1 | 206 to 212 |
| 4-$CF_3$ | H | 6-Br | $CO_2Me$ | O | 1 | |
| 4-$CF_3$ | H | 6-$NO_2$ | $CO_2Me$ | O | 1 | |
| 4-$CF_3$ | H | 6-Me | $CO_2Me$ | O | 1 | |
| 4-$CF_3$ | H | 4-Cl | $CO_2Me$ | O | 1 | 209 to 211 |
| 4-$CF_3$ | H | 4-F | $CO_2Me$ | O | 1 | |
| 4-$CF_3$ | H | 4-Br | $CO_2Me$ | O | 1 | |
| 4-$CF_3$ | H | H | H | S | 0 | |
| 4-Cl | H | H | Me | S | 0 | |
| 4-$CF_3$ | H | H | $CO_2Me$ | S | 0 | |
| 4-Cl | H | H | H | S | 1 | |
| 4-$CF_3$ | H | H | Me | S | 1 | |

| $R_1^a$ | $R_1^b$ | $R_2$ | B | x | t | |
|---|---|---|---|---|---|---|
| 4-Cl | H | H | $CO_2Me$ | S | 1 | |
| 4-$CF_3$ | H | H | H | S | 2 | |
| 4-Cl | H | H | Me | S | 2 | |
| 4-$CF_3$ | H | H | $CO_2Me$ | S | 2 | |
| 4-Cl | H | 5-Cl | H | S | 1 | |
| 4-$CF_3$ | H | 5-Cl | Me | S | 1 | |
| 4-Cl | H | 5-Cl | $CO_2Me$ | S | 1 | |
| 4-$CF_3$ | H | 5-F | H | S | 1 | |
| 4-Cl | H | 5-F | Me | S | 1 | |
| 4-$CF_3$ | H | 5-F | $CO_2Me$ | S | 1 | |
| 4-Cl | H | H | 4-Cl-Ph | O | 1 | 171 to 173 |
| 4-$CF_3$ | H | 5-Cl | 4-Cl-Ph | O | 1 | 226 to 228 |
| 4-Br | H | 5-Cl | 4-Cl-Ph | O | 1 | 208 to 210 |
| 4-$CF_3$ | H | H | 4-F-Ph | O | 1 | 209 to 211 |
| 4-$CF_3$ | H | 6-F | H | O | 1 | 165 to 166 |
| 4-Cl | H | 6-F | H | O | 1 | 157 to 159 |
| 4-$CF_3$ | H | 4,5-Di-Cl | H | O | 1 | 215 to 216 |
| 4-$CF_3$ | H | 4-OMe | H | O | 1 | 190 to 191 |
| 4-Cl | H | 4-OMe | H | O | 1 | 199 to 201 |
| 4-Br | H | 4-OMe | H | O | 1 | 207 to 209 |
| 4-$CF_3$ | H | 4-OMe | $CO_2Me$ | O | 1 | >240 |
| 4-Cl | H | 4-OMe | $CO_2Me$ | O | 1 | 213 to 215 |
| 4-Br | H | 4-OMe | $CO_2Me$ | O | 1 | 234 to 235 |
| 4-$CF_3$ | H | 6-OMe | H | O | 1 | 83 to 84 |
| 4-Cl | H | 6-OMe | H | O | 1 | 143 to 145 |
| 4-Br | H | 6-OMe | H | O | 1 | 170 to 172 |
| 4-$CF_3$ | H | 6-OMe | $CO_2Me$ | O | 1 | 214 to 215 |
| 4-Br | H | 6-OMe | $CO_2Me$ | O | 1 | 189 to 191 |
| 4-$CF_3$ | H | 6-Cl | H | O | 1 | 184 to 186 |
| 4-Cl | H | 6-Cl | H | O | 1 | 168 to 170 |
| 4-Br | H | 6-Cl | H | O | 1 | 187 to 188 |
| 4-Cl | H | 6-Cl | $CO_2Me$ | O | 1 | 217 to 219 |
| 4-Br | H | 6-Cl | $CO_2Me$ | O | 1 | 215 to 217 |
| 4-$CF_3$ | H | 5-Me | H | O | 1 | 166 to 168 |
| 4-$CF_3$ | H | 5-Me | $CO_2Me$ | O | 1 | 199 to 201 |

| $R_1^a$ | $R_1^b$ | $R_2$ | B | x | t | |
|---|---|---|---|---|---|---|
| 4-$CF_3$ | H | 4-Me | H | O | 1 | 177 to 179 |
| 4-$CF_3$ | H | 4-Me | $CO_2Me$ | O | 1 | 199 to 201 |
| 4-$OCF_3$ | H | 4-F | H | O | 1 | 148 to 150 |
| 4-$CF_3$ | H | 5-Cl | $CO_2CH_2CO_2Me$ | O | 1 | 180 to 182 |
| 4-$CF_3$ | H | 5-Cl | $CO_2CH_2$-4-Cl-Ph | O | 1 | 182 to 184 |
| 4-$CF_3$ | H | 5-Cl | $CO_2CH_2$(penta-F-Ph) | O | 1 | 227 to 229 |
| 4-$CF_3$ | H | 5,7-Di-F | H | O | 1 | 181 to 183 |
| 4-$CF_3$ | H | 4,7-Di-F | H | O | 1 | 196 to 198 |
| 4-$CF_3$ | H | 5-Br | H | O | 1 | 203 to 205 |
| 4-$CF_3$ | H | 5-Br | $CO_2Me$ | O | 1 | 216 to 218 |
| 4-$CF_3$ | H | 5-Br | $CO_2CH_2CH=CH_2$ | O | 1 | 148 to 150 |
| 4-$CF_3$ | H | 5-Br | $CH_2OH$ | O | 1 | 177 to 179 |
| 4-$CF_3$ | H | 5-Br | $(CH_2)_3CN$ | O | 1 | 207 to 209 |
| 4-$CF_3$ | H | 4-F | $CH_2OH$ | O | 1 | 194 to 196 |
| 4-$CF_3$ | H | 4,5-Di-F | H | O | 1 | 240 to 242 |
| 4-$CF_3$ | H | 4-F,5-Cl | H | O | 1 | 211 to 213 |
| 4-Br | H | 4-F,5-Cl | H | O | 1 | 182 to 184 |
| 4-$CF_3$ | H | 4-F,5-Cl | $CO_2Me$ | O | 1 | 250 to 252 |
| 4-$CF_3$ | H | 5-Cl | $CO_2CH_2CF_3$ | O | 1 | 165 to 167 |
| 4-$CF_3$ | H | 5-Cl | $CO_2Et$ | O | 1 | 207 to 209 |
| 4-$CF_3$ | H | 5-Cl | $CO_2CH_2$-i-Pr | O | 1 | 207 to 209 |

TABLE 2

| $R_1^a$ | $R_1^b$ | $R_2$ | B | x | t | y | |
|---|---|---|---|---|---|---|---|
| 4-$CF_3$ | H | H | H | 0 | 0 | 0 | |
| 4-$CF_2Cl$ | 3-Cl | H | H | 0 | 1 | 0 | |
| 4-$CF_3$ | H | H | H | 0 | 1 | 0 | 161 to 164 |
| 4-Cl | 3-Cl | H | H | 0 | 1 | 0 | 220 to 224 |
| 4-Br | H | H | H | 0 | 1 | 0 | 215 to 220 |
| 3-$CF_3$ | H | H | H | 0 | 1 | 0 | 165 to 167 |
| 4-Cl | H | H | H | 0 | 1 | 0 | 192 to 199 |
| 3-Cl | H | H | H | 0 | 1 | 0 | 162 to 171 |
| 4-$CF_3$ | H | 5-Br | H | 0 | 1 | 0 | 192 to 195 |
| 4-Cl | H | 5-Br | H | 0 | 1 | 0 | 144 to 150 |
| 4-Br | H | 5-Br | H | 0 | 1 | 0 | 218 to 221 |
| 4-Cl | H | 7-Br | H | 0 | 1 | 0 | 180 to 184 |
| 4-$CF_3$ | H | 7-Br | H | 0 | 1 | 0 | 161 to 182 |
| 4-Br | H | 7-Br | H | 0 | 1 | 0 | 179 to 202 |
| 4-$CF_3$ | H | 5-Cl | H | 0 | 1 | 0 | 152 to 182 |
| 4-Cl | H | 5-Cl | H | 0 | 1 | 0 | 193 to 207 |
| 4-Cl | H | 5-F | H | 0 | 1 | 0 | |
| 4-$CO_2Me$ | H | 5-F | H | 0 | 2 | 0 | |
| 4-Br | H | 5-Cl | H | 0 | 1 | 0 | 228 to 232 |
| 4-$CF_3$ | H | 7-Cl | H | 0 | 1 | 0 | 178 to 202 |
| 4-Cl | H | 7-Cl | H | 0 | 1 | 0 | 179 to 193 |
| 4-Br | H | 7-Cl | H | 0 | 1 | 0 | 201 to 208 |
| 4-$CF_3$ | H | H | Me | 0 | 1 | 0 | 145 to 147 |
| 4-$CF_2Cl$ | 3-Cl | 5-Cl | Me | 0 | 2 | 0 | |
| 4-$OCF_3$ | H | 5-Cl | Me | 0 | 0 | 0 | |
| 4-$OCF_2H$ | H | 5-F | Me | 0 | 1 | 0 | |
| 4-Cl | H | 5-F | Me | 0 | 2 | 0 | |
| 4-$CO_2Me$ | H | H | Me | 0 | 0 | 0 | |
| 4-Br | H | H | Me | 0 | 0 | 0 | |
| 4-$CF_3$ | H | 5-Cl | $CO_2Me$ | 0 | 2 | 0 | |
| 4-$CF_2Cl$ | 3-Cl | 5-Cl | $CO_2Me$ | 0 | 0 | 0 | |
| 4-$OCF_3$ | H | H | CO Me | 0 | 0 | 0 | |
| 4-$OCF_2H$ | H | 5-F | $CO_2Me$ | 0 | 1 | 0 | |
| 4-Cl | H | 5-Cl | $CO_2Me$ | 0 | 2 | 0 | |
| 4-$CO_2Me$ | H | 5-Cl | $CO_2Me$ | 0 | 0 | 0 | |
| 4-Br | H | 5-F | $CO_2Me$ | 0 | 1 | 0 | |

| $R_1^a$ | $R_1^b$ | $R_2$ | B | X | t | Y | |
|---|---|---|---|---|---|---|---|
| 4-$CF_3$ | H | H | $CO_2Et$ | O | 2 | O | |
| 4-Cl | H | H | $CO_2CH_2CF_3$ | O | 1 | O | |
| 4-$CF_3$ | H | 5-Cl | $CH_2CF_3$ | O | 2 | O | |
| 4-Cl | H | 5-Cl | C(O)Me | O | 0 | O | |
| 4-$CF_3$ | H | 5-$OCF_2H$ | Me | O | 1 | O | |
| 4-$CF_3$ | H | 5-$OCF_2H$ | $CO_2Me$ | O | 2 | O | |
| 4-Cl | H | 5-F | CONHMe | O | 0 | O | |
| 4-$CF_3$ | H | 5-F | CONHPh | O | 1 | O | |
| 4-Cl | H | H | allyl | O | 2 | O | |
| 4-$CF_3$ | H | H | $CH_2Ph$ | O | 0 | O | |
| 4-Cl | H | 5-Cl | n-Pr | O | 1 | O | |
| 4-$CF_3$ | H | 5-Cl | $C(O)(CH_2)_3Cl$ | O | 2 | O | |
| 4-Cl | H | 5-$OCF_2H$ | Me | O | 0 | O | |
| 4-$CF_3$ | H | 5-$OCF_2H$ | $CO_2Me$ | O | 1 | O | |
| 4-Cl | H | 5-F | $CH_2CO_2Me$ | O | 2 | O | |
| 4-$CF_3$ | H | 5-F | $CO_2H$ | O | 0 | O | |
| 4-Cl | H | H | 4-Cl-Ph | O | 1 | O | |
| 4-$CF_3$ | H | H | 4-Cl-Ph | O | 2 | O | |
| 4-Cl | H | 5-Cl | $CO_2Me$ | S | 0 | O | |
| 4-$CF_3$ | H | 5-Cl | $CO_2Me$ | S | 1 | O | |
| 4-Cl | H | 5-$OCF_2H$ | $CO_2Me$ | S | 2 | O | |
| 4-$CF_3$ | H | H | H | O | 0 | S | |
| 4-$CF_2Cl$ | 3-Cl | H | H | O | 1 | S | |
| 4-$OCF_3$ | H | 5-Cl | H | O | 2 | S | |
| 4-$OCF_2H$ | H | 5-Cl | H | O | O | S | |
| 4-Cl | H | 5-F | H | O | 1 | S | 179 to 181 |
| 4-$CO_2Me$ | H | 5-F | H | O | 2 | S | |
| 4-Br | H | H | H | O | O | S | |
| 4-$CF_3$ | H | H | H | O | 1 | S | 170 to 171 |
| 4-Cl | 3-Cl | H | H | O | 1 | S | 235 to 239 |
| 4-Br | H | H | H | O | 1 | S | 189 to 192 |
| 3-$CF_3$ | H | H | H | O | 1 | S | 203 to 204 |
| 4-Cl | H | H | H | O | 1 | S | 178 to 180 |
| 3-$CF_3$ | H | 5-Cl | H | O | 1 | S | 197 to 200 |

| $R_1^a$ | $R_1^b$ | $R_2$ | B | x | t | y | |
|---|---|---|---|---|---|---|---|
| 4-Cl | H | 5-Cl | H | O | 1 | S | 202 to 206 |
| 3-Cl | H | 5-Cl | H | O | 1 | S | 224 to 227 |
| 4-Br | H | 5-Cl | H | O | 1 | S | 207 to 211 |
| 4-Cl | 3-Cl | 5-Cl | H | O | 1 | S | 220 to 232 |
| 4-$CF_3$ | H | 6-Cl | H | O | 1 | S | 195 to 202 |
| 3-$CF_3$ | H | 6-Cl | H | O | 1 | S | 207 to 213 |
| 4-Cl | H | 6-Cl | H | O | 1 | S | 180 to 188 |
| 3-Cl | H | 6-Cl | H | O | 1 | S | 230 to 240 |
| 4-Br | H | 6-Cl | H | O | 1 | S | 184 to 189 |
| 4-Cl | 3-Cl | 6-Cl | H | O | 1 | S | 227 to 137 |
| 4-$CF_3$ | H | 5-Br | H | O | 1 | S | 212 to 215 |
| 3-$CF_3$ | H | 5-Br | H | O | 1 | S | 187 to 200 |
| 4-Cl | H | 5-Br | H | O | 1 | S | 192 to 209 |
| 3-Cl | H | 5-Br | H | O | 1 | S | 175 to 190 |
| 4-Br | H | 5-Br | H | O | 1 | S | 188 to 215 |
| 4-Cl | 3-Cl | 5-Br | H | O | 1 | S | 187 to 200 |
| 4-$CF_3$ | H | 4-Cl | H | O | 1 | S | 207 to 210 |
| 4-Cl | H | 4-Cl | H | O | 1 | S | 191 to 193 |
| 4-Br | H | 4-Cl | H | O | 1 | S | 188 to 190 |
| 4-$CF_3$ | H | H | Me | O | 1 | S | |
| 4-$CF_2Cl$ | 3-Cl | 5-Cl | Me | O | 2 | S | |
| 4-$OCF_3$ | H | 5-Cl | Me | O | 0 | S | |
| 4-$OCF_2H$ | H | 5-F | Me | O | 1 | S | |
| 4-Cl | H | 5-F | Me | O | 2 | S | |
| 4-$CO_2Me$ | H | H | Me | O | 0 | S | |
| 4-Br | H | H | Me | O | 1 | S | |
| 4-$CF_3$ | H | 5-Cl | $CO_2Me$ | O | 2 | S | 167 to 170 |
| 4-$CF_2Cl$ | 3-Cl | 5-Cl | $CO_2Me$ | O | 0 | S | |
| 4-$OCF_3$ | H | H | CO Me | O | 1 | S | |
| 4-$OCF_2H$ | H | H | $CO_2Me$ | O | 2 | S | |
| 4-Cl | H | 5-Cl | $CO_2Me$ | O | 0 | S | |
| 4-$CO_2Me$ | H | 5-Cl | $CO_2Me$ | O | 1 | S | |
| 4-Br | H | 5-F | $CO_2Me$ | O | 2 | S | |
| 4-$CF_3$ | H | H | $CO_2Et$ | O | 0 | S | |
| 4-Cl | H | H | $CO_2CH_2CF_3$ | O | 1 | S | |
| 4-$CF_3$ | H | 5-Cl | $CH_2CF_3$ | O | 2 | S | |

| $R_1^a$ | $R_1^b$ | $R_2$ | B | x | t | y |
|---|---|---|---|---|---|---|
| 4-Cl | H | 5-Cl | C(O)Me | O | 0 | S |
| $4-CF_3$ | H | $5-OCF_2H$ | Me | O | 1 | S |
| $4-CF_3$ | H | $5-OCF_2H$ | $CO_2Me$ | O | 2 | S |
| 4-Cl | H | 5-F | CONHMe | O | 0 | S |
| $4-CF_3$ | H | 5-F | CONHPh | O | 1 | S |
| 4-Cl | H | H | allyl | O | 2 | S |
| $4-CF_3$ | H | H | $CH_2Ph$ | O | 0 | S |
| 4-Cl | H | 5-Cl | n-Pr | O | 1 | S |
| $4-CF_3$ | H | 5-Cl | $C(O)(CH_2)_3Cl$ | O | 2 | S |
| 4-Cl | H | $5-OCF_2H$ | Me | O | 0 | S |
| $4-CF_3$ | H | $5-OCF_2H$ | $CO_2Me$ | O | 1 | S |
| 4-Cl | H | 5-F | $CH_2CO_2Me$ | O | 2 | S |
| $4-CF_3$ | H | 5-F | $CO_2H$ | O | 0 | S |
| 4-Cl | H | H | 4-Cl-Ph | O | 1 | S |
| $4-CF_3$ | H | H | 4-Cl-Ph | O | 2 | S |
| 4-Cl | H | 5-Cl | $CO_2Me$ | S | 0 | S |
| $4-CF_3$ | H | 5-Cl | $CO_2Me$ | S | 1 | S |
| 4-Cl | H | $5-OCF_2H$ | $CO_2Me$ | S | 2 | S |
| $4-CF_3$ | H | H | H | O | 0 | NMe |
| $4-CF_2Cl$ | 3-Cl | H | H | O | 1 | NMe |
| $4-OCF_3$ | H | 5-Cl | H | O | 2 | NMe |
| $4-OCF_2H$ | H | 5-Cl | H | O | 0 | NMe |
| 4-Cl | H | 5-F | H | O | 1 | NMe |
| $4-CO_2Me$ | H | 5-F | H | O | 2 | NMe |
| 4-Br | H | H | H | O | 0 | NMe |
| $4-CF_3$ | H | H | Me | O | 1 | NMe |
| $4-CF_2Cl$ | 3-Cl | 5-Cl | Me | O | 2 | NMe |
| $4-OCF_3$ | H | 5-Cl | Me | O | 0 | NMe |
| $4-OCF_2H$ | H | 5-F | Me | O | 1 | NMe |
| 4-Cl | H | 5-F | Me | O | 2 | NMe |
| $4-CO_2Me$ | H | H | Me | O | 0 | NMe |
| 4-Br | H | H | Me | O | 1 | NMe |
| $4-CF_3$ | H | 5-Cl | $CO_2Me$ | O | 2 | NMe |

| $R_1^a$ | $R_1^b$ | $R_2$ | B | x | t | y | |
|---|---|---|---|---|---|---|---|
| 4-$CF_2$Cl | 3-Cl | 5-Cl | $CO_2$Me | O | 0 | NMe | |
| 4-$OCF_3$ | H | H | $CO_2$Me | O | 1 | NMe | |
| 4-$OCF_2$H | H | H | $CO_2$Me | O | 2 | NMe | |
| 4-Cl | H | 5-Cl | $CO_2$Me | O | 0 | NMe | |
| 4-$CO_2$Me | H | 5-Cl | $CO_2$Me | O | 1 | NMe | |
| 4-Br | H | 5-F | $CO_2$Me | O | 2 | NMe | |
| 4-$CF_3$ | H | H | $CO_2$Et | O | 0 | NMe | |
| 4-Cl | H | H | $CO_2CH_2CF_3$ | O | 1 | NMe | |
| 4-$CF_3$ | H | 5-Cl | $CH_2CF_3$ | O | 2 | NMe | |
| 4-Cl | H | 5-Cl | C(O)Me | O | 0 | NMe | |
| 4-$CF_3$ | H | 5-$OCF_2$H | Me | O | 1 | NMe | |
| 4-$CF_3$ | H | 5-$OCF_2$H | $CO_2$Me | O | 2 | NMe | |
| 4-Cl | H | 5-F | CONHMe | O | 0 | NMe | |
| 4-$CF_3$ | H | 5-F | CONHPh | O | 1 | NMe | |
| 4-Cl | H | H | allyl | O | 2 | NMe | |
| 4-$CF_3$ | H | H | $CH_2$Ph | O | 0 | NMe | |
| 4-Cl | H | 5-Cl | n-Pr | O | 1 | NMe | |
| 4-$CF_3$ | H | 5-Cl | C(O)$(CH_2)_3$Cl | O | 2 | NMe | |
| 4-Cl | H | 5-$OCF_2$H | Me | O | 0 | NMe | |
| 4-$CF_3$ | H | 5-$OCF_2$H | $CO_2$Me | O | 1 | NMe | |
| 4-Cl | H | 5-F | $CH_2CO_2$Me | O | 2 | NMe | |
| 4-$CF_3$ | H | 5-F | $CO_2$H | O | 0 | NMe | |
| 4-Cl | H | H | 4-Cl-Ph | O | 1 | NMe | |
| 4-$CF_3$ | H | H | 4-Cl-Ph | O | 2 | NMe | |
| 4-Cl | H | 5-Cl | $CO_2$Me | S | 0 | NMe | |
| 4-$CF_3$ | H | 5-Cl | $CO_2$Me | S | 1 | NMe | |
| 4-Cl | H | 5-$OCF_2$H | $CO_2$Me | S | 2 | NMe | |
| 4-$CF_3$ | H | H | H | O | 1 | $SO_2$ | 255 to 260 |
| 4-Cl | 3-Cl | H | H | O | 1 | $SO_2$ | 250 to 255 |
| 4-Br | H | H | H | O | 1 | $SO_2$ | 272 to 274 |
| 3-$CF_3$ | H | H | H | O | 1 | $SO_2$ | 261 to 263 |

| $R_1^a$ | $R_1^b$ | $R_2$ | B | x | t | y | |
|---|---|---|---|---|---|---|---|
| 4-Cl | H | H | H | O | 1 | $SO_2$ | 247 to 250 |
| 3-Cl | H | H | H | O | 1 | $SO_2$ | 245 to 249 |
| 4-$CF_3$ | H | 5-Cl | H | O | 1 | $SO_2$ | 293 to 297 |
| 3-$CF_3$ | H | 5-Cl | H | O | 1 | $SO_2$ | 217 to 234 |
| 4-Cl | H | 5-Cl | H | O | 1 | $SO_2$ | 318 to 321 |
| 3-Cl | H | 5-Cl | H | O | 1 | $SO_2$ | 241 to 270 |
| 4-Br | H | 5-Cl | H | O | 1 | $SO_2$ | 290 to 292 |
| 4-Cl | 3-Cl | 5-Cl | H | O | 1 | $SO_2$ | 280 to 285 |
| 4-$CF_3$ | H | 6-Cl | H | O | 1 | $SO_2$ | 290 to 292 |
| 3-$CF_3$ | H | 6-Cl | H | O | 1 | $SO_2$ | 175 to 182 |
| 4-Cl | H | 6-Cl | H | O | 1 | $SO_2$ | 230 to 280 |
| 3-Cl | H | 6-Cl | H | O | 1 | $SO_2$ | 245 to 269 |
| 4-Br | H | 6-Cl | H | O | 1 | $SO_2$ | 307 to 310 |
| 4-Cl | 3-Cl | 6-Cl | H | O | 1 | $SO_2$ | 275 to 278 |
| 4-$CF_3$ | H | H | Me | O | 0 | $SO_2$ | |
| 4-Cl | H | 5-Cl | Me | O | 1 | $SO_2$ | |
| 4-$OCF_3$ | H | H | Me | O | 2 | $SO_2$ | |
| 4-$CF_3$ | H | 5-Cl | $CO_2Me$ | O | 0 | $SO_2$ | |
| 4-Cl | H | H | $CO_2Me$ | O | 1 | $SO_2$ | |
| 4-$OCF_3$ | H | 5-Cl | $CO_2Me$ | O | 2 | $SO_2$ | |
| 3-$CF_3$ | H | H | H | O | 1 | SO(α) | 225 to 260 |
| 4-$CF_3$ | H | H | H | O | 1 | SO(α) | 250 to 255 |
| 4-Br | H | H | H | O | 1 | SO(α) | 278 to 282 |
| 4-Br | H | H | H | O | 1 | SO(α) | 258 to 260 |
| 4-Cl | 3-Cl | H | H | O | 1 | SO(α) | 275 to 280 |
| 4-Cl | 3-Cl | H | H | O | 1 | SO(ß) | 265 to 269 |
| 4-Cl | H | H | H | O | 1 | SO(α) | 262 to 264 |
| 4-Cl | H | H | H | O | 1 | SO(ß) | 247 to 250 |
| 3-Cl | H | H | H | O | 1 | SO(α) | 231 to 232 |
| 3-Cl | H | H | H | O | 1 | SO(ß) | 255 to 258 |
| 4-$CF_3$ | H | 5-Cl | H | O | 1 | SO(α) | 271 to 275 |
| 4-$CF_3$ | H | 5-Cl | H | O | 1 | SO(ß) | 256 to 277 |
| 3-$CF_3$ | H | 5-Cl | H | O | 1 | SO(α) | 210 to 213 |

| $R_1^a$ | $R_1^b$ | $R_2$ | B | x | t | Y | |
|---|---|---|---|---|---|---|---|
| $3-CF_3$ | H | 5-Cl | H | O | 1 | SO(ß) | 250 to 252 |
| 4-Cl | H | 5-Cl | H | O | 1 | SO(α) | 284 to 286 |
| 4-Cl | H | 5-Cl | H | O | 1 | SO(ß) | 278 to 283 |
| 3-Cl | H | 5-Cl | H | O | 1 | SO(α) | 240 to 241 |
| 3-Cl | H | 5-Cl | H | O | 1 | SO(ß) | 230 to 246 |
| 4-Br | H | 5-Cl | H | O | 1 | SO(α) | 285 to 287 |
| 4-Br | H | 5-Cl | H | O | 1 | SO(ß) | 267 to 272 |
| 4-Cl | 3-Cl | 5-Cl | H | O | 1 | SO(α) | 276 to 280 |
| 4-Cl | 3-Cl | 5-Cl | H | O | 1 | SO(ß) | 254 to 268 |
| $4-CF_3$ | H | 6-Cl | H | O | 1 | SO(α) | 252 to 256 |
| $4-CF_3$ | H | 6-Cl | H | O | 1 | SO(ß) | 218 to 232 |
| $3-CF_3$ | H | 6-Cl | H | O | 1 | SO(α) | 263 to 265 |
| $3-CF_3$ | H | 6-Cl | H | O | 1 | SO(ß) | |
| 4-Cl | H | 6-Cl | H | O | 1 | SO(α) | 243 to 245 |
| 4-Cl | H | 6-Cl | H | O | 1 | SO(ß) | 269 to 272 |
| 3-Cl | H | 6-Cl | H | O | 1 | SO(α) | 154 to 160 |
| 3-Cl | H | 6-Cl | H | O | 1 | SO(ß) | |
| 4-Br | H | 6-Cl | H | O | 1 | SO(α) | 248 to 250 |
| 4-Br | H | 6-Cl | H | O | 1 | SO(ß) | 283 to 285 |
| 4-Cl | 3-Cl | 6-Cl | H | O | 1 | SO(α ) | 272 to 275 |
| 4-Cl | 3-Cl | 6-Cl | H | O | 1 | SO(ß) | 289 to 295 |
| $3-CF_3$ | H | H | H | O | 1 | SO(ß) | 229 to 236 |
| $4-CF_3$ | H | H | H | O | 1 | SO(ß) | 235 to 258 |
| $4-OCF_3$ | H | H | Me | O | O | N-n-Bu | |
| $4-CF_3$ | H | 5-Cl | $CO_2Me$ | O | 1 | N-allyl | |
| 4-Cl | H | H | $CO_2Me$ | O | O | N-Ph | |
| $4-OCF_3$ | H | 5-Cl | $CO_2Me$ | O | 1 | $N-CH_2Ph$ | |
| $4-CF_3$ | H | H | $CO_2Me$ | O | O | N-4-F-Ph | |
| $4-CF_3$ | H | H | H | O | 1 | NH | |
| $4-CF_3$ | H | H | CO Me | O | 1 | NH | |
| $4-CF_3$ | H | 5-Cl | H | O | 1 | NMe | |
| $4-CF_3$ | H | 5-Cl | $CO_2Me$ | O | 1 | NMe | |
| $4-CF_3$ | H | H | OH | O | 1 | $CH_2$ | 173 to 180 |
| $4-CF_3$ | H | 5-F | H | O | 1 | S | 180 to 181 |
| 4-Br | H | 5-F | H | O | 1 | S | 188 to 192 |
| $4-CF_3$ | H | 4-F | H | O | 1 | S | 198 to 203 |

| $R_1^a$ | $R_1^b$ | $R_2$ | B | x | t | y | |
|---|---|---|---|---|---|---|---|
| 4-Cl | H | 4-F | H | O | 1 | S | 191 to 194 |
| 4-Br | H | 4-F | H | O | 1 | S | 208 to 211 |
| $4-CF_3$ | H | 5-Cl | $CO_2Me$ | O | 1 | S | 195 to 207 |
| $4-CF_3$ | H | 5-Cl | Me | O | 2 | S | 175 to 178 |
| 4-Cl | H | 5-Cl | $CO_2Me$ | O | 2 | S | 163 to 165 |
| 4-Cl | H | 5-Cl | Me | O | 2 | S | 58 to 62 |
| 4-Br | H | 5-Cl | $CO_2Me$ | O | 2 | S | 150 to 152 |
| 4-Br | H | 5-Cl | Me | O | 2 | S | 125 to 127 |
| $4-CF_3$ | H | 4-F | H | O | 1 | O | 190 to 192 |
| $4-CF_3$ | H | 4-F | H | O | 1 | O | 215 to 220 |
| 4-Br | H | 4-F | H | O | 1 | O | 235 to 240 |
| $4-CF_3$ | H | $5-CF_3$ | H | O | 1 | S | 188 to 200 |
| 4-Cl | H | $5-CF_3$ | H | O | 1 | S | 208 to 210 |
| 4-Br | H | $5-CF_3$ | H | O | 1 | S | 212 to 218 |
| $4-CF_3$ | H | 4-Cl | H | O | 1 | O | 186 to 208 |
| 4-Cl | H | 4-Cl | H | O | 1 | O | 190 to 191 |
| 4-Br | H | 4-Cl | H | O | 1 | O | 205 to 211 |
| $4-CF_3$ | H | $5-CF_3$ | H | O | 1 | O | 160 to 165 |
| 4-Cl | H | $5-CF_3$ | H | O | 1 | O | 180 to 190 |
| 4-Br | H | $5-CF_3$ | H | O | 1 | O | 175 to 180 |
| $4-CF_3$ | H | 4,5-Di-Cl | H | O | 1 | O | 235 to 237 |
| $4-CF_3$ | H | H | $CO_2Me$ | O | 1 | O | 158 to 160 |
| $4-CF_3$ | H | H | Ph | O | 1 | O | 218 to 221 |

TABLE 3

| $R_1^a$ | $R_1^b$ | $R_2$ | B | x | t | v |
|---|---|---|---|---|---|---|
| 4-$CF_3$ | H | H | H | O | 0 | O |
| 4-$CF_2Cl$ | 3-Cl | H | H | O | 1 | O |
| 4-$OCF_3$ | H | 5-Cl | H | O | 2 | O |
| 4-$OCF_2H$ | H | 5-Cl | H | O | 2 | O |
| 4-Cl | H | 5-F | H | O | 1 | O |
| 4-$CO_2Me$ | H | 5-F | H | O | 2 | O |
| 4-Br | H | H | H | O | 0 | O |
| 4-$CF_3$ | H | H | Me | O | 1 | O |
| 4-$CF_2Cl$ | 3-Cl | 5-Cl | Me | O | 2 | O |
| 4-$OCF_3$ | H | 5-Cl | Me | O | 0 | O |
| 4-$OCF_2H$ | H | 5-F | Me | O | 1 | O |
| 4-Cl | H | 5-F | Me | O | 2 | O |
| 4-$CO_2Me$ | H | H | Me | O | 0 | O |
| 4-Br | H | H | Me | O | 1 | O |
| 4-$CF_3$ | H | 5-Cl | $CO_2Me$ | O | 2 | O |
| 4-$CF_2Cl$ | 3-Cl | 5-Cl | $CO_2Me$ | O | 0 | O |
| 4-$OCF_3$ | H | H | $CO_2Me$ | O | 1 | O |
| 4-$OCF_2H$ | H | H | $CO_2Me$ | O | 2 | O |
| 4-Cl | H | 5-Cl | $CO_2Me$ | O | 0 | O |
| 4-$CO_2Me$ | H | 5-CL | $CO_2Me$ | O | 1 | O |
| 4-Br | H | 5-F | $CO_2Me$ | O | 2 | O |
| 4-$CF_3$ | H | H | $CO_2Et$ | O | 0 | O |
| 4-Cl | H | H | $CO_2CH_2CF_3$ | O | 1 | O |
| 4-$CF_3$ | H | 5-Cl | $CH_2CF_3$ | O | 2 | O |
| 4-Cl | H | 5-Cl | C(O)Me | O | 0 | O |
| 4-$CF_3$ | H | 5-$OCF_2H$ | Me | O | 1 | O |
| 4-$CF_3$ | H | 5-$OCF_2H$ | $CO_2Me$ | O | 2 | O |
| 4-Cl | H | 5-F | CONHMe | O | 0 | O |
| 4-$CF_3$ | H | 5-F | CONHPh | O | 1 | O |
| 4-Cl | H | H | allyl | O | 2 | O |
| 4-$CF_3$ | H | H | $CH_2Ph$ | O | 0 | O |
| 4-Cl | H | 5-Cl | n-Pr | O | 1 | O |

| $R_1^a$ | $R_1^b$ | $R_2$ | B | x | t | y |
|---|---|---|---|---|---|---|
| 4-$CF_3$ | H | 5-Cl | $C(O)(CH_2)_3Cl$ | O | 2 | O |
| 4-Cl | H | 5-$OCF_2H$ | $CO_2Me$ | O | 1 | O |
| 4-Cl | H | 5-F | $CH_2CO_2Me$ | O | 2 | O |
| 4-$CF_3$ | H | 5-F | $CO_2H$ | O | 0 | O |
| 4-Cl | H | H | 4-Cl-Ph | O | 1 | O |
| 4-$CF_3$ | H | H | 4-Cl-Ph | O | 2 | O |
| 4-Cl | H | 5-Cl | $CO_2Me$ | S | 0 | O |
| 4-$CF_3$ | H | 5-Cl | $CO_2Me$ | S | 1 | O |
| 4-Cl | H | 5-$OCF_2H$ | $CO_2Me$ | S | 2 | O |
| 4-$CF_3$ | H | H | H | O | 0 | S |
| 4-$CF_2Cl$ | 3-Cl | H | H | O | 1 | S |
| 4-$OCF_3$ | H | 5-Cl | H | O | 2 | S |
| 4-$OCF_2H$ | H | 5-Cl | H | O | 2 | S |
| 4-Cl | H | 5-F | H | O | 1 | S |
| 4-$CO_2Me$ | H | 5-F | H | O | 2 | S |
| 4-Br | H | H | H | O | 0 | S |
| 4-$CF_3$ | H | H | Me | O | 1 | S |
| 4-$CF_2Cl$ | 3-Cl | 5-Cl | Me | O | 2 | S |
| 4-$OCF_3$ | H | 5-Cl | Me | O | 0 | S |
| 4-$OCF_2H$ | H | 5-F | Me | O | 1 | S |
| 4-Cl | H | 5-F | Me | O | 2 | S |
| 4-$CO_2Me$ | H | H | Me | O | 0 | S |
| 4-Br | H | H | Me | O | 1 | S |
| 4-$CF_3$ | H | 5-Cl | $CO_2Me$ | O | 2 | S |
| 4-$CF_2Cl$ | 3-Cl | 5-Cl | $CO_2Me$ | O | 0 | S |
| 4-$OCF_3$ | H | H | $CO_2Me$ | O | 1 | S |
| 4-$OCF_2H$ | H | H | $CO_2Me$ | O | 2 | S |
| 4-Cl | H | 5-Cl | $CO_2Me$ | O | 0 | S |
| 4-$CO_2Me$ | H | 5-Cl | $CO_2Me$ | O | 1 | S |
| 4-Br | H | 5-F | $CO_2Me$ | O | 2 | S |
| 4-$CF_3$ | H | H | $CO_2Et$ | O | 0 | S |
| 4-Cl | H | H | $CO_2CH_2CF_3$ | O | 1 | S |
| 4-$CF_3$ | H | 5-Cl | $CH_2CF_3$ | O | 2 | S |

| $R_1^a$ | $R_1^b$ | $R_2$ | B | X | t | Y |
|---|---|---|---|---|---|---|
| 4-Cl | H | 5-Cl | C(O)Me | O | 0 | S |
| 4-$CF_3$ | H | 5-$OCF_2H$ | Me | O | 1 | S |
| 4-$CF_3$ | H | 5-$OCF_2H$ | $CO_2Me$ | O | 2 | S |
| 4-Cl | H | 5-F | CONHMe | O | 0 | S |
| 4-$CF_3$ | H | 5-F | CONHPh | O | 1 | S |
| 4-Cl | H | H | allyl | O | 2 | S |
| 4-$CF_3$ | H | H | $CH_2Ph$ | O | 0 | S |
| 4-Cl | H | 5-Cl | n-Pr | O | 1 | S |
| 4-$CF_3$ | H | 5-Cl | $C(O)(CH_2)_3Cl$ | O | 2 | S |
| 4-Cl | H | 5-$OCF_2H$ | $CO_2Me$ | O | 1 | S |
| 4-Cl | H | 5-F | $CH_2CO_2Me$ | O | 2 | S |
| 4-$CF_3$ | H | 5-F | $CO_2H$ | O | 0 | S |
| 4-Cl | H | H | 4-Cl-Ph | O | 1 | S |
| 4-$CF_3$ | H | H | 4-Cl-Ph | O | 2 | S |
| 4-Cl | H | 5-Cl | $CO_2Me$ | S | 0 | S |
| 4-$CF_3$ | H | 5-Cl | $CO_2Me$ | S | 1 | S |
| 4-Cl | H | 5-$OCF_2H$ | $CO_2Me$ | S | 2 | S |
| 4-$CF_3$ | H | H | H | O | 0 | NMe |
| 4-$CF_2Cl$ | 3-Cl | H | H | O | 1 | NMe |
| 4-$OCF_3$ | H | 5-Cl | H | O | 2 | NMe |
| 4-$OCF_2H$ | H | 5-Cl | H | O | 2 | NMe |
| 4-Cl | H | 5-F | H | O | 1 | NMe |
| 4-$CO_2Me$ | H | 5-F | H | O | 2 | NMe |
| 4-Br | H | H | H | O | 0 | NMe |
| 4-$CF_3$ | H | H | Me | O | 1 | NMe |
| 4-$CF_2Cl$ | 3-Cl | 5-Cl | Me | O | 2 | NMe |
| 4-$OCF_3$ | H | 5-Cl | Me | O | 0 | NMe |
| 4-$OCF_2H$ | H | 5-F | Me | O | 1 | NMe |
| 4-Cl | H | 5-F | Me | O | 2 | NMe |
| 4-$CO_2Me$ | H | H | Me | O | 0 | NMe |
| 4-Br | H | H | Me | O | 1 | NMe |
| 4-$CF_3$ | H | 5-Cl | $CO_2Me$ | O | 2 | NMe |
| 4-$CF_2Cl$ | 3-Cl | 5-Cl | $CO_2Me$ | O | 0 | NMe |

| $R_1^a$ | $R_1^b$ | $R_2$ | B | X | t | Y |
|---|---|---|---|---|---|---|
| 4-$OCF_3$ | H | H | $CO_2Me$ | O | 1 | NMe |
| 4-$OCF_2H$ | H | H | $CO_2Me$ | O | 2 | NMe |
| 4-Cl | H | 5-Cl | $CO_2Me$ | O | 0 | NMe |
| 4-$CO_2Me$ | H | 5-Cl | $CO_2Me$ | O | 1 | NMe |
| 4-Br | H | 5-F | $CO_2Me$ | O | 2 | NMe |
| 4-$CF_3$ | H | H | $CO_2Et$ | O | 0 | NMe |
| 4-Cl | H | H | $CO_2CH_2CF_3$ | O | 1 | NMe |
| 4-$CF_3$ | H | 5-Cl | $CH_2CF_3$ | O | 2 | NMe |
| 4-Cl | H | 5-Cl | C(O)Me | O | 0 | NMe |
| 4-$CF_3$ | H | 5-$OCF_2H$ | Me | O | 1 | NMe |
| 4-$CF_3$ | H | 5-$OCF_2H$ | $CO_2Me$ | O | 2 | NMe |
| 4-Cl | H | 5-F | CONHMe | O | 0 | NMe |
| 4-$CF_3$ | H | 5-F | CONHPh | O | 1 | NMe |
| 4-Cl | H | H | allyl | O | 2 | NMe |
| 4-$CF_3$ | H | H | $CH_2Ph$ | O | 0 | NMe |
| 4-Cl | H | 5-Cl | N-Pr | O | 1 | NMe |
| 4-$CF_3$ | H | 5-Cl | $C(O)(CH_2)_3Cl$ | O | 2 | NMe |
| 4-Cl | H | 5-$OCF_2H$ | $CO_2Me$ | O | 1 | NMe |
| 4-Cl | H | 5-F | $CH_2CO_2Me$ | O | 2 | NMe |
| 4-$CF_3$ | H | 5-F | $CO_2H$ | O | 0 | NMe |
| 4-Cl | H | H | 4-Cl-Ph | O | 1 | NMe |
| 4-$CF_3$ | H | H | 4-Cl-Ph | O | 2 | NMe |
| 4-Cl | H | 5-Cl | $CO_2Me$ | S | 0 | NMe |
| 4-$CF_3$ | H | 5-Cl | $CO_2Me$ | S | 1 | NMe |
| 4-Cl | H | 5-$OCF_2H$ | $CO_2Me$ | S | 2 | NMe |
| 4-$CF_3$ | H | H | Me | O | 0 | $SO_2$ |
| 4-Cl | H | 5-Cl | Me | O | 1 | $SO_2$ |
| 4-$OCF_3$ | H | H | Me | O | 2 | $SO_2$ |
| 4-$CF_3$ | H | 5-Cl | $CO_2Me$ | O | 0 | $SO_2$ |
| 4-Cl | H | H | $CO_2Me$ | O | 1 | $SO_2$ |

| $R_1^a$ | $R_1^b$ | $R_2$ | B | x | t | y |
|---|---|---|---|---|---|---|
| 4-$OCF_3$ | H | 5-Cl | $CO_2Me$ | O | 2 | $SO_2$ |
| 4-$CF_3$ | H | H | Me | O | 0 | SO |
| 4-Cl | H | 5-Cl | Me | O | 1 | SO |
| 4-$OCF_3$ | H | H | Me | O | 2 | SO |
| 4-$CF_3$ | H | 5-Cl | $CO_2Me$ | O | 0 | SO |
| 4-Cl | H | H | $CO_2Me$ | O | 1 | SO |
| 4-$OCF_3$ | H | 5-Cl | $CO_2Me$ | O | 2 | SO |
| 4-$CF_3$ | H | H | Me | O | 0 | SO |
| 4-Cl | H | 5-Cl | Me | O | 1 | NH |
| 4-$OCF_3$ | H | H | Me | O | 0 | N-n-Bu |
| 4-$CF_3$ | H | 5-Cl | $CO_2Me$ | O | 1 | N-allyl |
| 4-Cl | H | H | $CO_2Me$ | O | 0 | N-Ph |
| 4-$OCF_3$ | H | 5-Cl | $CO_2Me$ | O | 1 | N-$CH_2$Ph |
| 4-$CF_3$ | H | H | $CO_2Me$ | O | 0 | N-4-F-Ph |

TABLE 4

| $R_1^a$ | $R_1^b$ | $R_2$ | B | X | Z |
|---|---|---|---|---|---|
| 4-$CF_3$ | H | H | H | O | O |
| 4-Cl | H | H | H | O | O |
| 4-F | H | 5-Cl | H | O | O |
| 4-$OCF_2H$ | H | 5-Cl | Me | O | O |
| 4-SMe | H | H | Me | O | O |
| 4-Cl | 3-Cl | H | Me | O | O |
| 4-$CF_3$ | H | 5-Cl | Et | O | O |
| 4-Cl | H | 5-Cl | Et | O | O |
| 4-F | H | H | Et | O | O |
| 4-$OCF_2H$ | H | H | allyl | O | O |
| 4-SMe | H | 5-Cl | allyl | O | O |
| 4-Cl | 3-Cl | 5-Cl | allyl | O | O |
| 4-$CF_3$ | H | 5-F | $CO_2Me$ | O | O |
| 4-Cl | H | 5-F | C(O)Me | O | O |
| 4-F | H | 5-$OCF_2H$ | $CH_2Ph$ | O | O |
| 4-$OCF_2H$ | H | 5-$OCF_2H$ | $CH_2Ph$ | O | O |
| 4-SMe | H | 5-$CF_3$ | Ph | O | O |
| 4-Cl | 3-Cl | 5-$CF_3$ | Ph | O | O |
| 4-$CF_3$ | H | H | Me | S | O |
| 4-Cl | H | H | Me | S | O |
| 4-F | H | H | Me | S | O |
| 4-$CF_3$ | H | H | H | O | NMe |
| 4-Cl | H | H | H | O | NMe |
| 4-F | H | 5-Cl | H | O | NMe |
| 4-$OCF_2H$ | H | 5-Cl | Me | O | NMe |
| 4-SMe | H | H | Me | O | NMe |
| 4-Cl | 3-Cl | H | Me | O | NMe |
| 4-$CF_3$ | H | 5-Cl | Et | O | NMe |
| 4-Cl | H | 5-Cl | Et | O | NMe |
| 4-F | H | H | Et | O | NMe |
| 4-$OCF_2H$ | H | H | allyl | O | NMe |
| 4-SMe | H | 5-Cl | allyl | O | NMe |
| 4-Cl | 3-Cl | 5-Cl | allyl | O | NMe |
| 4-$CF_3$ | H | 5-F | $CO_2Me$ | O | NMe |

| $R_1^a$ | $R_1^b$ | $R_2$ | B | X | Z |
|---|---|---|---|---|---|
| 4-Cl | H | 5-F | C(O)Me | O | NMe |
| 4-F | H | 5-$OCF_2H$ | $CH_2Ph$ | O | NMe |
| 4-$OCF_2H$ | H | 5-$OCF_2H$ | $CH_2Ph$ | O | NMe |
| 4-SMe | H | 5-$CF_3$ | Ph | O | NMe |
| 4-Cl | 3-Cl | 5-$CF_3$ | Ph | O | NMe |
| 4-$CF_3$ | H | H | Me | S | NMe |
| 4-Cl | H | H | Me | S | NMe |
| 4-F | H | H | Me | S | NMe |
| 4-$CF_3$ | H | H | Me | O | NH |
| 4-Cl | H | 5-Cl | Me | O | NH |
| 4-Br | H | 5-F | Me | O | NH |
| 4-$OCF_3$ | H | H | Me | O | NH |
| 4-$CF_3$ | H | 5-Cl | Me | O | NEt |
| 4-Cl | H | 5-F | Me | O | NEt |
| 4-Br | H | H | Me | O | NEt |
| 4-$OCF_3$ | H | 5-Cl | Me | O | N-n-$C_4H_9$ |
| 4-$CF_3$ | H | 5-F | Me | O | N-n-$C_4H_9$ |
| 4-Cl | H | H | Me | O | N-allyl |
| 4-Br | H | 5-Cl | Me | O | N-allyl |
| 4-$OCF_3$ | H | 5-F | Me | O | N-4-Cl-Ph |
| 4-$CF_3$ | H | H | Me | O | N-4-F-Ph |
| 4-Cl | H | 5-Cl | Me | O | NPh |
| 4-Br | H | 5-F | Me | O | $NCH_2Ph$ |
| 4-$OCF_3$ | H | H | Me | O | $NCH_2Ph$ |
| 4-$CF_3$ | H | 5-Cl | Me | O | N-$CH_2C(Cl)=CH_2$ |

TABLE 5

| $R_1^a$ | $R_1^b$ | $R_2$ | B | X | Z |
|---|---|---|---|---|---|
| 4-$CF_3$ | H | H | H | O | O |
| 4-$OCF_3$ | H | 5-Cl | H | O | O |
| 4-$OCF_2H$ | H | H | H | O | O |
| 4-Cl | H | 5-Cl | $CO_2Me$ | O | O |
| 4-F | H | H | $CO_2Me$ | O | O |
| 4-Br | H | 5-Cl | $CO_2Me$ | O | O |
| 4-$CF_3$ | H | H | $CO_2Me$ | O | O |
| 4-$OCF_3$ | H | 5-Cl | $CO_2Me$ | O | O |
| 4-$OCF_2H$ | H | H | $CO_2Me$ | O | O |
| 4-Cl | H | 5-Cl | Me | O | O |
| 4-F | H | H | Me | O | O |
| 4-Br | H | 5-Cl | Me | O | O |
| 4-$CF_3$ | H | H | Et | O | O |
| 4-$OCF_3$ | H | 5-Cl | n-Pr | O | O |
| 4-$OCF_2H$ | H | H | allyl | O | O |
| 4-Cl | H | 5-Cl | allyl | O | O |
| 4-F | H | H | $CH_2$-4-F-Ph | O | O |
| 4-Br | H | 5-Cl | $CH_2$-4-Cl-Ph | O | O |
| 4-$CF_3$ | H | H | $CH_2CF_3$ | O | O |
| 4-$OCF_3$ | H | 5-Cl | $(CH_2)_4Cl$ | O | O |
| 4-$OCF_2H$ | H | H | i-Pr | O | O |
| 4-$CF_3$ | H | 5-Cl | OH | O | O |
| 4-$OCF_3$ | H | 5-Cl | OAc | O | O |
| 4-$OCF_2H$ | 3-Cl | 5-Cl | OMe | O | O |
| 4-Cl | H | 5-Cl | $C(O)CH_3$ | O | O |
| 4-F | H | 5-Cl | C(O)NHMe | O | O |
| 4-Br | H | 5-Cl | $C(S)NMe_2$ | O | O |
| 4-$CF_3$ | H | 5-F | Me | O | O |
| 4-$OCF_3$ | H | 5-F | n-Bu | O | O |
| 4-$OCF_2H$ | 3-Cl | 5-F | alkyl | O | O |
| 4-Cl | H | 5-F | i-Pr | O | O |

| $R_1^a$ | $R_1^b$ | $R_2$ | B | X | Z |
|---|---|---|---|---|---|
| 4-F | H | 5-F | $CH_2Ph$ | O | O |
| 4-Br | H | 5-F | Ph | O | O |
| $4-CF_3$ | H | $5-CF_3$ | $CH_2CH_2F$ | O | O |
| $4-OCF_3$ | H | $5-CF_3$ | $CH_2SCH_3$ | O | O |
| $4-OCF_2H$ | 3-Cl | $5-CF_3$ | $CH_2OCH_3$ | O | O |
| 4-Cl | H | $5-CF_3$ | $CH_2CN$ | O | O |
| 4-F | H | $5-CF_3$ | $CH_2CO_2Me$ | O | O |
| 4-Br | H | $5-CF_3$ | $CS_2Me$ | O | O |
| $4-CF_3$ | H | H | Me | S | O |
| $4-OCF_3$ | H | H | Me | S | O |
| $4-OCF_2H$ | 3-Cl | H | Me | S | O |
| $4-CF_3$ | H | H | H | O | NMe |
| $4-OCF_3$ | H | 5-Cl | H | O | NMe |
| $4-OCF_2H$ | H | H | H | O | NMe |
| 4-Cl | H | 5-Cl | $CO_2Me$ | O | NMe |
| 4-F | H | H | $CO_2Me$ | O | NMe |
| 4-Br | H | 5-Cl | $CO_2Me$ | O | NMe |
| $4-CF_3$ | H | H | $CO_2Me$ | O | NMe |
| $4-OCF_3$ | H | 5-Cl | $CO_2Me$ | O | NMe |
| $4-OCF_2H$ | H | H | $CO_2Me$ | O | NMe |
| 4-Cl | H | 5-Cl | Me | O | NMe |
| 4-F | H | H | Me | O | NMe |
| 4-Br | H | 5-Cl | Me | O | NMe |
| $4-CF_3$ | H | H | Et | O | NMe |
| $4-OCF_2H$ | H | H | allyl | O | NMe |
| 4-Cl | H | 5-Cl | allyl | O | NMe |
| 4-F | H | H | $CH_2$-4-F-Ph | O | NMe |
| 4-Br | H | 5-Cl | $CH_2$-4-Cl-Ph | O | NMe |
| $4-CF_3$ | H | H | $CH_2CF_3$ | O | NMe |
| $4-OCF_3$ | H | 5-Cl | $(CH_2)_4Cl$ | O | NMe |

| $R_1^a$ | $R_1^b$ | $R_2$ | B | X | Z |
|---|---|---|---|---|---|
| 4-$OCF_2H$ | H | H | i-Pr | O | NMe |
| 4-$CF_3$ | H | 5-Cl | OH | O | NMe |
| 4-$OCF_3$ | H | 5-Cl | OAc | O | NMe |
| 4-$OCF_2H$ | 3-Cl | 5-Cl | OMe | O | NMe |
| 4-Cl | H | 5-Cl | C(O)$CH_3$ | O | NMe |
| 4-F | H | 5-Cl | C(O)NHMe | O | NMe |
| 4-Br | H | 5-Cl | C(S)$NMe_2$ | O | NMe |
| 4-$CF_3$ | H | 5-F | Me | O | NMe |
| 4-$OCF_3$ | H | 5-F | n-Bu | O | NMe |
| 4-$OCF_2H$ | 3-Cl | 5-F | allyl | O | NMe |
| 4-Cl | H | 5-F | i-Pr | O | NMe |
| 4-F | H | 5-F | $CH_2Ph$ | O | NMe |
| 4-Br | H | 5-F | Ph | O | NMe |
| 4-$CF_3$ | H | 5-$CF_3$ | $CH_2CH_2F$ | O | NMe |
| 4-$OCF_3$ | H | 5-$CF_3$ | $CH_2SCH_3$ | O | NMe |
| 4-$OCF_2H$ | 3-Cl | 5-$CF_3$ | $CH_2OCH_3$ | O | NMe |
| 4-Cl | H | 5-$CF_3$ | $CH_2CN$ | O | NMe |
| 4-F | H | 5-$CF_3$ | $CH_2CO_2Me$ | O | NMe |
| 4-Br | H | 5-$CF_3$ | $CS_2Me$ | O | NMe |
| 4-$CF_3$ | H | H | Me | S | NMe |
| 4-$OCF_3$ | H | H | Me | S | NMe |
| 4-$OCF_2H$ | 3-Cl | H | Me | S | NMe |
| 4-$CF_3$ | H | H | Me | O | NEt |
| 4-$CF_3$ | H | 5-F | $CO_2Me$ | O | NEt |
| 4-$CF_3$ | H | 5-Cl | Me | O | N-n-Bu |
| 4-$CF_3$ | H | H | $CO_2Me$ | O | N-n-Bu |
| 4-$CF_3$ | H | 5-F | Me | O | N-allyl |
| 4-$CF_3$ | H | 5-Cl | $CO_2Me$ | O | N-allyl |
| 4-$CF_3$ | H | H | Me | O | N-Ph |
| 4-$CF_3$ | H | 5-F | $CO_2Me$ | O | N-Ph |

| $R_1^a$ | $R_1^b$ | $R_2$ | B | X | Z |
|---|---|---|---|---|---|
| 4-$CF_3$ | H | 5-Cl | Me | O | N-4-Cl-Ph |
| 4-$CF_3$ | H | H | $CO_2Me$ | O | N-4-Cl-Ph |

TABLE 6

| $R_1$ | $R_2$ | B | t | Y | |
|---|---|---|---|---|---|
| 4-$CF_3$ | H | H | 0 | Me | |
| 4-Cl | H | Me | 0 | C(O)Me | |
| 4-$OCF_2H$ | H | $CO_2Me$ | 0 | n-Bu | |
| 4-$CF_3$ | H | C(O)Me | 0 | SMe | |
| 4-Cl | H | $CO_2Et$ | 0 | $SCCl_3$ | |
| 4-$OCF_3$ | H | H | 1 | Me | |
| 4-$CF_3$ | H | H | 1 | C(O)Me | oil |
| 4-F | H | $CO_2Me$ | 1 | Me | |
| 4-$OCF_2H$ | H | $CO_2Me$ | 1 | C(O)Me | |
| 4-$CF_3$ | H | Me | 1 | $C(O)CF_3$ | |
| 4-Cl | H | Me | 1 | $CO_2Et$ | |
| 4-$CF_3$ | H | $CO_2CH_2CF_3$ | 1 | Me | |
| 4-$OCF_2H$ | H | $CO_2Et$ | 1 | Me | |
| 4-$OCF_3$ | H | $CO_2CH_2CH_2Cl$ | 1 | Me | |
| 4-Br | H | CONHMe | 1 | Me | |
| 4-$SCF_2H$ | H | H | 2 | 2-$NO_2$PhS | |
| 4-$CF_3$ | H | Me | 2 | $CO_2Me$ | |
| 4-Cl | H | Me | 2 | C(O)Me | |
| 4-$CF_3$ | H | $CO_2Me$ | 2 | C(O)Me | |
| 4-Cl | H | $CO_2Me$ | 2 | Me | |
| 4-$CF_3$ | H | $CO_2Me$ | 1 | C(O)Me | 110 to 115 |
| 4-$CF_3$ | H | $CO_2Me$ | 1 | Me | glass |
| 4-$CF_3$ | H | $CO_2Me$ | 1 | $CO_2Me$ | glass |
| 4-$CF_3$ | 5-Cl | $CO_2Me$ | 1 | C(O)Me | |
| 4-$CF_3$ | 5-Cl | $CO_2Me$ | 1 | Me | |
| 4-$CF_3$ | 5-Cl | $CO_2Me$ | 1 | $CO_2Me$ | |
| 4-$CF_3$ | 4-F | $CO_2Me$ | 1 | C(O)Me | |
| 4-$CF_3$ | 5-F | $CO_2Me$ | 1 | C(O)Me | |
| 4-$CF_3$ | 4-F | $CO_2Me$ | 1 | $CO_2Me$ | |
| 4-$CF_3$ | 5-F | $CO_2Me$ | 1 | $CO_2Me$ | |
| 4-$CF_3$ | H | H | 1 | $CO_2$-t-Bu | 178 to 179 |

TABLE 7

| $R_1$ | $R_2$ | B | t | y | V | |
|---|---|---|---|---|---|---|
| 4-$CF_3$ | H | Me | 0 | Me | O | |
| 4-$CF_3$ | H | Me | 1 | Me | NMe | |
| 4-$CF_3$ | 5-Cl | $CO_2Me$ | 2 | Me | S | |
| 4-$CF_3$ | 5-Cl | $CO_2Me$ | 0 | Me | O | |
| 4-$CF_3$ | 5-Cl | Me | 1 | Me | NMe | |
| 4-$CF_3$ | 5-Cl | Me | 2 | C(O)Me | S | |
| 4-$CF_3$ | 5-F | $CO_2Me$ | 0 | C(O)Me | O | |
| 4-$CF_3$ | 5-F | $CO_2Me$ | 1 | C(O)Me | NMe | |
| 4-$CF_3$ | 5-F | Me | 2 | C(O)Me | SO | |
| 4-$CF_3$ | 5-F | Me | 0 | C(O)Me | $SO_2$ | |
| 4-$CF_3$ | H | $CO_2Me$ | 1 | $SCCl_3$ | O | |
| 4-$CF_3$ | H | $CO_2Me$ | 2 | $SCCl_3$ | NMe | |
| 4-$CF_3$ | H | Me | 0 | $CO_2Me$ | S | |
| 4-$CF_3$ | H | Me | 1 | $CO_2Me$ | O | |
| 4-$CF_3$ | 5-$OCF_2H$ | $CO_2Me$ | 2 | n-Pr | NMe | |
| 4-$CF_3$ | 5-$OCF_2H$ | $CO_2Me$ | 0 | n-Pr | S | |
| 4-$CF_3$ | 5-$CF_3$ | Me | 1 | C(O)$CF_3$ | O | |
| 4-$CF_3$ | 5-$CF_3$ | Me | 2 | C(O)$CF_3$ | NMe | |
| 4-$CF_3$ | 5-Cl | $CO_2Me$ | 0 | SMe | S | |
| 4-$CF_3$ | 5-Cl | $CO_2Me$ | 1 | SMe | O | |
| 4-$CF_3$ | H | H | 1 | Me | S | 151 to 152 |
| 4-$CF_3$ | H | H | 1 | $CO_2Me$ | S | 150 to 158 |
| 4-$CF_3$ | H | H | 1 | C(O)Me | S | 80 to 85 |
| 4-$CF_3$ | 5-Cl | H | 1 | C(O)Me | S | 97 to 99 |
| 4-$CF_3$ | 5-Cl | H | 1 | $CO_2Me$ | S | 170 to 172 |
| 4-$CF_3$ | 5-Cl | H | 1 | Me | S | 180 to 183 |
| 4-Cl | H | H | 1 | C(O)Me | S | 140 to 150 |
| 4-Cl | H | H | 1 | Me | S | 125 to 127 |
| 4-Cl | 5-Cl | H | 1 | C(O)Me | S | 127 to 142 |
| 4-Cl | 5-Cl | H | 1 | Me | S | 160 to 165 |

TABLE 8

| $R_1$ | $R_2$ | B | t | Y | V |
|---|---|---|---|---|---|
| 4-$CF_3$ | H | Me | 0 | Me | O |
| 4-$CF_3$ | H | Me | 1 | Me | NMe |
| 4-$CF_3$ | 5-Cl | $CO_2Me$ | 2 | Me | S |
| 4-$CF_3$ | 5-Cl | $CO_2Me$ | 0 | Me | O |
| 4-$CF_3$ | 5-Cl | Me | 1 | Me | NMe |
| 4-$CF_3$ | 5-Cl | Me | 2 | C(O)Me | S |
| 4-$CF_3$ | 5-F | $CO_2Me$ | 0 | C(O)Me | O |
| 4-$CF_3$ | 5-F | $CO_2Me$ | 1 | C(O)Me | NMe |
| 4-$CF_3$ | 5-F | Me | 2 | C(O)Me | SO |
| 4-$CF_3$ | 5-F | Me | 0 | C(O)Me | $SO_2$ |
| 4-$CF_3$ | H | $CO_2Me$ | 1 | $SCCl_3$ | O |
| 4-$CF_3$ | H | $CO_2Me$ | 2 | $SCCl_3$ | NMe |
| 4-$CF_3$ | H | Me | 0 | $CO_2Me$ | S |
| 4-$CF_3$ | H | Me | 1 | $CO_2Me$ | O |
| 4-$CF_3$ | 5-$OCF_2H$ | $CO_2Me$ | 2 | n-Pr | NMe |
| 4-$CF_3$ | 5-$OCF_2H$ | $CO_2Me$ | 0 | n-Pr | S |
| 4-$CF_3$ | 5-$CF_3$ | Me | 1 | C(O)$CF_3$ | O |
| 4-$CF_3$ | 5-$CF_3$ | Me | 2 | C(O)$CF_3$ | NMe |
| 4-$CF_3$ | 5-Cl | $CO_2Me$ | 0 | SMe | S |
| 4-$CF_3$ | 5-Cl | $CO_2Me$ | 1 | SMe | O |

TABLE 9

| $R_1$ | $R_2$ | B | Y | Z |
|---|---|---|---|---|
| 4-$CF_3$ | H | Me | Me | O |
| 4-Cl | H | Me | C(O)Me | NMe |
| 4-$CF_3$ | H | Me | Et | O |
| 4-Cl | 5-Cl | Me | $CO_2$Me | NMe |
| 4-$CF_3$ | 5-Cl | Et | n-Pr | O |
| 4-Cl | 5-F | Et | $CO_2$Et | NMe |
| 4-$CF_3$ | 5-F | Et | C(O)Me | O |
| 4-Cl | 5-Cl | Et | S-$CCl_3$ | NMe |
| 4-$CF_3$ | 5-Cl | $(CH_2)_3$Cl | SPh | O |
| 4-Cl | H | $(CH_2)_3$Cl | Me | NMe |
| 4-$CF_3$ | H | $CH_2$Ph | Me | O |
| 4-Cl | H | $CH_2$Ph | C(O)Me | NMe |
| 4-$CF_3$ | 5-$OCF_3$ | Ph | C(O)$CF_3$ | O |
| 4-Cl | 5-OMe | Ph | 2-$NO_2$PhS | NMe |
| 4-$CF_3$ | 5-Cl | 4-Cl-Ph | Me | O |
| 4-Cl | 5-F | 4-Cl-Ph | Et | NMe |
| 4-$CF_3$ | H | allyl | C(O)Me | O |
| 4-Cl | H | allyl | $CO_2$Et | NMe |
| 4-$CF_3$ | 5-Cl | OH | $SCCl_3$ | O |
| 4-Cl | H | OH | Me | NMe |
| 4-$CF_3$ | H | OAc | C(O)Me | O |
| 4-Cl | H | OAc | Me | NMe |

TABLE 10

| $R_1$ | $R_2$ | B | Y | Z |
|---|---|---|---|---|
| 4-$CF_3$ | H | Me | Me | O |
| 4-Cl | H | Me | C(O)Me | NMe |
| 4-$CF_3$ | H | Me | Et | O |
| 4-Cl | 5-Cl | Me | $CO_2$Me | NMe |
| 4-$CF_3$ | 5-Cl | Et | n-Pr | O |
| 4-Cl | 5-F | Et | $CO_2$Et | NMe |
| 4-$CF_3$ | 5-F | Et | C(O)Me | O |
| 4-Cl | 5-Cl | Et | S-$CCl_3$ | NMe |
| 4-$CF_3$ | 5-Cl | $(CH_2)_3$Cl | SPh | O |
| 4-Cl | H | $(CH_2)_3$Cl | Me | NMe |
| 4-$CF_3$ | H | $CH_2$Ph | Me | O |
| 4-Cl | H | $CH_2$Ph | C(O)Me | NMe |
| 4-$CF_3$ | 5-$OCF_3$ | Ph | C(O)$CF_3$ | O |
| 4-Cl | 5-OMe | Ph | 2-$NO_2$PhS | NMe |
| 4-$CF_3$ | 5-Cl | 4-Cl-Ph | Me | O |
| 4-Cl | 5-F | 4-Cl-Ph | Et | NMe |
| 4-$CF_3$ | H | allyl | C(O)Me | O |
| 4-Cl | H | allyl | $CO_2$Et | NMe |
| 4-$CF_3$ | 5-Cl | OH | $SCCl_3$ | O |
| 4-Cl | H | OH | Me | NMe |
| 4-$CF_3$ | H | OAc | C(O)Me | O |
| 4-Cl | H | OAc | Me | NMe |

TABLE 11

| $R_1$ | $R_2$ | B | t | J | K | |
|---|---|---|---|---|---|---|
| 4-$CF_3$ | H | H | 0 | Me | H | |
| 4-Cl | H | H | 1 | Me | H | |
| 4-$CF_3$ | H | Me | 2 | Me | H | |
| 4-Cl | 5-Cl | Me | 0 | Ph | H | |
| 4-$CF_3$ | 5-Cl | $CO_2Me$ | 1 | Ph | H | |
| 4-Cl | 5-Cl | $CO_2Me$ | 2 | 4-Cl-Ph | H | |
| 4-$CF_3$ | H | H | 0 | 4-F-Ph | H | |
| 4-Cl | H | H | 1 | 4-$CF_3$-Ph | H | |
| 4-$CF_3$ | H | Me | 2 | Et | H | |
| 4-Cl | 5-F | Me | 0 | n-Pr | H | |
| 4-$CF_3$ | 5-F | $CO_2Me$ | 1 | n-Bu | H | |
| 4-Cl | 5-F | $CO_2Me$ | 2 | Me | Me | |
| 4-$CF_3$ | H | H | 0 | Me | Me | |
| 4-Cl | H | H | 1 | Me | Me | |
| 4-$CF_3$ | H | Me | 2 | Me | Me | |
| 4-Cl | 5-Cl | Me | 0 | Ph | Me | |
| 4-$CF_3$ | 5-Cl | $CO_2Me$ | 1 | Ph | Me | |
| 4-Cl | 5-Cl | $CO_2Me$ | 2 | Ph | Me | |
| 4-$CF_3$ | H | H | 0 | Ph | Me | |
| 4-Cl | H | H | 1 | Et | Me | |
| 4-$CF_3$ | H | Me | 2 | n-Pr | Me | |
| 4-Cl | H | Me | 0 | n-Bu | Me | |
| 4-$CF_3$ | H | H | 1 | 4-F-Ph | H | 199 to 201 |
| 4-$CF_3$ | H | H | 1 | 4-Cl-Ph | H | 187 to 188 |
| 4-$CF_3$ | 5-Cl | H | 1 | 4-F-Ph | H | |
| 4-$CF_3$ | 5-Cl | H | 1 | 4-Cl-Ph | H | |
| 4-$CF_3$ | 4-F | H | 1 | 4-F-Ph | H | |
| 4-$CF_3$ | 4-F | H | 1 | 4-Cl-Ph | H | |
| 4-$CF_3$ | 5-$OCF_2H$ | H | 1 | 4-F-Ph | H | |
| 4-$CF_3$ | 5-$OCF_2H$ | H | 1 | 4-Cl-Ph | H | |
| 4-Cl | H | H | 1 | 4-F-Ph | H | 208 to 209 |
| 4-Cl | H | H | 1 | 4-Cl-Ph | H | 179 to 181 |

TABLE 12

| $R_1$ | $R_2$ | B | t | J | K | V |
|---|---|---|---|---|---|---|
| 4-$CF_3$ | H | H | 0 | Me | H | O |
| 4-$CF_3$ | H | Me | 1 | Me | H | O |
| 4-$CF_3$ | 4-F | Et | 2 | Ph | H | O |
| 4-$CF_3$ | H | $CO_2Me$ | 0 | Ph | Me | O |
| 4-$CF_3$ | H | $CO_2Et$ | 1 | Me | Me | O |
| 4-$CF_3$ | H | H | 2 | Me | Me | O |
| 4-$CF_3$ | 4-Cl | Me | 0 | 4-Cl-Ph | Me | O |
| 4-$CF_3$ | H | Et | 1 | Me | H | S |
| 4-$CF_3$ | H | $CO_2Me$ | 2 | Me | H | S |
| 4-$CF_3$ | 4-Br | $CO_2Et$ | 0 | Ph | H | S |
| 4-$CF_3$ | H | H | 1 | Ph | Me | S |
| 4-$CF_3$ | H | Me | 2 | Me | Me | S |
| 4-$CF_3$ | H | Et | 0 | Me | Me | S |
| 4-$CF_3$ | H | $CO_2Me$ | 1 | 4-F-Ph | Me | S |
| 4-$CF_3$ | H | $CO_2Et$ | 2 | Me | H | NMe |
| 4-$CF_3$ | H | H | 0 | Me | H | NMe |
| 4-$CF_3$ | 5-Cl | Me | 1 | Ph | H | NMe |
| 4-$CF_3$ | H | Et | 2 | Ph | Me | NMe |
| 4-$CF_3$ | 5-Me | $CO_2Me$ | 0 | Me | Me | NMe |
| 4-$CF_3$ | H | $CO_2Et$ | 1 | Me | Me | NMe |
| 4-$CF_3$ | H | H | 2 | 4-Cl-Ph | Me | NMe |

TABLE 13

| $R_1$ | $R_2$ | B | t | J | K | Y |
|---|---|---|---|---|---|---|
| 4-$CF_3$ | H | H | 0 | Me | H | O |
| 4-$CF_3$ | H | Me | 1 | Me | H | O |
| 4-$CF_3$ | 4-F | Et | 2 | Ph | H | O |
| 4-$CF_3$ | H | $CO_2$Me | 0 | Ph | Me | O |
| 4-$CF_3$ | H | $CO_2$Et | 1 | Me | Me | O |
| 4-$CF_3$ | H | H | 2 | Me | Me | O |
| 4-$CF_3$ | 4-Cl | Me | 0 | 4-Cl-Ph | Me | O |
| 4-$CF_3$ | H | Et | 1 | Me | H | S |
| 4-$CF_3$ | H | $CO_2$Me | 2 | Me | H | S |
| 4-$CF_3$ | 4-Br | $CO_2$Et | 0 | Ph | H | S |
| 4-$CF_3$ | H | H | 1 | Ph | Me | S |
| 4-$CF_3$ | H | Me | 2 | Me | Me | S |
| 4-$CF_3$ | H | Et | 0 | Me | Me | S |
| 4-$CF_3$ | H | $CO_2$Me | 1 | 4-F-Ph | Me | S |
| 4-$CF_3$ | H | $CO_2$Et | 2 | Me | H | NMe |
| 4-$CF_3$ | H | H | 0 | Me | H | NMe |
| 4-$CF_3$ | 5-Cl | Me | 1 | Ph | H | NMe |
| 4-$CF_3$ | H | Et | 2 | Ph | Me | NMe |
| 4-$CF_3$ | 5-Me | $CO_2$Me | 0 | Me | Me | NMe |
| 4-$CF_3$ | H | $CO_2$Et | 1 | Me | Me | NMe |
| 4-$CF_3$ | H | H | 2 | 4-Cl-Ph | Me | NMe |

TABLE 14

| $R_1$ | $R_2$ | B | J | K | Z |
|---|---|---|---|---|---|
| 4-$CF_3$ | H | H | Me | H | O |
| 4-Cl | H | Me | Me | Me | O |
| 4-$OCF_2H$ | H | Me | Ph | H | O |
| 4-$CF_3$ | H | allyl | 4-Cl-Ph | Me | NMe |
| 4-Cl | H | allyl | Me | H | NMe |
| 4-$OCF_2H$ | 5-F | $CH_2Ph$ | 4-F-Ph | Me | NMe |
| 4-$CF_3$ | H | $CH_2Ph$ | Me | H | O |
| 4-Cl | H | Ph | Me | Me | O |
| 4-$OCF_2H$ | H | Ph | Ph | H | O |
| 4-$CF_3$ | H | 4-F-Ph | Ph | Me | NMe |
| 4-Cl | H | 4-F-Ph | Me | H | NMe |
| 4-$OCF_2H$ | 5-Cl | OH | Me | Me | NMe |
| 4-$CF_3$ | H | OH | Me | H | O |
| 4-Cl | H | OMe | Et | Me | O |
| 4-$OCF_2H$ | H | OMe | Ph | H | O |
| 4-$CF_3$ | H | $CO_2Et$ | 4-Cl-Ph | Me | NMe |
| 4-Cl | H | $CO_2Et$ | Me | H | NMe |
| 4-$OCF_2H$ | 5-Cl | CONHMe | Ph | Me | NMe |
| 4-$CF_3$ | H | CONHMe | Me | H | O |
| 4-Cl | H | n-Bu | Ph | Me | O |
| 4-$OCF_2H$ | H | n-Bu | Me | H | O |

TABLE 15

| $R_1$ | $R_2$ | B | J | K | Z |
|---|---|---|---|---|---|
| 4-$CF_3$ | H | Me | Me | H | O |
| 4-Cl | H | $CO_2Me$ | Me | H | O |
| 4-$OCF_2H$ | H | H | Me | H | O |
| 4-$CF_3$ | H | Me | Ph | H | O |
| 4-Cl | H | $CO_2Me$ | Ph | H | O |
| 4-$OCF_2H$ | H | H | Ph | H | O |
| 4-$CF_3$ | 5-Cl | Et | 4-Cl-Ph | H | NMe |
| 4-Cl | H | $CO_2Et$ | 4-Cl-Ph | H | NMe |
| 4-$COF_2H$ | H | $(CH_2)_3Cl$ | 4-Cl-Ph | H | NMe |
| 4-$CF_3$ | H | Me | Me | H | NMe |
| 4-Cl | H | H | Me | H | NMe |
| 4-$OCF_2H$ | H | $CO_2Me$ | Me | Me | O |
| 4-$CF_3$ | H | H | Me | Me | O |
| 4-Cl | H | Me | Me | Me | O |
| 4-$OCF_2H$ | 5-F | C(O)Me | Ph | Me | O |
| 4-$CH_3$ | H | C(O)Et | Ph | Me | O |
| 4-Cl | H | Me | Me | Me | NMe |
| 4-$OCF_2H$ | H | Et | Me | Me | NMe |
| 4-$CF_3$ | H | n-Pr | Ph | Me | NMe |
| 4-Cl | H | $CO_2Me$ | Ph | Me | NMe |
| 4-$OCF_2H$ | H | Me | 4-Cl-Ph | Me | NMe |
| 4-$CF_3$ | H | Et | 4-Cl-Ph | Me | NMe |

TABLE 16

| $R_1$ | $R_2$ | B | $R_8^a$ | $R_8^b$ | $R_8^c$ | $R_8^d$ | |
|---|---|---|---|---|---|---|---|
| 4-$CF_3$ | H | H | Me | H | H | H | 152 to 159 |
| 4-Cl | H | H | Me | H | H | H | 120 to 123 |
| 4-Br | H | H | Me | H | H | H | 124 to 126 |
| 4-$OCF_2H$ | H | H | Me | H | H | H | |
| 4-$CF_3$ | H | $CO_2Me$ | Me | H | H | H | 170 to 172 |
| 4-Cl | H | $CO_2Me$ | Me | H | H | H | 166 to 170 |
| 4-Br | H | $CO_2Me$ | Me | H | H | H | 185 to 188 |
| 4-$OCF_2H$ | H | $CO_2Me$ | Me | H | H | H | |
| 4-$CF_3$ | 5-Cl | H | Me | H | H | H | |
| 4-Cl | 5-Cl | H | Me | H | H | H | |
| 4-Br | 5-Cl | H | Me | H | H | H | |
| 4-$OCF_2H$ | 5-Cl | H | Me | H | H | H | |
| 4-$CF_3$ | 5-Cl | $CO_2Me$ | Me | H | H | H | |
| 4-Cl | 5-Cl | $CO_2Me$ | Me | H | H | H | |
| 4-Br | 5-Cl | $CO_2Me$ | Me | H | H | H | |
| 4-$OCF_2H$ | 4-F | $CO_2Me$ | Me | H | H | H | |
| 4-$CF_3$ | 4-F | H | Me | H | H | H | |
| 4-Cl | 4-F | H | Me | H | H | H | |
| 4-Br | 4-F | H | Me | H | H | H | |
| 4-$OCF_2H$ | 4-F | H | Me | H | H | H | |
| 4-$CF_3$ | 4-F | $CO_2Me$ | Me | H | H | H | |
| 4-Cl | 4-F | $CO_2Me$ | Me | H | H | H | |
| 4-Br | 4-F | $CO_2Me$ | Me | H | H | H | |
| 4-$OCF_2H$ | 4-F | $CO_2Me$ | Me | H | H | H | |
| 4-$CF_3$ | H | H | Me | Me | H | H | |
| 4-Cl | H | H | Me | Me | H | H | |
| 4-Br | H | H | Me | Me | H | H | |
| 4-$OCF_3$ | H | H | Me | Me | H | H | |
| 4-$CF_3$ | H | $CO_2Me$ | Me | Me | H | H | |
| 4-Cl | H | $CO_2Me$ | Me | Me | H | H | |
| 4-Br | H | $CO_2Me$ | Me | Me | H | H | |
| 4-$OCF_3$ | H | $CO_2Me$ | Me | Me | H | H | |

| $R_1$ | $R_2$ | B | $R_8^a$ | $R_8^b$ | $R_8^c$ | $R_8^d$ |
|---|---|---|---|---|---|---|
| 4-$CF_3$ | 5-$OCF_2H$ | H | Me | Me | H | H |
| 4-Cl | 5-$OCF_2H$ | H | Me | Me | H | H |
| 4-Br | 5-$OCF_2H$ | H | Me | Me | H | H |
| 4-$OCF_3$ | 5-$OCF_2H$ | H | Me | Me | H | H |
| 4-$CF_3$ | 5-$OCF_2H$ | $CO_2Me$ | Me | Me | H | H |
| 4-Cl | 5-$OCF_2H$ | $CO_2Me$ | Me | Me | H | H |
| 4-Br | 5-$OCF_2H$ | $CO_2Me$ | Me | Me | H | H |
| 4-$OCF_3$ | 5-$OCF_2H$ | $CO_2Me$ | Me | Me | H | H |
| 4-$CF_3$ | 5-Cl | H | Me | Me | H | H |
| 4-Cl | 5-Cl | H | Me | Me | H | H |
| 4-Br | 5-Cl | H | Me | Me | H | H |
| 4-$OCF_3$ | 5-Cl | H | Me | Me | H | H |
| 4-$CF_3$ | 5-Cl | $CO_2Me$ | Me | Me | H | H |
| 4-Cl | 5-Cl | $CO_2Me$ | Me | Me | H | H |
| 4-Br | 5-Cl | $CO_2Me$ | Me | Me | H | H |
| 4-$OCF_3$ | 5-Cl | $CO_2Me$ | Me | Me | H | H |
| 4-$CF_3$ | H | H | H | Me | H | H |
| 4-Cl | H | H | H | Me | H | H |
| 4-$CF_3$ | H | $CO_2Me$ | H | Me | H | H |
| 4-Cl | H | $CO_2Me$ | H | Me | H | H |
| 4-$CF_3$ | 5-Cl | H | H | Me | H | H |
| 4-Cl | 5-Cl | H | H | Me | H | H |
| 4-$CF_3$ | 5-Cl | $CO_2Me$ | H | Me | H | H |
| 4-Cl | 5-Cl | $CO_2Me$ | H | Me | H | H |
| 4-$CF_3$ | H | H | H | H | Me | H |
| 4-Cl | H | H | H | H | Me | H |
| 4-$CF_3$ | H | $CO_2Me$ | H | H | Me | H |
| 4-Cl | H | $CO_2Me$ | H | H | Me | H |
| 4-$CF_3$ | 5-Cl | H | H | H | Me | H |
| 4-Cl | 5-Cl | H | H | H | Me | H |
| 4-$CF_3$ | 5-Cl | $CO_2Me$ | H | H | Me | H |

| $R_1$ | $R_2$ | B | $R_8^a$ | $R_8^b$ | $R_8^c$ | $R_8^d$ |
|---|---|---|---|---|---|---|
| 4-Cl | 5-Cl | $CO_2Me$ | H | H | Me | H |
| 4-$CF_3$ | H | H | H | H | H | Me |
| 4-Cl | H | H | H | H | H | Me |
| 4-$CF_3$ | H | $CO_2Me$ | H | H | H | Me |
| 4-Cl | H | $CO_2Me$ | H | H | H | Me |
| 4-$CF_3$ | 5-Cl | H | H | H | H | Me |
| 4-Cl | 5-Cl | H | H | H | H | Me |
| 4-$CF_3$ | 5-Cl | $CO_2Me$ | H | H | H | Me |
| 4-Cl | 5-Cl | $CO_2Me$ | H | H | H | Me |
| $CF_3$ | H | H | H | H | Me | Me |
| Cl | H | H | H | H | Me | Me |
| Br | H | H | H | H | Me | Me |
| $CF_3$ | H | $CO_2Me$ | H | H | Me | Me |
| Cl | H | $CO_2Me$ | H | H | Me | Me |
| Br | H | $CO_2Me$ | H | H | Me | Me |
| $CF_3$ | 5-Cl | H | H | H | Me | Me |
| Cl | 5-Cl | H | H | H | Me | Me |
| Br | 5-Cl | H | H | H | Me | Me |
| $CF_3$ | 5-Cl | $CO_2Me$ | H | H | Me | Me |
| Cl | 5-Cl | $CO_2Me$ | H | H | Me | Me |
| Br | 5-Cl | $CO_2Me$ | H | H | Me | Me |
| $CF_3$ | 5-$OCF_2H$ | H | H | H | Me | Me |
| Cl | 5-$OCF_2H$ | H | H | H | Me | Me |
| Br | 5-$OCF_2H$ | H | H | H | Me | Me |
| $CF_3$ | 5-$OCF_2H$ | $CO_2Me$ | H | H | Me | Me |
| Cl | 5-$OCF_2H$ | $CO_2Me$ | H | H | Me | Me |
| Br | 5-$OCF_2H$ | $CO_2Me$ | H | H | Me | Me |
| $CF_3$ | 4-F | H | H | H | Me | Me |
| Cl | 4-F | H | H | H | Me | Me |
| Br | 4-F | H | H | H | Me | Me |
| $CF_3$ | 4-F | $CO_2Me$ | H | H | Me | Me |
| Cl | 4-F | $CO_2Me$ | H | H | Me | Me |

| $R_1$ | $R_2$ | B | $R_8^a$ | $R_8^b$ | $R_8^c$ | $R_8^d$ | |
|---|---|---|---|---|---|---|---|
| Br | 4-F | $CO_2Me$ | H | H | Me | Me | |
| 4-$CF_3$ | H | $CO_2Me$ | i-Pr | H | H | H | |
| 4-$CF_3$ | H | $CO_2Me$ | H | i-Pr | H | H | |
| 4-$CF_3$ | H | $CO_2Me$ | H | H | Ph | H | |
| 4-$CF_3$ | H | $CO_2Me$ | H | H | H | Ph | |
| 4-$CF_3$ | 5-Cl | $CO_2Me$ | Cl | H | H | H | |
| 4-$CF_3$ | 5-Cl | $CO_2Me$ | H | Cl | H | H | |
| 4-$CF_3$ | 5-Cl | $CO_2Me$ | F | H | H | H | |
| 4-$CF_3$ | 5-Cl | $CO_2Me$ | H | F | H | H | |
| 4-$CF_3$ | 5-F | $CO_2Me$ | H | H | $CO_2Me$ | H | |
| 4-$CF_3$ | 5-F | $CO_2Me$ | H | H | H | $CO_2Me$ | |
| 4-$CF_3$ | H | $CO_2H$ | Me | H | H | H | 207 to 209 |
| 4-$CF_3$ | 5-F | $CO_2Me$ | Et | H | H | H | |
| 4-$CF_3$ | 5-F | $CO_2Me$ | H | Et | H | H | |
| 4-$CF_3$ | 5-$OCF_2H$ | $CO_2Me$ | Ph | H | H | H | |
| 4-$CF_3$ | 5-$OCF_2H$ | $CO_2Me$ | H | Ph | H | H | |
| 4-$CF_3$ | 5-$OCF_2H$ | $CO_2Me$ | H | Me | Me | H | |
| 4-$CF_3$ | 5-$OCF_2H$ | $CO_2Me$ | Me | H | H | Me | |
| 4-$CF_3$ | 4-Cl | $CO_2Me$ | i-Pr | H | H | H | |
| 4-$CF_3$ | 4-Cl | $CO_2Me$ | H | i-Pr | H | H | |
| 4-$CF_3$ | 4-Cl | $CO_2Me$ | Et | Et | H | H | |
| 4-$CF_3$ | 4-Cl | $CO_2Me$ | Et | Me | H | H | |
| 4-$CF_3$ | 4-F | $CO_2Me$ | Br | H | H | H | |
| 4-$CF_3$ | 4-F | $CO_2Me$ | H | Br | H | H | |
| 4-$CF_3$ | 4-F | $CO_2Me$ | i-Pr | H | H | H | |
| 4-$CF_3$ | 4-F | $CO_2Me$ | H | i-Pr | H | H | |

TABLE 17

| $R_1$ | $R_2$ | B | V | $R_8^a$ | $R_8^b$ |
|---|---|---|---|---|---|
| 4-$CF_3$ | H | H | O | Me | H |
| 4-Cl | H | H | O | Me | H |
| 4-Br | H | H | O | Me | H |
| 4-$OCF_2H$ | H | H | O | Me | H |
| 4-$CF_3$ | H | H | O | H | Me |
| 4-Cl | H | H | O | H | Me |
| 4-Br | H | H | O | H | Me |
| 4-$OCF_2H$ | H | H | O | H | Me |
| 4-$CF_3$ | H | H | O | Me | Me |
| 4-Cl | H | H | O | Me | Me |
| 4-Br | H | H | O | Me | Me |
| 4-$OCF_2H$ | H | H | O | Me | Me |
| 4-$CF_3$ | H | H | O | Ph | H |
| 4-Cl | H | H | O | Ph | H |
| 4-Br | H | H | O | Ph | H |
| 4-$OCF_2H$ | H | H | O | Ph | H |
| 4-$CF_3$ | H | H | O | H | Ph |
| 4-Cl | H | H | O | H | Ph |
| 4-Br | H | H | O | H | Ph |
| 4-$OCF_2H$ | H | H | O | H | Ph |
| 4-$CF_3$ | H | H | O | $CO_2Me$ | H |
| 4-Cl | H | H | O | $CO_2Me$ | H |
| 4-Br | H | H | O | H | $CO_2Me$ |
| 4-$OCF_2H$ | H | H | O | H | CO Me |
| 4-$CF_3$ | 5-Cl | H | O | Me | H |
| 4-Cl | 5-Cl | H | O | Me | H |
| 4-Br | 5-Cl | H | O | Me | H |
| 4-$OCF_2H$ | H | H | O | Me | H |
| 4-$CF_3$ | 5-Cl | H | O | H | Me |
| 4-Cl | 5-Cl | H | O | H | Me |
| 4-Br | 5-Cl | H | O | H | Me |
| 4-$OCF_2H$ | H | H | O | H | Me |
| 4-$CF_3$ | 5-Cl | H | O | Me | Me |
| 4-Cl | 5-Cl | H | O | Me | Me |
| 4-Br | 5-Cl | H | O | Me | Me |

| $R_1$ | $R_2$ | B | Y | $R_8^a$ | $R_8^b$ | |
|---|---|---|---|---|---|---|
| 4-$OCF_2H$ | H | H | O | Me | Me | |
| 4-$CF_3$ | 5-Cl | H | O | Ph | H | |
| 4-Cl | 5-Cl | H | O | Ph | H | |
| 4-Br | 5-Cl | H | O | Ph | H | |
| 4-$OCF_2H$ | H | H | O | Ph | H | |
| 4-$CF_3$ | 5-Cl | H | O | H | Ph | |
| 4-Cl | 5-Cl | H | O | H | Ph | |
| 4-Br | 5-Cl | H | O | H | Ph | |
| 4-$OCF_2H$ | H | H | O | H | Ph | |
| 4-$CF_3$ | 5-Cl | H | O | $CO_2Me$ | H | |
| 4-Cl | 5-Cl | H | O | $CO_2Me$ | H | |
| 4-Br | 5-Cl | H | O | H | $CO_2Me$ | |
| 4-$OCF_2H$ | H | H | O | H | $CO_2Me$ | |
| 4-$CF_3$ | H | H | S | Me | H | 208 to 210 |
| 4-Cl | H | H | S | Me | H | 172 to 178 |
| 4-Br | H | H | S | Me | H | 185 to 187 |
| 4-$OCF_2H$ | H | H | S | Me | H | |
| 4-$CF_3$ | H | H | S | H | Me | 168 to 170 |
| 4-Cl | H | H | S | H | Me | 155 to 156 |
| 4-Br | H | H | S | H | Me | 172 to 180 |
| 4-$OCF_2H$ | H | H | S | H | Me | |
| 4-$CF_3$ | H | H | S | Me | Me | 183 to 184 |
| 4-Cl | H | H | S | Me | Me | 181 to 186 |
| 4-Br | H | H | S | Me | Me | 182 to 185 |
| 4-$OCF_2H$ | H | H | S | Me | Me | |
| 4-$CF_3$ | H | H | S | Ph | H | |
| 4-Cl | H | H | S | Ph | H | |
| 4-Br | H | H | S | Ph | H | |
| 4-$OCF_2H$ | H | H | S | Ph | H | |
| 4-$CF_3$ | H | H | S | H | Ph | |

| $R_1$ | $R_2$ | B | V | $R_8^a$ | $R_8^b$ | |
|---|---|---|---|---|---|---|
| 4-Cl | H | H | S | H | Ph | |
| 4-Br | H | H | S | H | Ph | |
| 4-$OCF_2H$ | H | H | S | H | Ph | |
| 4-$CF_3$ | H | H | S | $CO_2Me$ | H | |
| 4-Cl | H | H | S | $CO_2Me$ | H | |
| 4-Br | H | H | S | H | $CO_2Me$ | |
| 4-$OCF_2H$ | H | H | S | H | $CO_2Me$ | |
| 4-$CF_3$ | 5-Cl | H | S | Me | H | 251 to 255 |
| 4-Cl | 5-Cl | H | S | Me | H | 222 to 226 |
| 4-Br | 5-Cl | H | S | Me | H | 228 to 230 |
| 4-$OCF_2H$ | 5-Cl | H | S | Me | H | |
| 4-$CF_3$ | 5-Cl | H | S | H | Me | 171 to 173 |
| 4-Cl | 5-Cl | H | S | H | Me | 170 to 176 |
| 4-Br | 5-Cl | H | S | H | Me | 120 to 125 |
| 4-$OCF_2H$ | 5-Cl | H | S | H | Me | |
| 4-$CF_3$ | 5-Cl | H | S | Me | Me | 200 to 202 |
| 4-Cl | 5-Cl | H | S | Me | Me | 190 to 193 |
| 4-Br | 5-Cl | H | S | Me | Me | 204 to 207 |
| 4-$OCF_2H$ | 5-Cl | H | S | Me | Me | |
| 4-$CF_3$ | 5-Cl | H | S | Ph | H | |
| 4-Cl | 5-Cl | H | S | Ph | H | |
| 4-Br | 5-Cl | H | S | Ph | H | |
| 4-$OCF_2H$ | 5-Cl | H | S | Ph | H | |
| 4-$CF_3$ | 5-Cl | H | S | H | Ph | |
| 4-Cl | 5-Cl | H | S | H | Ph | |
| 4-Br | 5-Cl | H | S | H | Ph | |
| 4-$OCF_2H$ | 5-Cl | H | S | H | Ph | |
| 4-$CF_3$ | 5-Cl | H | S | $CO_2Me$ | H | |
| 4-Cl | 5-Cl | H | S | $CO_2Me$ | H | |
| 4-Br | 5-Cl | H | S | H | $CO_2Me$ | |
| 4-$OCF_2H$ | 5-Cl | H | S | H | $CO_2Me$ | |

| $R_1$ | $R_2$ | B | V | $R_8^a$ | $R_8^b$ | |
|---|---|---|---|---|---|---|
| 4-$CF_3$ | 5-Br | H | S | Me | H | |
| 4-Cl | 5-Br | H | S | Me | H | |
| 4-Br | 5-Br | H | S | Me | H | |
| 4-$CF_3$ | 5-F | H | S | Me | H | |
| 4-Cl | 5-F | H | S | Me | H | |
| 4-Br | 5-F | H | S | Me | H | |
| 4-$CF_3$ | 4-F | H | S | Me | H | |
| 4-Cl | 4-F | H | S | Me | H | |
| 4-Br | 4-F | H | S | Me | H | |
| 4-$CF_3$ | 5-Br | H | S | H | Me | |
| 4-Cl | 5-Br | H | S | H | Me | |
| 4-Br | 5-Br | H | S | H | Me | |
| 4-$CF_3$ | 5-F | H | S | H | Me | |
| 4-Cl | 4-F | H | S | H | Me | |
| 4-Br | 4-F | H | S | H | Me | |
| 4-$CF_3$ | 4-F | H | S | H | Me | |
| 4-Cl | 4-F | H | S | H | Me | |
| 4-Br | 4-F | H | S | H | Me | |
| 4-$CF_3$ | 5-Br | H | S | Me | Me | 190 to 196 |
| 4-Cl | 5-Br | H | S | Me | Me | 165 to 186 |
| 4-Br | 5-Br | H | S | Me | Me | 208 to 211 |
| 4-$CF_3$ | 5-F | H | S | Me | Me | 206 to 208 |
| 4-Cl | 5-F | H | S | Me | Me | 182 to 185 |
| 4-Br | 5-F | H | S | Me | Me | 198 to 202 |
| 4-$CF_3$ | 4-F | H | S | Me | Me | 192 to 193 |
| 4-Cl | 4-F | H | S | Me | Me | 170 to 171 |
| 4-Br | 4-F | H | S | Me | Me | 184 to 186 |
| 4-$CF_3$ | 4-Cl | H | S | Me | Me | 170 to 173 |
| 4-Cl | 4-Cl | H | S | Me | Me | 150 to 168 |
| 4-Br | 4-Cl | H | S | Me | Me | 158 to 170 |
| 4-$CF_3$ | 5-$CF_3$ | H | S | Me | Me | 208 to 215 |

TABLE 18

| $R_1$ | $R_2$ | B | V | $R_8^a$ | $R_8^b$ |
|---|---|---|---|---|---|
| 4-$CF_3$ | H | H | O | Me | H |
| 4-$CF_3$ | G | $CO_2Me$ | O | Me | H |
| 4-$CF_3$ | 5-Cl | H | S | Me | H |
| 4-$CF_3$ | 5-Cl | $CO_2Me$ | S | Me | H |
| 4-$CF_3$ | 5-Cl | H | O | H | Me |
| 4-$CF_3$ | 5-Cl | $CO_2Me$ | O | H | Me |
| 4-$CF_3$ | 5-F | H | S | Me | Me |
| 4-$CF_3$ | 5-F | $CO_2Me$ | S | Me | Me |
| 4-$CF_3$ | 5-$OCF_2H$ | H | O | Me | Me |
| 4-$CF_3$ | 5-$OCF_2H$ | $CO_2Me$ | O | H | Me |
| 4-$CF_3$ | 4-Cl | H | S | H | Me |
| 4-$CF_3$ | 4-Cl | $CO_2Me$ | S | H | Me |
| 4-Cl | H | H | O | Me | Me |
| 4-Cl | H | $CO_2Me$ | O | Me | Me |
| 4-Cl | 5-Cl | H | S | Me | Me |
| 4-Cl | 5-Cl | $CO_2Me$ | S | H | Me |
| 4-Cl | 5-Cl | H | O | H | Me |
| 4-Cl | 5-Cl | $CO_2Me$ | O | H | Me |
| 4-Cl | 5-$OCF_2H$ | H | S | Me | Me |
| 4-Cl | 5-$OCF_2H$ | $CO_2Me$ | S | Me | Me |
| 4-Cl | 5-$OCF_2H$ | H | O | Me | Me |
| 4-Cl | 5-$OCF_2H$ | $CO_2Me$ | O | H | Me |
| 4-Cl | 4-F | H | S | H | Me |
| 4-Cl | 4-F | $CO_2Me$ | S | H | Me |

Formulation and Use

The described compounds will generally be used in formulation with a suitable carrier comprising a liquid or solid diluent, including an organic solvent and any other materials that one skilled in the art would appreciate to be useful in the formulation such as an adjuvant, wetting agent, sticking agent, bulking component, and the like. Useful formulations of the compounds of Formula I can be prepared in conventional ways. They include dusts, granules, pellets, solutions, suspensions, emulsions, wettable powders, emulsifiable concentrates, dry flowables and the like. Many of these can be applied directly. High strength compositions are primarily used as intermediates for further formulation. The formulations, broadly, contain about 1% to 99% by weight of active ingredient(s) and at least one of a) about 0.1% to 20% surfactant(s) and b) about 5% to 99% solid or liquid diluent(s). More specifically, they will contain effective amounts of these ingredients in the following approximate proportions:

| | Percent by Weight | | |
|---|---|---|---|
| | Active Ingredient | Diluent(s) | Surfactant(s) |
| Wettable Powders | 25-90 | 0-74 | 1-10 |
| Oil Suspensions, Emulsions, Solutions, (including Emulsifiable Concentrates) | 5-50 | 40-95 | 0-15 |
| Dusts | 1-25 | 70-99 | 0-5 |
| Granules, Baits and Pellets | 0.01-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", Allured Publ. Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pages 147 and following, and "Perry's Chemical Engineer's Handbook", 4th Ed., McGraw-Hill, New York, 1963, pages 8 to 59 and following.

Examples of useful formulations are as follows:

| Example 1 | |
|---|---|
| Emulsifiable Concentrate | |
| 3,3a,4,5-tetrahydro-3a-methyl-N-[4-(trifluoromethyl)phenyl]-2H-benz[g]indazole-2-carboxamide | 5% |
| blend of oil soluble sulfonates and polyoxyethylene ethers | 4% |
| xylene | 91% |

The ingredients are combined and stirred with gentle warming to speed solution. A fine screen filter is included in packaging operation to insure the absence of any extraneous undissolved material in the product.

| Example 2 | |
|---|---|
| Wettable Powder | |
| Methyl 3,3a,4,5-tetrahydro-2-[[4-(trifluoromethyl)phenylamino]carbonyl]-2H-benz[g]indazole-3a-carboxylate | 30% |
| sodium alkylnaphthalenesulfonate | 2% |
| sodium ligninsulfonate | 2% |
| synthetic amorphous silica | 3% |
| kaolinite | 63% |

The active ingredient, warmed to reduce viscosity, is sprayed onto the inert materials in a blender. After grinding in a hammer-mill, the material is re-blended and sifted through a 50 mesh screen.

| Example 3 | |
|---|---|
| Dust | |
| Wettable powder of Example 2 | 10% |
| pyrophyllite (powder) | 90% |

The wettable powder and the pyrophyllite diluent are thoroughly blended and then packaged. The product is suitable for use as a dust.

| Example 4 | |
|---|---|
| Granule | |
| 3,3a,4,5-tetrahydro-3a-methyl-N-[4-(trifluoromethyl)phenyl]-2H-benz[g]indazole-2-carboxamide | 10% |
| attapulgite granules (low volative matter, 0.71/0.30 mm; U.S.S. No. 25-50 sieves) | 90% |

The active ingredient is dissolved in a suitable solvent and sprayed onto dedusted attapulgite granules in a double cone blender. The granules are warmed to drive off solvent, cooled and packaged.

| Example 5 | |
|---|---|
| Granule | |
| Wettable powder of Example 4 | 15% |
| gypsum | 69% |
| potassium sulfate | 16% |

The ingredients are blended in a rotating mixer and water sprayed on to accomplish granulation. When most of the material has reached the desired range of 0.1 to 0.42 mm (U.S.S. No. 18 to 40 sieves), the granules are removed, dried, and screened. Oversize material is crushed to produce additional material in the desired range. These granules contain 4.5% active ingredient.

| Example 6 | |
|---|---|
| Solution | |
| Methyl 3,3a,4,5-tetrahydro-2-[[4-(trifluoromethyl)phenylamino]carbonyl]-2H-benz[g]indazole-3a-carboxylate | 10% |
| isophorone | 90% |

The ingredients are combined and stirred to produce a solution suitable for direct, low volume application.

The rate of application of the Formula I compounds required for effective control will depend on such factors as the species of arthropod to be controlled, the pest's life cycle, life stage, its feeding behavior, mating behavior, ambient moisture, temperature, etc. In general, application rates of 0.05 to 2 kg of active ingredient per hectare are sufficient to provide large-scale effective control of pests in agronomic ecosystems under normal circumstances, but as little as 0.001 kg/hectare or as much as 8 kg hectare may be required. For nonagronomic applications, effective use rates will range from about 0.1 to 5 mg/square foot but as little as about 0.01 mg/square foot or as much as 15 mg/square foot may be required.

The following Examples demonstrate the control efficacy of compounds of Formula I on specific pests; see Table A for compound descriptions.

| Example 7 | |
|---|---|
| Aqueous Suspension | |
| 3,3a,4,5-tetrahydro-3a-methyl-N-[4-(trifluoromethyl)phenyl]-2H-benz[g]indazole-2-carboxamide | 40% |
| polyacrylic acid thickener | 0.3% |
| dodecyclophenol polyethylene glycol ether | 0.5% |
| disodium phosphate | 1.0% |
| monosodium phosphate | 0.5% |
| polyvinyl alcohol | 1.0% |
| water | 56.7% |

The ingredients are blended and ground together in a sand mill to produce particles substantially all under 5 microns in size.

| Example 8 | |
|---|---|
| Oil Suspension | |
| 3,3a,4,5-tetrahydro-3a-methyl-N-[4-(trifluoromethyl)phenyl]-2H-benz[g]indazole-2-carboxamide | 35.0% |
| blend of polyalcohol carboxylic esters and oil soluble petroleum sulfonates | 6.0% |
| xylene range solvent | 59.0% |

The ingredients are combined and ground together in a sand mill to produce particles substantially all below 5 microns. The product can be used directly, extended with oils, or emulsified in water.

| Example 9 | |
|---|---|
| Bait Granules | |
| 3,3a,4,5-tetrahydro-3a-methyl-N-[4-(trifluoromethyl)phenyl]-2H-benz[g]indazole-2-carboxamide | 3.0% |
| blend of polyethoxylated nonylphenols and sodium dodecylbenzene sulfonates | 9.0% |
| ground up corn cobs | 88.0% |

The active ingredient and surfactant blend are dissolved in a suitable solvent such as acetone and sprayed onto the ground corn cobs. The granules are then dried and packaged. Compounds of Formula I can also be mixed with one or more other insecticides, fungicides, nematocides, bactericides, acaricides, or other biologically active compounds to form a multi-component pesticide giving an even broader spectrum of effective agricultural protection. Examples of other agricultural protectants with which the active compounds can be formulated are:

Insecticides:

O-[2,4,5-trichloro-α-(chloromethyl)benzyl]phosphoric acid, O′,O′-dimethyl ester (tetrachlorvinphos)
methylcarbamic acid, ester with α-naphthol (carbaryl)
methyl O-(methylcarbamoyl)thiolacetohydroxamate (methomyl)
O,O-diethyl-O-(2-isopropyl-4-methyl-6-pyrimidylphosphorothioate (diazinon)
(S)-a-cyano-m-phenoxybenzyl(1R,3R)-3-(2,2-dibromovinyl)-2,2-dimethylcyclopropanecarboxylate (deltamethrin)
cyano(3-phenoxyphenyl)-methyl-4-chloro-a-(1-methylethyl)benzeneacetate (fenvalerate)
(3-phenoxyphenyl)methyl(±)-cis,trans-3-(2,2-dichloro ethenyl)-2,2-dimethylcyclopropanecarboxylate (permethrin)
a-cyano-3-phenoxybenzyl 3-(2,2-dichlorovinyl)-2,2-dimethylcyclopropane carboxylate (cypermethrin)
(2,2-dimethyl-1,3-benzodioxo-4-yl-methylcarbamate (bendiocarb)

O,O-diethyl-O-(3,5,6-trichloro-2-pyridyl)phosphorothioate (chlorpyrifos)
[Isopropyl(2E, 4E)-11-methoxy-3,7,11-trimethyl-2,4-dodecadienoate] (methoprene)
N(((4-chlorophenyl)amino)carbonyl)-2,6-difluorobenzamide (diflubenzuron)
2-allyl-4-hydroxy-3-methyl-2-cyclopenten-1-one ester of 2,2-dimethyl-3-(2-methylpropenyl)cyclopropane barboxylic acid (pyrethrin)
2-(1-methylethoxy)phenyl methylcarbamate (propoxur)
N-cyclopropyl-1,3,5-triazine-2,4,6-triamine (cyromazine)
Dimethyl phosphate of alphamethylbenzyl-3-hydroxy-cis-crotonate (crotoxyphos)
O,O-Dimethyl-S-(N-methylcarbamoylmethyl)phosphorodithioate (dimethoate)
(5-benzyl-3-furyl)methyl-2,2-dimethyl-3-(2-methylpropenyl)cyclopropane carboxylate (resmethrin)

Additional insecticides are listed hereafter by their common names: isofenphos, methoxychlor, cyfluthrin, fenpropathrin, fluvalinate, flucythrinate, tralomethrin, fenoxycarb, propetamphos, hydroprene, esfenvalerate, hydramethylnon, sulfluramid, flufenoxuron, buprofezin, chlorfluazuron and hexafluron.

## Utility

The compositions of this invention exhibit activity against a wide spectrum of household and public health arthropods. Those skilled in the art will recognize that not all compositions will be equally effective against all arthropods. Nevertheless, the compositions of this invention display activity against important pest species, such as cockroaches including German or American roaches; house fly, Musca domestica, yellow fever mosquito, Aedes egypti; German cockroach, Blattella germanica; carpenter ants, Camponotus pennsylvanicus and eastern subterranean termite, Reticulitermes flavipes. The pest control afforded by the active compounds is not limited, however, to these species.

The specific species for which control is exemplified below, are: house fly, Musca domestica; German cockroach, Blattella germanica; and European subterranean termite, Reticulitermes santonensis. The pest control afforded by the active compounds is not limited, however, to these species.

## Application

Arthropods are controlled and animals and humans are protected by applying one or more of the compositions of this invention, in an effective amount, to the locus of infestation, to the area to be protected, directly to the pests to be controlled, or to their environment. Because of the diversity of behavior patterns and habitats of the pest species, many different methods of application are employed. These include direct and residual sprays, baits, soil treatment and many others. The indazole compound(s) used in the compositions of this invention can be applied directly, but most often application will be of a formulation comprising one or more compounds in a suitable carrier or diluent. A most preferred method of application involves spraying a water dispersion or refined oil solution of the compositions. The compositions can also be incorporated into baits which are consumed or in devices such as traps and the like that entice the arthropod to ingest or otherwise contact the toxicant compound(s).

The rate of application of Formula I compounds required for effective control will depend on such factors as the species of arthropod to be controlled, the pest's life stage, its size, its location, time of year of application, ambient moisture, temperature conditions, and the like. In general, application rates of 1 to 10 mg of active ingredient per square foot are sufficient to provide effective control under normal circumstances, but as little as 0.1 mg/sq ft may be sufficient or as much as 20 mg/sq ft may be required, depending on the factors listed above.

Another method of application is to incorporate the compound into a bait that will be ingested by the pest. In general, application rates of 0.1 to 0.5 weight percent of active ingredient are sufficient to provide effective control under normal circumstances, but as little as 0.01% may be sufficient or as much as 5% may be required depending upon the factors listed.

The following Examples illustrate the control efficacy of Compounds 1 and 2 on specific arthropod pests. Compound 1 was tested on German cockroachs and found to give less than 40% control after 24 hours.

Compound 1

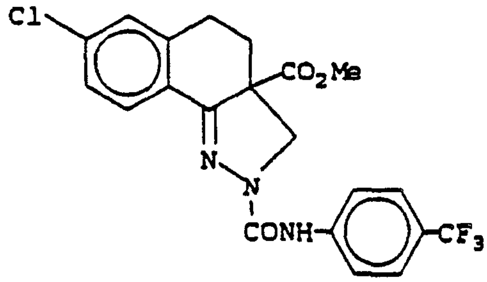

Compound 2

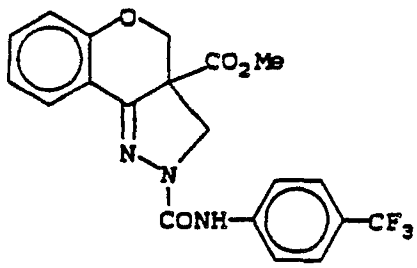

Example 10

House Fly Musca domestica

The test unit consisted of a stainless steel cylinder (1.5" L x 3" D) and two copper screens (2" square). The screens, secured with rubber bands, were used to confine approximately 25 adult house flies (Musca domestica) in the cylinder. A solution (acetone/water; 75/25) of compound 1 was sprayed on the flies in the cylinder. Spraying was accomplished by passing the test unit on a conveyor belt, directly beneath a flat fan hydraulic nozzle which discharged the spray at a rate of 2.6 mg of active ingredient per sg ft at 30 psi. The flat side of the test unit was then placed on a sponge cube, resting in a petri dish containing 10% sugar water. This was held at 27°C and 50% relative humidity for 24 hours, after which mortality readings were taken. There was one treated unit and one untreated control.

House fly mortality was 82% in the treated unit and zero in the control.

Example 11

European Subterranean Termites

(Reticulitermes santonensis)

Topical applications and soil tests were performed.

For topical application, the compound was dissolved in acetone and applied to the dorsal prothorax of worker termites, one microliter of test material per termite. For each rate there were five replicates of ten termites each. Mortality assessments were made 72 hours post-treatment, and $LD_{50}$ values derived.

In the soil bioassay, the test unit consisted of a plastic petri dish (D = 5 cm) and the bottom was lined

with a layer of 1% agar. The dish was filled with pre-treated sand, and 10 worker termites were confined. Each treatment was replicated four times. Mortality was determined 72 hours post-treatment and $LC_{50}$ values derived.

| | Topical Application | Soil Test |
|---|---|---|
| Compound | $LD_{50}$ (µg/mg) | $LC_{50}$ (ppm) |
| 1 | 0.007 | 2.7 |
| 2 | 0.002 | 2.1 |

## Claims

1. An arthropodicidal composition effective against nonagronomic pests comprising an effective amount of a compound of the formula:

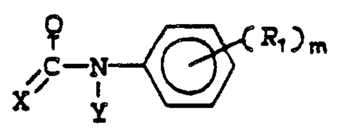

wherein

Q is 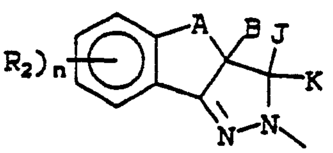

A is a 1, 2 or 3-atom bridge having 0 to 3 carbon atoms and 0 to 1 oxygen atom, $NR_6$ group, or $S(O)_q$ group, wherein each carbon individually can be substituted with 1 to 2 substituents selected from 1 to 2 halogen, $C_1$ to $C_6$ alkyl, $C_2$ to $C_4$ alkoxycarbonyl or phenyl optionally substituted with 1 to 3 substituents selected from W and one of the carbon atoms can be C(O) or C(S);
B is H, $C_1$ to $C_6$ alkyl, $C_4$ to $C_7$ cycloalkylalkyl, $C_3$ to $C_6$ cycloalkyl optionally substituted with 1 to 2 halogens or 1 to 2 $CH_3$; $C_1$ to $C_6$ haloalkyl, $C_2$ to $C_6$ alkenyl, $C_2$ to $C_6$ haloalkenyl, $C_2$ to $C_6$ alkynyl, $OR_7$, $C_2$ to $C_6$ alkoxyalkyl, $C_2$ to $C_6$ cyanoalkyl, $C_3$ to $C_8$ alkoxycarbonylalkyl, $CO_2R_3$, $C(O)R_3$, $C(O)NR_3R_4$, C(S)-$NR_3R_4$, $C(S)R_3$, $C(S)SR_3$, phenyl, phenyl substituted by $(R_5)_p$, benzyl, or benzyl substituted, with 1 to 3 substituents independently selected from W;
J is H, $C_1$ to $C_4$ alkyl or phenyl optionally substituted with W;
K is H or $CH_3$;
W is halogen, CN, $NO_2$, $C_1$ to $C_2$ alkyl, $C_1$ to $C_2$ haloalkyl, $C_1$ to $C_2$ alkoxy, $C_1$ to $C_2$ haloalkoxy, $C_1$ to $C_2$ alkylthio, $C_1$ to $C_2$ haloalkylthio, $C_1$ to $C_2$ alkylsulfonyl or $C_1$ to $C_2$ haloalkylsulfonyl;
$R_1$, $R_2$ and $R_5$ are independently $R_3$, halogen, CN, $N_3$, SCN, $NO_2$, $OR_3$, $SR_3$, $SOR_3$, $SO_2R_3$, $OC(O)R_3$, $OSO_2R_3$, $CO_2R_3$, $C(O)R_3$, $C(O)NR_3R_4$, $SO_2NR_3R_4$, $NR_3R_4$, $NR_4C(O)R_3$, $OC(O)NHR_3$, $NR_4C(O)NHR_3$, $NR_4SO_2R_3$, or when m, n or p is 2, $R_1$, $R_2$ or $R_5$ can be a 5-or 6-membered fused ring selected from the group $-OCH_2O-$, $-OCH_2CH_2O-$, or $-CH_2CH_2O-$, each of which can be substituted with 1 to 4 halogen atoms or 1 to 2 methyl groups;
$R_3$ is H, $C_1$ to $C_6$ alkyl, $C_1$ to $C_6$ haloalkyl, $C_2$ to $C_6$ alkenyl, $C_2$ to $C_6$ haloalkenyl, $C_2$ to $C_6$ alkynyl, $C_2$ to $C_6$ haloalkynyl, $C_2$ to $C_6$ alkoxyalkyl, $C_2$ to $C_6$ alkylthioalkyl, $C_1$ to $C_6$ nitroalkyl, $C_2$ to $C_6$ cyanoalkyl, $C_3$ to $C_8$ alkoxycarbonylalkyl, $C_3$ to $C_6$ cycloalkyl, $C_3$ to $C_6$ halocycloalkyl, phenyl, benzyl, or phenyl or benzyl substituted with 1 to 3 substituents independently selected from W;

$R_4$ is H, $C_1$ to $C_4$ alkyl or $R_3$ and $R_4$ can be taken together as $(CH_2)_4$, $(CH_2)_5$ or $(CH_2CH_2OCH_2CH_2)$;
$R_6$ is H, $C_1$ to $C_4$ alkyl, $C_1$ to $C_4$ haloalkyl, $C_2$ to $C_4$ alkenyl, $C_2$ to $C_4$ haloalkenyl, phenyl, benzyl, phenyl optionally substituted with W or benzyl optionally substituted with W;
$R_7$ is H, $C_1$ to $C_4$ alkyl, $C_2$ to $C_4$ alkenyl, $C_2$ to $C_4$ alkynyl, $C_2$ to $C_4$ alkylcarbonyl, $C_2$ to $C_4$ alkoxycarbonyl, $C_1$ to $C_4$ alkylsulfonyl;
X is O or S;
n is 1 to 4;
m is 1 to 5;
p is 1 to 3;
q is 0 to 2; and
Y is H, $C_1$ to $C_6$ alkyl, benzyl, $C_2$ to $C_6$ alkoxyalkyl, CHO, $C_2$ to $C_6$ alkylcarbonyl, $C_2$ to $C_6$ alkoxycarbonyl, $C_2$ to $C_6$ haloalkylcarbonyl, $C_1$ to $C_6$ alkylthio, $C_1$ to $C_6$ haloalkylthio, phenylthio, or phenylthio substituted with 1 to 3 substituents independently selected from W;
and an arthropodicidally compatible carrier therefor.

2. A composition according to Claim 1 wherein Q is:

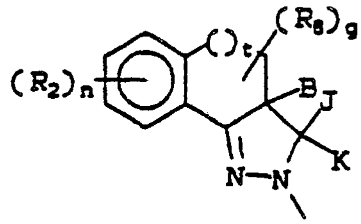 ; 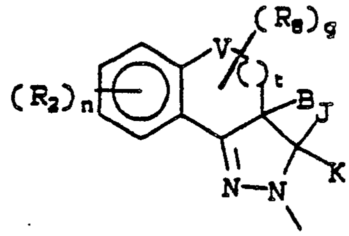 ;

Q-1 Q-2

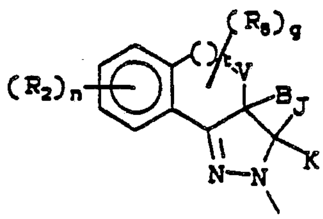 ; 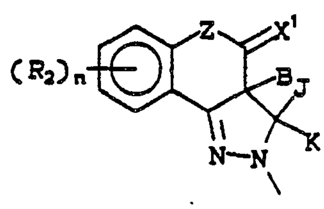 ; or

Q-3 Q-4

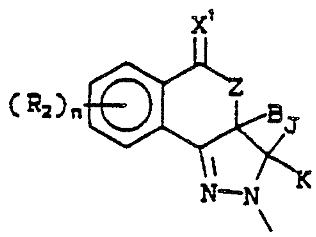 ;

Q-5

X is O or S;
$X^1$ is O or S;
t is 0, 1 or 2;
V is O, $S(O)_q$ or $NR_6$;
Z is O or $NR_6$;
$R_8$ is halogen, $C_1$ to $C_6$ alkyl, $C_2$ to $C_4$ alkoxycarbonyl or phenyl optionally substituted by 1 to 3 substitutents selected from W; and
g is 0, 1 or 2.

3. A composition according to Claim 2 wherein:
B is H, $C_1$ to $C_6$ alkyl, $C_1$ to $C_6$ haloalkyl, $C_2$ to $C_6$ alkenyl, $C_2$ to $C_6$ alkynyl, $C_2$ to $C_6$ alkoxyalkyl, $C_3$ to $C_8$ alkoxycarbonylalkyl, $CO_2R_3$, $C(O)R_3$, $C(O)NR_3R_4$, $C(S)NR_3R_4$, $C(S)R_3$, $C(S)SR_3$, phenyl or phenyl substituted by $(R_5)_p$;

J is H or $CH_3$;

$R_3$ is H, $C_1$ to $C_4$ alkyl, $C_1$ to $C_2$ haloalkyl, $C_3$ to $C_4$ alkenyl, $C_3$ to $C_4$ haloalkenyl, propargyl, phenyl, benzyl, or phenyl or benzyl substituted with one of F, Cl, Br, $CF_3$ or $NO_2$;

$R_6$ is H, $C_1$ to $C_4$ alkyl, allyl or propargyl;

n is 1 or 2;

p is 1 or 2;

m is 1 to 3; and

Y is H, $C_1$ to $C_4$ alkyl, $SCH_3$, $SCCl_3$, $SO_2CH_3$, $SC_6H_5$, 2-$NO_2$-$C_6H_4S$, C(O)$CH_3$, C(O)$CF_3$; $CO_2CH_3$ or $CO_2CH_2CH_3$.

4. A composition according to Claim 3 wherein:

B is H, $C_1$ to $C_4$ alkyl, $C_3$-$C_4$ alkoxycarbonylalkyl, $CO_2R_3$, C(O)$R_3$, phenyl or phenyl substituted by $(R_5)_p$;

J is H;

K is H;

$R_1$ is H, halogen, CN, SCN, $NO_2$, $OR_3$, $SR_3$, $SO_2R_3$, $CO_2R_3$, C(O)$R_3$ or $R_3$ with one $R_1$ substituent in the 4-position, or when m is 2 then $R_1$ can be taken together as -$CH_2C(CH_3)_2O$-, $OCH_2CH_2O$-, $OCF_2CF_2O$-or -$CF_2CF_2O$- to form a 5 or 6 membered fused ring;

$R_2$ and $R_5$ are H, $R_3$, halogen, CN, SCN, $NO_2$, $OR_3$, $SR_3$, $SO_2R_3$, OC(O)$R_3$, $OSO_2R_3$, $CO_2R_3$, C(O)$R_3$, C-(O)$NR_3R_4$, $SO_2NR_3R_4$ or $NR_3R_4$;

$R_3$ is $C_1$ to $C_4$ alkyl, $C_1$ to $C_2$ haloalkyl, $C_3$ to $C_4$ alkenyl or propargyl;

$R_4$ and $R_8$ are H or $CH_3$;

X is O;

X′ is O;

m is 1 or 2;

t is 1; and

q is O.

5. A composition according to Claim 4 wherein:

$R_1$ is Cl, F, Br, $CF_3$, CN, $OCH_2CF_3$, $OCF_3$, $OCF_2H$ or $SCF_2H$;

$R_2$ is H, Cl, F, Br, CN, $CF_3$, $CH_3$, $OCH_2CF_3$, $OCF_2H$, $OCF_3$, $SCH_3$, $SCF_2H$, $SO_2CH_3$ or $NO_2$;

$R_6$ is H or $CH_3$;

B is H, $CH_3$, $CH_2CH_3$, $CH(CH_3)_2$, $CO_2CH_3$, $CO_2CH_2CH_3$, phenyl or phenyl substituted by halogen, CN, $CF_3$ or $NO_2$; and

Y is H, $COCH_3$ or $CO_2CH_3$.

6. A composition according to Claim 5 wherein Q is Q-1.

7. A composition according to Claim 5 wherein Q is Q-2.

8. A composition according to Claim 6 which is:

methyl 3,3a,4,5-tetrahydro-2-[[4-(trifluoromethyl)phenylamino]carbonyl]-2H-benz[g]indazole-3a-carboxylate.

9. A composition according to Claim 6 which is:

methyl 7-chloro-3,3a,4,5-tetrahydro-2-[[4-trifluoromethyl)phenylamino]carbonyl]-2H-benz[g]indazole-3a-carboxylate.

10. A method for controlling nonagronomic arthropods comprising contacting them or their environment with an arthropodicidally effective amount of a compound having the formula:

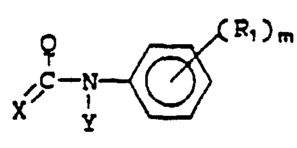

I

wherein

Q is 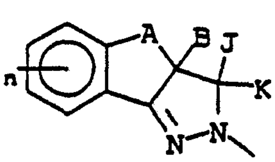

A is a 1, 2 or 3-atom bridge having 0 to 3 carbon atoms and 0 to 1 oxygen atom, $NR_6$ group, or $S(O)_q$ group, wherein each carbon individually can be substituted with 1 to 2 substituents selected from 1 to 2 halogen, $C_1$ to $C_6$ alkyl, $C_2$ to $C_4$ alkoxycarbonyl or phenyl optionally substituted with 1 to 3 substituents selected from W and one of the carbon atoms can be C(O) or C(S);
B is H, $C_1$ to $C_6$ alkyl, $C_4$ to $C_7$ cycloalkylalkyl, $C_3$ to $C_6$ cycloalkyl optionally substituted with 1 to 2 halogens or 1 to 2 $CH_3$; $C_1$ to $C_6$ haloalkyl, $C_2$ to $C_6$ alkenyl, $C_2$ to $C_6$ haloalkenyl, $C_2$ to $C_6$ alkynyl, $OR_7$, $C_2$ to $C_6$ alkoxyalkyl, $C_2$ to $C_6$ cyanoalkyl, $C_3$ to $C_8$ alkoxycarbonylalkyl, $CO_2R_3$, $C(O)R_3$, $C(O)NR_3R_4$, $C(S)NR_3R_4$, $C(S)R_3$, $C(S)SR_3$, phenyl, phenyl substituted by $(R_5)_p$, benzyl, or benzyl substituted with 1 to 3 substituents independently selected from W;
J is H, $C_1$ to $C_4$ alkyl or phenyl optionally substituted with W;
K is H or $CH_3$;
W is halogen, CN, $NO_2$, $C_1$ to $C_2$ alkyl, $C_1$ to $C_2$ haloalkyl, $C_1$ to $C_2$ alkoxy, $C_1$ to $C_2$ haloalkoxy, $C_1$ to $C_2$ alkylthio, $C_1$ to $C_2$ haloalkylthio, $C_1$ to $C_2$ alkylsulfonyl or $C_1$ to $C_2$ haloalkylsulfonyl;
$R_1$, $R_2$ and $R_5$ are independently $R_3$, halogen, CN, $N_3$, SCN, $NO_2$, $OR_3$, $SR_3$, $SOR_3$, $SO_2R_3$, $OC(O)R_3$, $OSO_2R_3$, $CO_2R_3$, $C(O)R_3$, $C(O)NR_3R_4$, $SO_2NR_3R_4$, $NR_3R_4$, $NR_4C(O)R_3$, $OC(O)NHR_3$, $NR_4C(O)NHR_3$, $NR_4SO_2R_3$, or when m, n or p is 2, $R_1$, $R_2$ or $R_5$ can be a 5- or 6-membered fused ring selected from the group $-OCH_2O-$, $-OCH_2CH_2O-$, or $-CH_2CH_2O-$, each of which can be substituted with 1 to 4 halogen atoms or 1 to 2 methyl groups;
$R_3$ is H, $C_1$ to $C_6$ alkyl, $C_1$ to $C_6$ haloalkyl, $C_2$ to $C_6$ alkenyl, $C_2$ to $C_6$ haloalkenyl, $C_2$ to $C_6$ alkynyl, $C_2$ to $C_6$ haloalkynyl, $C_2$ to $C_6$ alkoxyalkyl, $C_2$ to $C_6$ alkylthioalkyl, $C_1$ to $C_6$ nitroalkyl, $C_2$ to $C_6$ cyanoalkyl, $C_3$ to $C_8$ alkoxycarbonylalkyl, $C_3$ to $C_6$ cycloalkyl, $C_3$ to $C_6$ halocycloalkyl, phenyl, benzyl, or phenyl or benzyl substituted with 1 to 3 substituents independently selected from W;
$R_4$ is H, $C_1$ to $C_4$ alkyl or $R_3$ and $R_4$ can be taken together as $(CH_2)_4$, $(CH_2)_5$ or $(CH_2CH_2OCH_2CH_2)$;
$R_6$ is H, $C_1$ to $C_4$ alkyl, $C_1$ to $C_4$ haloalkyl, $C_2$ to $C_4$ alkenyl, $C_2$ to $C_4$ haloalkenyl, phenyl, benzyl, phenyl optionally substituted with W or benzyl optionally substituted with W;
$R_7$ is H, $C_1$ to $C_4$ alkyl, $C_2$ to $C_4$ alkenyl, $C_2$ to $C_4$ alkynyl, $C_2$ to $C_4$ alkylcarbonyl, $C_2$ to $C_4$ alkoxycarbonyl, $C_1$ to $C_4$ alkylsulfonyl;
X is O or S;
n is 1 to 4;
m is 1 to 5;
p is 1 to 3;
q is 0 to 2; and
Y is H, $C_1$ to $C_6$ alkyl, benzyl, $C_2$ to $C_6$ alkoxyalkyl, CHO, $C_2$ to $C_6$ alkylcarbonyl, $C_2$ to $C_6$ alkoxycarbonyl, $C_2$ to $C_6$ haloalkylcarbonyl, $C_1$ to $C_6$ alkylthio, $C_1$ to $C_6$ haloalkylthio, phenylthio, or phenylthio substituted with 1 to 3 substituents independently selected from W.

11. A method for controlling nonagronomic arthropods comprising contacting them or their environment with an arthropodicidally effective amount of a composition according to any one of Claims 1 to 9.

European Patent Office

# EUROPEAN SEARCH REPORT

Application Number

EP 89 31 0361

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-4 663 341 (RICHARD M. JACOBSON, ROHM AND HAAS CO.) --- | | C 07 D 231/54<br>A 01 N 47/38<br>C 07 D 471/04<br>C 07 D 487/04<br>C 07 D 491/04<br>C 07 D 495/04 |
| D,A | US-A-4 070 365 (JAN JOHANNES VAN DAALEN & RUDOLF MULDER, (U.S. PHILIPS CORP.)) --- | | |
| D,A | US-A-3 991 073 (RUDOLF MULDER & KOBUS WELLINGA, (U.S. PHILIPS CORP.)) --- | | |
| D,A | EP-A-0 286 346 (E.I. DU PONT DE NEMOURS AND CO.) ----- | | |
| | | | TECHNICAL FIELDS SEARCHED (Int. Cl.5) |
| | | | C 07 D 231/00<br>A 01 N 47/00<br>C 07 D 471/00<br>C 07 D 487/00<br>C 07 D 491/00<br>C 07 D 495/00 |

The present search report has been drawn up for all claims

| Place of search | Date of completion of the search | Examiner |
|---|---|---|
| THE HAGUE | 10-01-1990 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document